(19) Europäisches Patentamt / European Patent Office / Office européen des brevets

(11) **EP 3 143 038 B1**

(12) **EUROPÄISCHE PATENTSCHRIFT**


(45) Veröffentlichungstag und Bekanntmachung des Hinweises auf die Patenterteilung:
**01.08.2018 Patentblatt 2018/31**

(21) Anmeldenummer: **15724512.7**

(22) Anmeldetag: **15.05.2015**

(51) Int Cl.:
***C07K 14/00*** *(2006.01)* ***C07D 239/00*** *(2006.01)*
***C07D 239/46*** *(2006.01)*

(86) Internationale Anmeldenummer:
**PCT/EP2015/000998**

(87) Internationale Veröffentlichungsnummer:
**WO 2015/172889 (19.11.2015 Gazette 2015/46)**


(54) **PEPTID-NUKLEINSÄUREN-MONOMERE UND -OLIGOMERE**

PEPTIDE-NUCLEIC ACID-MONOMERS AND -OLIGOMERS

MONOMÈRES ET OLIGOMÈRES D'ACIDE NUCLÉIQUE PEPTIDIQUE


(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **16.05.2014 DE 102014007158**

(43) Veröffentlichungstag der Anmeldung:
**22.03.2017 Patentblatt 2017/12**



(73) Patentinhaber: **Ugisense AG**
**80333 München (DE)**

(72) Erfinder:
- **LINDHORST, Thomas**
  **A-6020 Innsbruck (AT)**
- **WERNER, Birgit**
  **85748 Garching (DE)**
- **BOCK, Holger**
  **A-6020 Innsbruck (AT)**

(74) Vertreter: **Forstmeyer, Dietmar**
**BOETERS & LIECK**
**Oberanger 32**
**80331 München (DE)**


(56) Entgegenhaltungen:
**WO-A1-00/52038** **WO-A1-2008/009470**
**WO-A2-2008/049583**


Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).


EP 3 143 038 B1

## Beschreibung

Die vorliegende Erfindung betrifft neue Peptid-Nukleinsäure-Monomere und Peptid-Nukleinsäure-Oligomere, die eine mit Phosphonsäureester- oder Phosphonsäure-Gruppe(n) substituierte Dialkylamin-Seitenkette aufweisen, deren Herstellung, sowie deren Verwendungen.

Peptid-Nukleinsäuren (PNAs) sind synthetische DNA/RNA-Analoga mit einem N-(2-Aminoethyl)glycin-Gerüst - siehe allgemeine Formel (PNA). In der allgemeinen Formel bezeichnet NB eine Nukleobase; n die Anzahl der PNA-Einheiten (n = 0-50); und F und G stellen Substituenten dar.

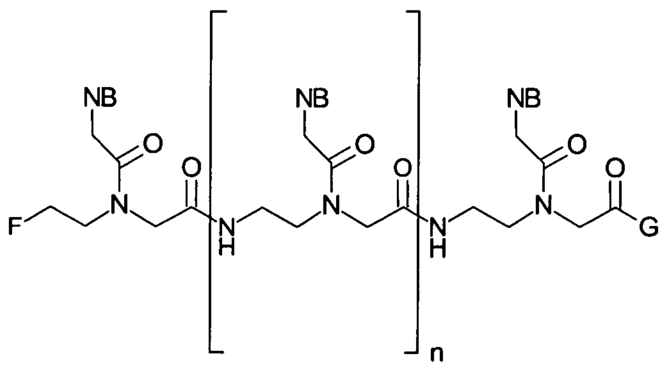

(PNA)

PNAs werden durch Knüpfen von Peptidbindungen zwischen N-Acetyl-N-(2-aminoethyl)glycin-Bausteinen (PNA-Monomeren) hergestellt. Jeder einzelne dieser N-Acetyl-N-(2-aminoethyl)glycin-Bausteine stellt eine PNA-Einheit dar.

Die Vorteile von PNAs sind, dass sie unter physiologischen Bedingungen resistent gegen hydrolytische (enzymatische) Spaltung sind sowie komplementäre Nukleinsäuresequenzen (DNA oder RNA) sequenz-spezifisch erkennen und an diese mit hoher Affinität binden können. PNAs werden deshalb als attraktive Verbindungen für biotechnologische und/oder medizinische Anwendungen, zum Beispiel als Diagnostika oder in der Antisense-Therapie, angesehen.

Für einen erfolgreichen Einsatz als Wirkstoff in einer Antisense-Therapie ist eine ausreichende Bioverfügbarkeit im lebenden Organismus essentiell. Der Antisense-Wirkstoff muss in einer, für einen therapeutischen Effekt, ausreichenden Menge am Wirkort, der Target-RNA bzw. DNA vorhanden sein. Das bedeutet, dass der Antisense-Wirkstoff nach seiner Vergabe in ausreichendender Menge zunächst (i) in die Gewebe, dann (ii) in die Gewebe-Zellen und schließlich (iii) innerhalb der Zellen in das Zellkompartiment zur Target-RNA bzw. DNA vordringen muss, um einen Antisense-Effekt in einem therapeutisch relevanten Ausmaß erzielen zu können.

Allerdings haben PNAs den Nachteil, dass sie, verglichen mit DNA, schwer wasserlöslich sind und Zellmembranen nur schlecht durchdringen können. Entsprechend ist die Verwendung von PNAs als Wirkstoff in der Antisense-Therapie in lebenden Organismen stark limitiert, wie beispielsweise von Beth M. et al., Antisense & Nucleic Acid Drug Development (2002) 12:65-70) anhand von Untersuchungen zur Aufnahme bzw. Bioverfügbarkeit von PNAs in verschiedenen Organen und Geweben gezeigt wurde. In ihren Untersuchungen stellten Beth M. et al. fest, dass PNAs nach intraperitonealer Gabe an Ratten innerhalb von 24 Stunden zu 90% unverändert wieder ausgeschieden werden. Die Peptidnukleinsäuren wurden von der Niere nur zu 2,5% - 4,5%, in allen anderen untersuchten Organen sogar deutlich unter 1% aufgenommen.

Zur Verbesserung bestimmter Eigenschaften wie Wasserlöslichkeit, Bindungseigenschaften an komplementäre DNA bzw. RNA oder Aufnahme in Zellen von PNAs wurden unter anderem eine Modifikation der PNAs durch Einführung einer Gruppe R an der alpha-Position der PNA-Einheit gemäß der folgenden allgemeinen Formel (PNA*) vorgeschlagen:

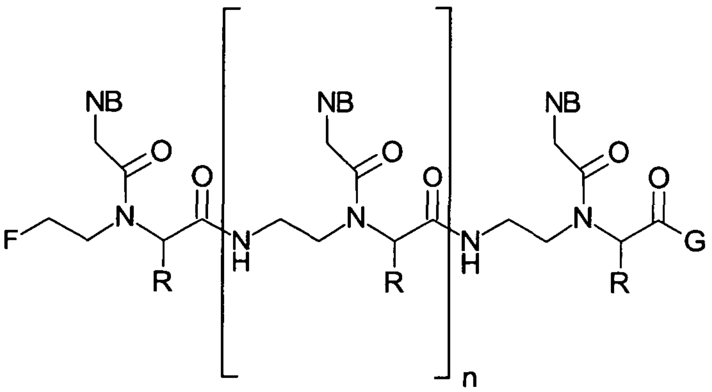

(PNA*)

Beispielsweise wurden als Gruppe R zur Modifikation von PNAs Seitenketten natürlicher Aminosäuren vorgeschlagen (Püschl A. et al., Tetrahedron Letters 39, (1998) 4707-4710; US 5719262). Obwohl durch die Modifikation die sterische Hinderung erhöht ist, ist die Bindung derartig modifizierter PNAs an komplementäre DNA/RNA nur minimal erniedrigt, wie anhand von Messungen des Schmelzpunktes von PNA/DNA-Hybriden festgestellt wurde (Püschl A. et al., loc. cit.).

PNAs mit einem Lysin modifizierten Grundgerüst (R=$-(CH_2)_4-NH_2$) zeigten eine verbesserte Wasserlöslichkeit und einen erhöhten Schmelzpunkt von PNA/DNA-Hybriden (US 5719262). Der Nachteil dieser Lysin modifizierten PNAs besteht allerdings darin, dass diese nach der Aufnahme in das Zellinnere in den intrazellulären Endosomen gefangen bleiben (Nielsen P., Quarterly Reviews of Biophysics 38, 4 (2005), 345-350; Koppelhus U. et al., Antisense & Nucleic Acid Drug Development (2002) 12:51-63). Dadurch stehen derart modifizierte PNAs innerhalb der Zelle für eine Bindung an RNA nicht ausreichend zur Verfügung und sind damit für eine therapeutische Anwendung unbrauchbar. Wie R. Corradini et al., Current Topics in Medicinal Chemistry, 11 (12), pp. 1535-1554, (2011) beobachteten, werden derartige mit Lysin modifizierte PNAs, wie sie beispielsweise in US 5719262 beschrieben sind, in einigen Zellen überhaupt nicht aufgenommen, und selbst wenn es zu einer Aufnahme in die Zellen kam, blieben sie oftmals in Vesikeln gefangen. Bei der untersuchten Auswahl von Zellen wurde keine Aufnahme in den Zellkern beobachtet (loc. cit., p. 1543).

PNAs mit einem Arginin modifizierten Grundgerüst, so genannte Guanidin basierte Peptidnukleinsäuren (GPNAs):

R =

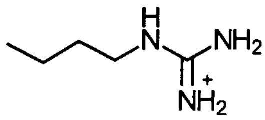

wurden zur Verbesserung der Aufnahme von PNAs in Zellen vorgeschlagen (Zhou P. et al., J. AM. CHEM. SOC. (2003) 125: 6878-6879). GPNAs sind nach der Zell-Aufnahme intrazellulär im Endoplasmatischen Retikulum (ER) lokalisiert und können damit für eine Bindung an zelleigene mRNA zur Verfügung stehen. Allerdings werden GPNAs im lebenden Organismus nach systemischer Vergabe nur von Niere, Leber und Tumorgewebe aufgenommen (Thomas S. M. et al., ACS Chem. Biol.2013 February 15; 8(2):345-352). Diese limitierte Bioverfügbarkeit verhindert eine breitere Anwendung der GPNAs als Therapeutika.

Aus EP 1157031, EP 2041161 und Posch W. et al., Mol Med. (2012) 18: 111-22, sind Alkyl-Phosphonsäureester modifizierte PNAs mit R= $-(CH_2)_n-P=O(OEt)_2$, (n= 1,2) bekannt. Derart modifizierte PNAs sind besser zellgängig und besser wasserlöslich im Vergleich zu PNAs, die diese Modifikation nicht enthalten. Darüber hinaus sind derart modifizierte PNAs in der Lage, bei HIV eine Wirksamkeit über zwei Virengenerationen zu zeigen. Ferner weisen diese mit Alkyl-Phosphonsäureester-Gruppe modifizierten PNAs im Vergleich zu GPNAs eine bessere Bioverfügbarkeit auf. Allerdings haben mit Alkyl-Phosphonsäureester-Gruppe modifizierte PNAs den Nachteil, dass ihre Wasserlöslichkeit von der Nukleobasen-Sequenz abhängig ist. So ist beispielsweise bei einer Sequenz, die eine größere Anzahl von Guanin- und Cytosin-Basen enthält, die Wasserlöslichkeit dieser PNAs reduziert. Diese sequenzabhängige Wasserlöslichkeit kann die therapeutische Anwendung erschweren.

Für einen breiten Einsatz als Therapeutikum ist für PNAs eine Kombination aus mehreren verschiedenen Eigenschaften wichtig: gute und sequenzunabhängige Wasserlöslichkeit; gute Zell-Aufnahme; gute Bindungseigenschaften an DNA und/oder RNA; gute Bioverfügbarkeit (je besser die Bioverfügbarkeit in verschiedenen Geweben, desto mehr Möglichkeiten einer therapeutischen Anwendung stehen zur Verfügung); lange Halbwertszeiten (um eine Bindung der PNA an die DNA und/oder RNA auch in einer Zelle im lebenden Organismus zu erreichen und dort den gewünschten

Effekt der Modulation der Genexpression zu erzielen); gute Bindung an Blut-Plasma-Proteine zur Unterstützung der Bioverfügbarkeit und Verlängerung der Halbwertszeit (an Blut-Plasma-Proteine gebundene Oligomere werden nicht so schnell in der Niere aus dem Blutkreislauf herausgefiltert und über den Urin ausgeschieden); und ein starker Effekt der Modulation der Genexpression, für die auch ein gute Zellaufnahme und intrazelluläre Verteilung sowie gute Bindungseigenschaften der PNA an DNA und/oder RNA erforderlich sind.

Der Nachteil der bekannten modifizierten PNAs besteht darin, dass sie lediglich einzelne der wichtigen Eigenschaften (i) gute und Sequenz-unabhängige Wasserlöslichkeit, (ii) gute Zell-Aufnahme und intrazelluläre Verteilung, (iii) gute Bioverfügbarkeit und lange Halbwertszeiten in möglichst vielen therapeutisch relevanten Geweben (iv) gute Bindung an Blut-Plasma-Proteine bzw. (v) starker Effekt der Modulation der Genexpression aufweisen, jedoch nicht alle für eine breite Anwendbarkeit als Therapeutikum notwendigen Eigenschaften besitzen.

Die Aufgabe der vorliegenden Erfindung besteht damit in der Bereitstellung neuartiger modifizierter PNA-Monomere; und in der Bereitstellung neuartiger modifizierter PNA-Oligomere mit verbesserten Eigenschaftsprofilen, welche die neuen PNA-Monomere als Bausteine enthalten. Das verbesserte Eigenschaftsprofil betrifft die Kombination der Eigenschaften (i) gute und Sequenz-unabhängige Wasserlöslichkeit, (ii) gute Zell-Aufnahme und intrazelluläre Verteilung, (iii) gute Bioverfügbarkeit und lange Halbwertszeiten in möglichst vielen therapeutisch relevanten Geweben (iv) starke Bindung an Blut-Plasma-Proteine sowie (v) starker Effekt der Modulation der Genexpression.

Weiter ist es Aufgabe der Erfindung, neue Verfahren zur Anwendung der vorgenannten modifizierten PNA-Oligomere, sowie diagnostische und therapeutische Zusammensetzungen, welche die genannten modifizierten PNA-Oligomere enthalten, zur Verfügung zu stellen.

Diese und andere Aspekte der vorliegenden Erfindung werden unter Berücksichtigung der folgenden detaillierten Beschreibung und Definitionen sichtbar.

Die Erfindung betrifft:

[1] eine Verbindung der allgemeinen Formel (I):

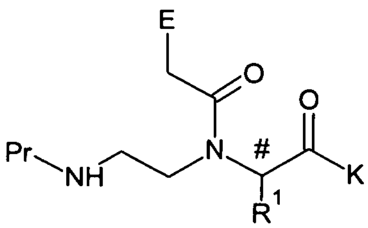

(I)

wobei

K eine Carbonsäure-Aktivester-Gruppe oder -O-$R_M$ darstellt; wobei $R_M$ ein H-Atom, eine Methyl-, Ethyl-, Allyl-, Benzyl-, Phenyl-, tert.-Butyl-, oder eine Trimethylsilyl-Gruppe darstellt;

Pr ein H-Atom oder eine Amino-Schutzgruppe darstellt;

# ein asymmetrisches C-Atom kennzeichnet;

E ein H-Atom, eine Phenylgruppe, ein Heterocyclus, eine Nukleobase, oder eine mit einer Nukleobasen-Schutzgruppe substituierte Nukleobase ist;

$R^1$ eine Gruppe ist, die durch die allgemeine Formel (II) dargestellt ist:

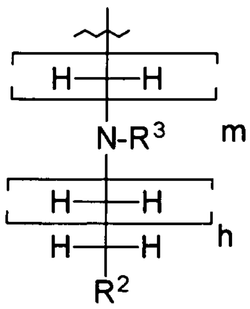

(II)

wobei

$R^2$ eine Phosphonsäureester- oder eine Phosphonsäure-Gruppe ist;

$R^3$ ein H-Atom, oder eine Amino-Schutzgruppe ist;

m eine ganze Zahl von 1 bis 5 darstellt; und

h eine ganze Zahl von 0 bis 4 darstellt;

mit der Maßgabe, dass für die Summe von m und h in der allgemeinen Formel (II) gilt: $2 \leq x \leq 5$.

Durch die Bindung der Gruppe $R^1$ an das Backbone der monomeren Verbindung gemäß der allgemeinen Formel (I) entsteht an der Verbindungsstelle von $R^1$ und Backbone ein asymmetrisches Zentrum (#) im Backbone. An jedem solchen asymmetrischen Zentrum im Backbone (#) kann entweder eine R-Konfiguration oder eine S-Konfiguration vorliegen. Dabei wird die Konfiguration an diesem asymmetrischen Zentrum (#) analog den Cahn-Ingold-Prelog-Regeln definiert, mit der zusätzlichen Maßgabe, dass die Priorität der Liganden immer wie folgt definiert ist: Das Stickstoffatom am asymmetrischen Zentrum erhält immer die Priorität 1. Das Kohlenstoffatom der Carboxylgruppe am asymmetrischen Zentrum erhält immer die Priorität 2. Das Kohlenstoffatom der Gruppe $R^1$ am asymmetrischen Zentrum erhält immer die Priorität 3. Das Wasserstoffatom am asymmetrischen Zentrum erhält immer die Priorität 4.

Der Ausdruck Carbonsäure-Aktivester-Gruppe bezeichnet dem Fachmann bekannte Carbonsäurederivate, die üblicherweise in der Peptidchemie zur Erhöhung der Kupplungsreaktivität der Carbonsäure-Funktion eingesetzt werden. Solche Carbonsäure-Aktivester-Gruppen sind beispielsweise beschrieben in: O. Marder, F.Albericio, Chimica Oggi, 2002, 37; N. Sewald, H.-D. Jakubke, (eds), Peptide Chemistry, Wiley-VCH Verlag, Weinheim 2002, Kap.4.3 Peptide Bond Formation, S.197. Beispiele für eine Carbonsäure-Aktivester-Gruppe sind Carbonsäurehalogenide, Acylphosphoniumsalze wie Tris(pyrrolidino)-phosphonium carboxylate (durch Umsetzung mit PyBroP), Anhydride, Thiophenylester, Cyanomethylester, Nitro- und Dinitrophenylester, Pentafluorphenylester, Chlorphenylester, Trichlorphenylster, Pentachlorphenylester, sowie die in nachfolgender Tabelle aufgelisteten Aktivester

| **Aktivierende Gruppe** | **Allgemein gebräuchliches Symbol** | **Struktur** | **(Kurz)name für das Reagenz, mit dem die aktivierende Gruppe unter anderem eingeführt wird** |
|---|---|---|---|
| N-Hydroxypiperidinyl | OPip | 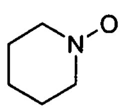 | N-Hydroxypiperidin (HOPip) |
| 8-Quinolyl | OQ | 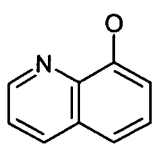 | 8-Hydroxyquinolin |

(fortgesetzt)

| Aktivierende Gruppe | Allgemein gebräuchliches Symbol | Struktur | (Kurz)name für das Reagenz, mit dem die aktivierende Gruppe unter anderem eingeführt wird |
|---|---|---|---|
| N-Hydroxysuccinimidyl | OSu | 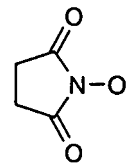 | N-Hydroxysuccinimid HOSu N,N'-Disuccinimidyl carbonat DSC |
| 1-Hydroxybenzotrizolyl | OBt | 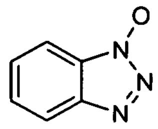 | HOBt, BOP, PyBOP HBTU, TBTU |
| 7-Aza-1-hydroxybenzotriazolyl | OAt | 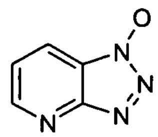 | HOAt, PyAOP, HATU, HAPyu, HAPipU, HAMDU, HAMTU |
| 6-Chloro-1-hydroxybenzotriazolyl | ClOBt | 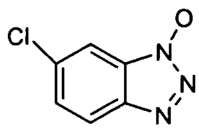 | HCTU |
| N-Norbornen-2,3-dicarboximidooxy | ONdc | 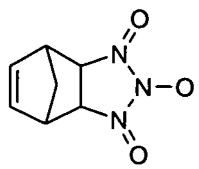 | n-Hydroxy-5-Norbornen-2,3-dicarboximide HONB bzw. HONdc |
| Ethyl-1-hydroxy-1H-1,2,3-triazol-4-carboxylat | OCt | 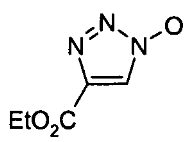 | Ethyl-1-hydroxy-1H-1,2,3-triazol-4-carboxylat HOCt |

Der Ausdruck Amino-Schutzgruppe bezeichnet dem Fachmann bekannte Schutzgruppen, die bei der organischen Synthese von Aminosäuren oder Peptiden eingesetzt werden, beispielsweise eine Trifluoracetyl-, Oxocarbamat-, Thiocarbamat-, Fluorenylmethoxycarbonyl- (Fmoc), Carbobenzoxy- (Cbz), Monomethoxytrityl- (Mmt), Phthaloyl-, t-Butoxycarbonyl- (Boc), Benzhydryloxycarbonyl- (Bhoc), oder eine Allyloxycarbonyl-(Alloc) Schutzgruppe.

Der Ausdruck Nukleobasen bezeichnet dem Fachmann bekannte Basen, die zur Basenpaarung mit DNA-Basen oder RNA-Basen fähig sind. Beispiele für Nukleobasen umfassen Basen mit einem Purin-Grundgerüst, z.B. Adenin, Guanin, Hypoxanthin, Xanthin und 7-Methylguanin; oder einem Pyrimidin-Grundgerüst, z.B. Cytosin, Uracil, Thymin, 5-Hydroxymethylcytosin, 5-Methylcytosin, und 5,6-Dihydouracil; sowie Analoga und Bioisostere derselben.

Der Ausdruck Nukleobasen-Schutzgruppe bezeichnet dem Fachmann bekannte Schutzgruppen, die bei der organischen Synthese von Verbindungen mit Nukleobasen eingesetzt werden, beispielsweise eine Acetyl-, Isobutyryl-, Benzyloxycarbonyl-, Diphenylmethyl-, Benzhydryloxycarbonyl-, Anisoyl-, 4-tert.-Butylbenzoyl-, Benzyl- oder Diphenylcarbamoyl-Gruppe.

Der Ausdruck Alkyl bezieht sich auf eine gesättigte, geradkettige oder verzweigte Kohlenwasserstoffgruppe, die 1 bis 40 Kohlenstoffatome, vorzugsweise 1 bis 20 Kohlenstoffatome, stärker bevorzugt 1 bis 12 Kohlenstoffatome, und besonders bevorzugt 1 bis 6 Kohlenstoffatome aufweist; z.B. die Methyl-, Ethyl-, Propyl-, Isopropyl-, n-Butyl-, Isobutyl-, tert-Butyl-, n-Pentyl-, n-Hexyl-, 2,2-Dimethylbutyl- oder n-Octyl-Gruppe.

Die Ausdrücke Alkenyl und Alkinyl beziehen sich auf zumindest teilweise ungesättigte, geradkettige oder verzweigte Kohlenwasserstoffgruppen, die 2 bis 40 Kohlenstoffatome, vorzugsweise 2 bis 20 Kohlenstoffatome, stärker bevorzugt 2 bis 12 Kohlenstoffatome, und besonders bevorzugt 2 bis 6 Kohlenstoffatome aufweisen, z.B. die Ethenyl-,

Allyl-, Acetylenyl-, Propargyl-, Isoprenyl- oder Hex-2-enyl-Gruppe. Bevorzugt weisen Alkenylgruppen eine oder zwei (besonders bevorzugt eine) Doppelbindungen bzw. Alkinylgruppen eine oder zwei (besonders bevorzugt eine) Dreifachbindungen auf.

Der Ausdruck Aryl bzw. Ar bezieht sich auf eine aromatische Gruppe, die einen oder mehrere Ringe aufweist, und 6 bis 14 Ringkohlenstoffatome, vorzugsweise 6 bis 10 (insbesondere 6) Ringkohlenstoffatome enthält. Konkrete Beispiele sind Benzol, Naphtalin oder Biphenyl.

Der Ausdruck Aralkyl bezieht sich auf Gruppen, die entsprechend den obigen Definitionen sowohl Aryl- wie auch Alkyl-, Alkenyl-, Alkinyl- und/oder Cycloalkylgruppen enthalten, wie z.B. Arylalkyl-, Arylalkenyl-, Arylalkinyl-, Arylcycloalkyl-, Arylcycloalkenyl-, Alkylaryl-cycloalkyl- und Alkylarylcycloalkenylgruppen. Konkrete Beispiele für Aralkyle sind Toluol, Xylol, Mesitylen, Styrol, 1H-Inden, Tetralin, Dihydronaphthalin, Indanon, Phenylcyclopentyl, Cyclohexylphenyl, Fluoren und Indan. Bevorzugt enthält eine Aralkylgruppe ein oder zwei aromatische Ringsysteme (1 oder 2 Ringe) mit 6 bis 10 Kohlenstoffatomen und ein oder zwei Alkyl-, Alkenyl- und/oder Alkinylgruppen mit 1 oder 2 bis 6 Kohlenstoffatomen und/oder eine Cycloalkylgruppe mit 5 oder 6 Ringkohlenstoffatomen.

Der Ausdruck Cycloalkyl bezieht sich auf eine gesättigte oder teilweise ungesättigte (z.B. Cycloalkenyl) cyclische Gruppe, die einen oder mehrere Ringe (bevorzugt 1 oder 2) aufweist, und 3 bis 14 Ringkohlenstoffatome, vorzugsweise 3 bis 10 (insbesondere 3, 4, 5, 6 oder 7) Ringkohlenstoffatome enthält. Konkrete Beispiele für Cycloalkylgruppen sind eine Cyclopropyl-, Cyclobutyl-, Cyclopentyl-, Spiro[4,5]decanyl-, Norbornyl-, Cyclohexyl-, Cyclopentenyl-, Cyclohexadienyl-, Decalinyl-, Bicyclo[4.3.0]nonyl-, Tetralin-, Cyclopentylcyclohexyl-, oder eine Cyclohex-2-enyl-Gruppe.

Der Ausdruck Alkylcycloalkyl bezieht sich auf Gruppen, die entsprechend den obigen Definitionen sowohl Cycloalkyl- wie auch Alkyl-, Alkenyl- oder Alkinylgruppen enthalten, z.B. Alkylcycloalkyl-, Cycloalkylalkyl-, Alkylcycloalkenyl-, Alkenylcycloalkyl- und Alkinylcycloalkylgruppen. Bevorzugt enthält eine Alkylcycloalkylgruppe eine Cycloalkylgruppe, die einen oder zwei Ringe aufweist, und 3 bis 14 Ringkohlenstoff¬atome, vorzugsweise 3 bis 10, insbesondere 3, 4, 5, 6 oder 7, Ringkohlenstoffatome enthält; und eine, zwei oder drei, bevorzugt 1 oder 2, Alkyl-, Alkenyl- oder Alkinylgruppe(n) mit jeweils 1 bzw. 2 bis 6 Kohlenstoffatomen; wobei eine $C_4$-$C_{11}$ Alkylcycloalkylgruppe bevorzugt ist, und eine $C_4$-$C_7$ Alkylcycloalkylgruppe besonders bevorzugt ist. Konkrete Beispiele für Alkylcycloalkylgruppen sind eine Methylcyclopropyl ($C_4$)-, Methylcyclobutyl ($C_5$)-, Ethylcyclopropyl ($C_5$)-, Methylcyclopentyl ($C_6$)-, Propylcyclopropyl ($C_6$)-, Ethylcyclopentyl ($C_7$)-, Methylcyclohexyl ($C_7$)-, Ethylcyclopentenyl ($C_7$)-, oder eine Ethylcyclohexadienyl ($C_8$)-Gruppe.

Die Ausdrücke Alkyloxy, Alkenyloxy, Alkinyloxy, Alkyloxyaryl, und Cycloalkyloxy beziehen sich auf eine Alkyl-, Alkenyl-, Alkinyl-, Alkylaryl- bzw. Cycloalkyl-Gruppe, wie vorstehend angegeben, die eine oder mehrere Gruppen -O- enthalten. Beispiele sind eine Methoxy-, Ethoxy-, Furan-, Tetrahydrofuran, oder eine 4-Methoxybenzyl-Gruppe.

Der Ausdruck Heterocyclus bezieht sich auf eine Cycloalkyl-, eine Aryl- oder eine Aralkyl-Gruppe, wie vorstehend angegeben, in der ein oder mehrere, bevorzugt 1, 2 oder 3, Kohlenstoffatome durch ein Sauerstoff-, Stickstoff-, oder Schwefelatom ersetzt sind. Beispiele sind die Piperidyl-, Piperazinyl-, Morpholinyl-, Urotropinyl-, Pyrrolidinyl-, Tetrahydrothiophenyl-, Tetrahydropyranyl-, Tetrahydrofuryl- oder 2-Pyrazolinyl-Gruppe. Der Ausdruck Heterocyclus umfasst beispielsweise auch eine aromatische Gruppe, die einen oder mehrere Ringe aufweist, und 5 bis 14 Ringatome, vorzugsweise 5 bis 10, insbesondere 5 oder 6 Ringatome enthält, wobei ein oder mehrere, bevorzugt 1, 2, 3 oder 4, Sauerstoff-, Stickstoff-, oder Schwefel-Ringatome sind. Beispiele sind die 4-Pyridyl-, 2-Imidazolyl-, 3-Phenylpyrrolyl-, Thiazolyl-, Oxazolyl-, Triazolyl-, Tetrazolyl-, Isoxazolyl-, Indazolyl-, Indolyl-, Benzimidazolyl-, Pyridazinyl-, Chinolinyl-, Purinyl-, Carbazolyl-, Acridinyl-, Pyrimidyl-, 2,3'-Bifuryl-, 3-Pyrazolyl- und Isochinolinyl-Gruppe.

Der Ausdruck Aminosäure bezeichnet eine Carbonsäure, bei der ein oder mehrere Wasserstoff-Atome an einem Kohlenstoffatom durch eine Aminogruppe ersetzt sind. Eine Aminosäure kann beispielsweise eine $\alpha$-Aminosäure wie Glycin, Leucin, Isoleucin, Valin, Alanin, Phenylalanin, Tyrosin, Tryptophan, Asparaginsäure, Asparagin, Glutaminsäure, Glutamin, Cystein, Methionin, Arginin, Lysin, Prolin, Serin, Threonin, Histidin, Selenocystein, Pyrrolysin, Thyroxin, DOPA und L-Ornithin, 5-Hydroxytryptophan, Lanthionine, $\beta$-Chloroalanin, 2-Methylalanin, Citrullin, Canavanin, Theanin, Cucurbitin, eine $\beta$-Aminosäure wie $\beta$-Alanin, oder eine $\gamma$-Aminosäure wie $\gamma$-Aminobuttersäure (GABA) sein.

Ferner umfasst die Erfindung:

[2] Die in [1] beschriebene Verbindung, wobei K -O-$R_M$ darstellt und $R_M$ wie in [1] definiert ist.

[3] Die in [1] oder [2] beschriebene Verbindung, wobei $R_M$ ein H-Atom, eine Methyl-, eine Ethyl-, eine Allyl- oder eine Trimethylsilyl-Gruppe darstellt.

[4] Die in [1] bis [3] beschriebene Verbindung, wobei Pr eine Amino-Schutzgruppe darstellt.

[5] Die in [4] beschriebene Verbindung, wobei die Amino-Schutzgruppe ausgewählt ist aus einer Oxocarbamat-, einer Thiocarbamat- oder einer Mmt-Schutzgruppe.

[6] Die in [4] oder [5] beschriebene Verbindung, wobei die Amino-Schutzgruppe eine Fmoc-, Boc-, Cbz-, Bhoc-,

Alloc- oder eine Mmt-Schutzgruppe ist.

[7] Die in [4] bis [6] beschriebene Verbindung, wobei die Amino-Schutzgruppe eine Boc- oder eine Fmoc-Schutzgruppe ist.

[8] Die in [1] bis [7] beschriebene Verbindung, wobei E eine gegebenenfalls mit einer Nukleobasen-Schutzgruppe substituierte Adeninyl-, Cytosinyl-, Pseudoisocytosinyl-, Guaninyl-, Thyminyl-, Uracilyl- oder Phenyl-Gruppe darstellt.

[9] Die in [8] beschriebene Verbindung, wobei E eine Gruppe darstellt, die ausgewählt ist aus:

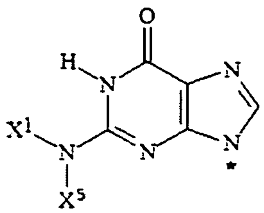
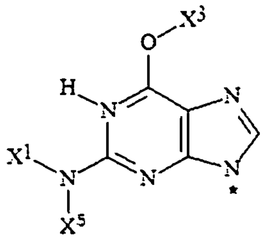
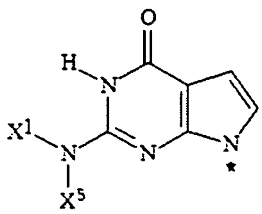
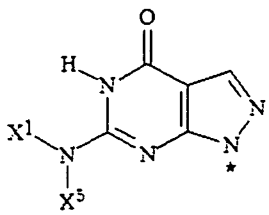
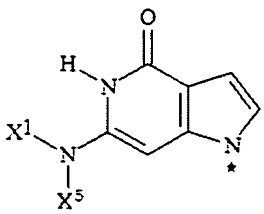
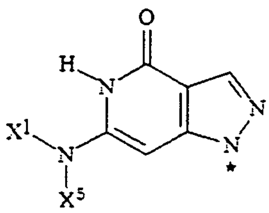
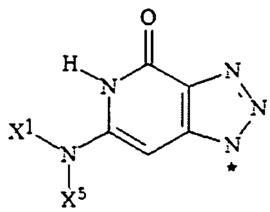
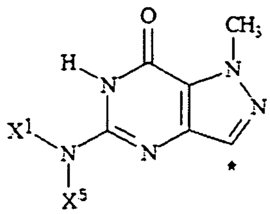
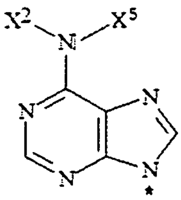
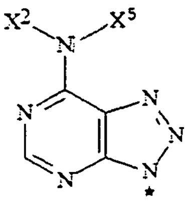
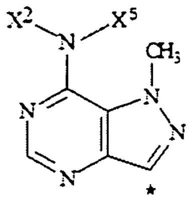
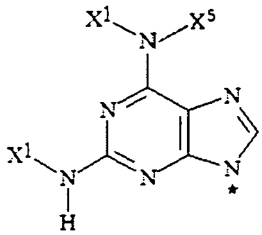
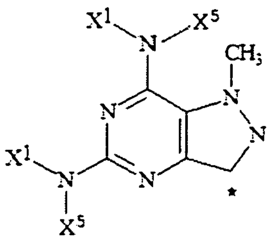
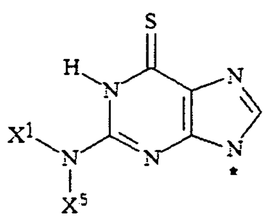
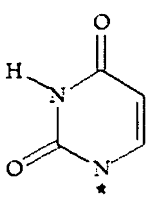
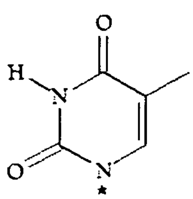
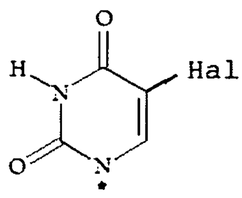
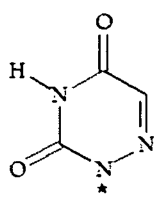
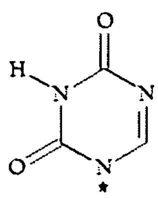

Hal=F, Cl, Br, I

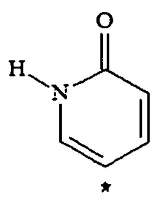 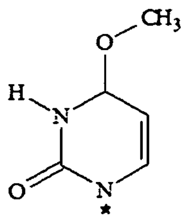 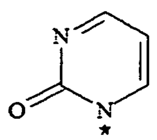 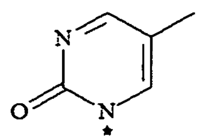 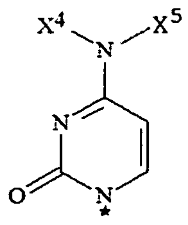

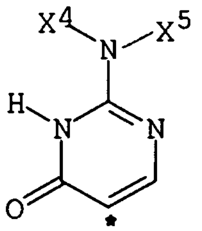 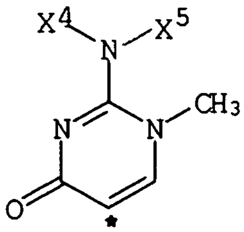 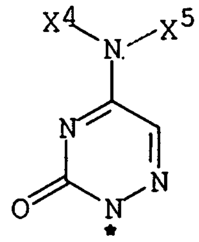 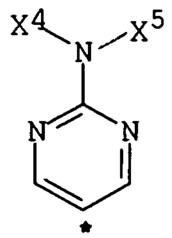

* Substitutionsposition ; wobei
$X^1$ bis $X^4$ jeweils unabhängig ein H-Atom oder eine Nukleobasen- Schutzgruppe darstellen; und $X^5$ jeweils unabhängig ein H-Atom, oder eine Boc- oder Bhoc-Schutzgruppe darstellt.

[10] Die in [9] beschriebene Verbindung, wobei $X^1$, $X^2$, und $X^4$ jeweils unabhängig ein H-Atom, Acetyl (Ac), tert-Butyloxycarbonyl (Boc) Isobutyryl (iBu-CO), Benzyloxycarbonyl (Cbz), (4-Methoxyphenyl)-diphenylmethyl (Mmt), Benzhydryloxycarbonyl (Bhoc), Anisoyl (An), oder 4-tert.-Butylbenzoyl (tBuBz) darstellen;
$X^5$ jeweils unabhängig ein H-Atom, oder eine Boc- oder Bhoc-Schutzgruppe darstellt; und
$X^3$ jeweils unabhängig ein H-Atom, Benzyl (Bn), oder Diphenylcarbamoyl (Dpc) darstellt.

[11] Die in [1] bis [10] beschriebene Verbindung, wobei E eine Thyminyl-, Uracilyl-, Phenyl-, N2-Acetyl-guaninyl-, N2-Isobutyryl-guaninyl-, N2-Benzyloxycar-bonyl-guaninyl-, N2-(4-Methoxyphenyl)-diphenylmethyl-guaninyl-, N2-Benzhydryloxycarbonyl-guaninyl-, N2-Di-benzhydryloxycarbonyl-guaninyl-, N2-tert-Butyloxycarbonyl-guaninyl, N2-Di-tert-butyloxycarbonyl-guaninyl-, N6-Benzyloxycarbonyl-adeninyl-, N6-(4-Methoxyphenyl)-diphenylmethyl-adeninyl-, N6-Anisoyl-adeninyl-, N6-Benzhydryloxycarbonyl-adeninyl-, N6-Di-benzhydryloxycarbonyl-adeninyl-, N6-tert-Butyloxycarbonyl-adeninyl-, N6-Di-tert-butyloxycarbonyl-adeninyl-, O6-Benzylguaninyl- , N2-Acetyl-06-diphenylcar-bamoyl-guaninyl-, N2-Isobutyryl-O6-diphenylcarbamoyl-guaninyl-, N2-Benzyloxycarbonyl-06-diphenylcarbamoyl-guaninyl-, N2-(4-Methoxyphenyl)-diphenylmethyl-O6-diphenylcarbamoyl-guaninyl-, N2-Benzhydryloxycarbonyl-O6-diphenylcarbamoyl-guaninyl-, N4-Benzyloxycarbonyl-cytosinyl-, N4-(4-Methoxyphenyl)-diphenyl-methyl-cytosinyl-, N4-4-tert.Butylbenzoyl-cytosinyl-, N4-Benz-hydryloxycarbonyl-cytosinyl-, N4-Di-benzhydryloxycarbonyl-cytosinyl-, N4-tert-Butyloxycarbonyl-cytosinyl-, N4-Di-tert-butyloxycarbonyl-cytosinyl-, N2-Benzyloxycarbonyl-pseudo-isocytosinyl-, N2-(4-Methoxyphenyl)-diphenylmethyl-pseudoisocyto-sinyl-, N2-4-tert.-Butylbenzoyl-pseudoisocytosinyl-, N2-Benz-hydryloxycarbonyl-pseudoisocytosinyl-, N2-Di-benzhydryloxycarbonyl-pseudoisocytosinyl-, N2-tert-Butyloxycarbonyl-pseudoisocytosinyl- oder eine N2-Di-tert-butyloxycarbonyl-pseudoisocytosinyl-Gruppe darstellt.

[12] Die in [1] bis [11] beschriebene Verbindung, wobei E eine Thyminyl-, Uracilyl-, Phenyl-, N2-Benzyloxycar-bonyl-guaninyl-, N2-Benzhydryloxycarbonyl-guaninyl-, N2-tert-Butyloxycarbonyl-guaninyl-, N2-Benzyloxycarbonyl-06-diphenylcarbamoyl-guaninyl-, N2-Benzhydryloxycarbonyl-06-diphenylcarbamoyl-guaninyl-, N6-Benzyloxycarbonyl-adeninyl-, N6-Benzhydryloxycarbonyl-adeninyl-, N6-tert-Butyloxycarbonyl-adeninyl-, N6-Di-tert-Butyloxycarbonyl-adeninyl-, N4-Benzyloxycarbonyl-cytosinyl-, N4-Benzhydryloxycarbonyl-Cytosinyl-, N4-Di-tert-butyloxycarbonyl-cytosinyl-, N2-Benzyloxycarbonyl-pseudo-isocytosinyl-, N2-Benzhydryloxycarbonyl-pseudoisocytosinyl- oder eine N2-tert-Butyloxycarbonyl-pseudoisocytosinyl-Gruppe darstellt.

[13] Die in [1] bis [12] beschriebene Verbindung, wobei $R^2$ eine Phosphonsäureester-Gruppe der Formel $-P(=O)(OV)_2$ oder $-P(=O)(OV)(OH)$ darstellt; und jedes V unabhängig eine unsubstituierte $C_1$-$C_7$ Alkyl-, $C_3$-$C_7$ Cycloalkyl-, $C_4$-$C_7$ Alkylcycloalkyl-, Phenyl-, oder Benzyl-Gruppe darstellt.

[14] Die in [13] beschriebene Verbindung, wobei jedes V unabhängig eine Methyl-, Ethyl-, Cyclohexyl-, oder Benzyl-Gruppe darstellt.

[15] Die in [14] beschriebene Verbindung, wobei V jeweils eine Ethyl-Gruppe darstellt.

[16] Die in [1] bis [15] beschriebene Verbindung, wobei $R^3$ ein H-Atom ist.

[17] Die in [1] bis [15] beschriebene Verbindung, wobei $R^3$ eine Oxocarbamat-, Thiocarbamat-, oder eine Mmt-Schutzgruppe darstellt.

[18] Die in [17] beschriebene Verbindung, wobei $R^3$ eine Cbz-, Alloc-, Bhoc- oder Boc-Schutzgruppe darstellt.

[19] Die in [1] bis [18] beschriebene Verbindung, wobei m 1, 2, 3 oder 4 ist.

[20] Die in [1] bis [19] beschriebene Verbindung, wobei h 0, 1, 2, oder 3 ist.

[21] Die in [1] bis [20] beschriebene Verbindung, wobei $R^1$ eine Gruppe der Formel $-CH_2-CH_2-CH_2-CH_2-NH-CH_2-CH_2-P{=}O(OEt)_2$, oder eine Gruppe der Formel $-CH_2-CH_2-CH_2-NH-CH_2-CH_2-P{=}O(OEt)_2$ darstellt.

[22] Die in [1] bis [15], [19] oder [20] beschriebene Verbindung, wobei $R^1$ eine Gruppe der Formel $-CH_2-CH_2-CH_2-CH_2-NR^3-CH_2-CH_2-P{=}O(OEt)_2$, oder eine Gruppe der Formel $-CH_2-CH_2-CH_2-NR^3-CH_2-CH_2-P{=}O(OEt)_2$ darstellt; und $R^3$ wie in [17] oder [18] definiert ist.
Erfindungsgemäße Verbindungen der allgemeinen Formel (I), wie sie beispielsweise in [1] bis [22] oben beschreiben sind, können zur Herstellung neuartiger oligomerer Verbindungen genutzt werden. Entsprechend betrifft die Erfindung ferner:

[23] eine Verbindung, umfassend mindestens eine Wiederholungseinheit der allgemeinen Formel (III):

$$-[Y_d-Z_f-Y_g-Z_j]_n- \qquad (III)$$

wobei
jedes Y jeweils unabhängig eine Gruppe der allgemeinen Formel (IV) darstellt:

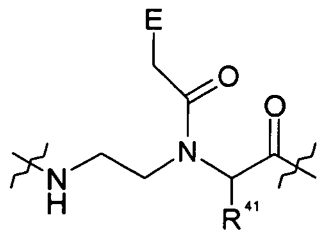

(IV);

jedes Z jeweils unabhängig eine Gruppe der allgemeinen Formel (V) darstellt:

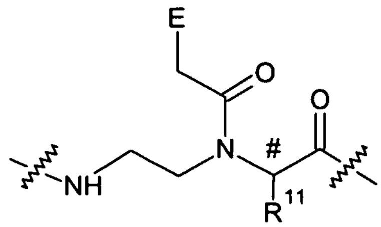

(V);

wobei

jedes E jeweils unabhängig ein H-Atom, eine Phenylgruppe, einen Heterocyclus, oder eine Nukleobase darstellt;

# ein asymmetrisches C-Atom kennzeichnet;

jedes $R^{41}$ jeweils unabhängig ein H-Atom, oder eine Seitenkette der Aminosäure Alanin, Arginin, Asparagin, Asparaginsäure, Cystein, Glutamin, Glutaminsäure, Histidin, Isoleucin, Leucin, Lysin, Methionin, Ornithin, Phenylalanin, Prolin, Histidin, Serin, Threonin, Tryptophan, Tyrosin, oder Valin darstellt;

jedes $R^{11}$ eine Gruppe - $(CH_2)_m$-NH-$(CH_2)_h$-$CH_2$-$R^{12}$ darstellt; wobei $R^{12}$ jeweils eine Phosphonsäureester- oder eine Phosphonsäure-Gruppe ist; m eine ganze Zahl von 1 bis 5 darstellt; und h eine ganze Zahl von 0 bis 4 darstellt; mit der Maßgabe, dass für die Summe von m und h gilt: $2 \leq x \leq 5$;

d jeweils eine ganze Zahl von 0 bis 5 darstellt;

f jeweils eine ganze Zahl von 0 bis 5 darstellt

g jeweils eine ganze Zahl von 0 bis 5 darstellt;

j jeweils eine ganze Zahl von 0 bis 5 darstellt;

n eine ganze Zahl von 1 bis 10 darstellt;

mit der Maßgabe, dass die Summe aller Wiederholungseinheiten $Y_d$, $Z_f$, $Y_g$, und $Z_j$ in der allgemeinen Formel (III) $\leq 40$ ist und mindestens eine der Variablen f oder j eine ganze Zahl von 1 bis 5 darstellt.

Wenn eine Wiederholungseinheit, z.B. die Formel (III), eine Gruppe, z.B. Z oder Y, oder ein Substituent oder eine Variable, z.B. E, $R^{11}$ oder $R^{41}$, mehr als einmal in einer hierin beschriebenen Formel enthalten ist, wird jede Wiederholungseinheit, jede Gruppe in der Wiederholungseinheit, und jeder Substituent oder jede Variable unabhängig voneinander ausgewählt, ob explizit angegeben oder nicht. Beispielsweise wird in der Formel (III) jeweils jede Gruppe Z und Y und jede Variable E, $R^{11}$ oder $R^{41}$ unabhängig voneinander ausgewählt. In anderen Worten, die allgemeine Formel (III) enthält mindestens eine erfindungsgemäße Monomereinheit Z, wie vorstehend, oder beispielsweise in [1] bis [22], definiert, und insgesamt maximal 40 Einheiten Z bzw. Z und Y.
Beispielsweise wird in der allgemeinen Formel (III) jede Gruppe Y und Z, und jede Variable d, f, g und j unabhängig ausgewählt. Entsprechend stellt die Kombination $[Y_d$-$Z_f$-$Y_g$-$Z_j]_n$ mit d, f, g, und j = 1; und n = 10 beispielsweise folgende Kombination der Einheiten Y und Z dar: -[Y-Z-Y-Z-Y-Z-Y-Z-Y-Z-Y-Z-Y-Z-Y-Z-Y-Z-Y-Z-Y-Z-Y-Z-Y-Z-Y-Z-Y-Z-Y-Z-Y-Z-Y-Z-Y-Z-Y-Z]-, wobei die Summe der Anzahl aller Einheiten Y und Z gleich 40 ist. Die Kombination $[Y_d$-$Z_f$-$Y_g$-$Z_j]_n$ mit d=3, f=1, g=1, und j=3; n=3 stellt beispielsweise die folgende Kombination der Einheiten Y und Z dar: -[Y-Y-Y-Z-Y-Z-Z-Z-Y-Y-Y-Z-Y-Z-Z-Z-Y-Y-Y-Z-Y-Z-Z-Z]-, wobei die Summe der Anzahl aller Einheiten Y und Z gleich 24 ist.
Es ist aber auch möglich, dass sich die Variablen d, f, g, und j in den jeweiligen Wiederholungseinheiten $[Y_d$-$Z_f$-$Y_g$-$Z_j]$ voneinander unterscheiden. Beispielsweise könnten folgende 4 Wiederholungseinheiten $[Y_d$-$Z_f$-$Y_g$-$Z_j]$: ($Y_1$-$Z_1$-$Y_1$-$Z_1$), ($Y_1$-$Z_1$-$Y_0$-$Z_0$), ($Y_5$-$Z_1$-$Y_0$-$Z_0$), ($Y_1$-$Z_1$-$Y_1$-$Z_1$), d.h. n=4, zu folgender Kombination der Gruppen Y und Z: -[Y-Z-Y-Z-Y-Z-Y-Y-Y-Y-Y-Z-Y-Z-Y-Z]- kombiniert werden; die Summe der Anzahl aller Gruppen Y und Z ist gleich 16. Gleichermaßen können beispielsweise auch die folgenden 3 (d.h. n=3) Wiederholungseinheiten $[Y_d$-$Z_f$-$Y_g$-$Z_j]$: ($Y_5$-$Z_1$-$Y_1$-$Z_1$), ($Y_1$-$Z_1$-$Y_0$-$Z_0$), und ($Y_1$-$Z_1$-$Y_5$-$Z_0$) miteinander kombiniert werden zu: - [Y-Y-Y-Y-Y-Z-Y-Z-Y-Z-Y-Z-Y-Y-Y-Y-Y]-; die Summe der Anzahl aller Gruppen Y und Z ist gleich 17. Dabei wird jede in den Wiederholungseinheiten enthaltene Gruppe bzw. jeder Substituent oder jede Variable, die mehr als einmal in den Wiederholungseinheiten gemäß der allgemeinen Formel (III) enthalten ist, jeweils unabhängig aus den vorstehenden Definitionen ausgewählt, ob explizit angegeben oder nicht.
Ferner umfasst die Erfindung:

[24] eine Verbindung, dargestellt durch die allgemeine Formel (VI) :

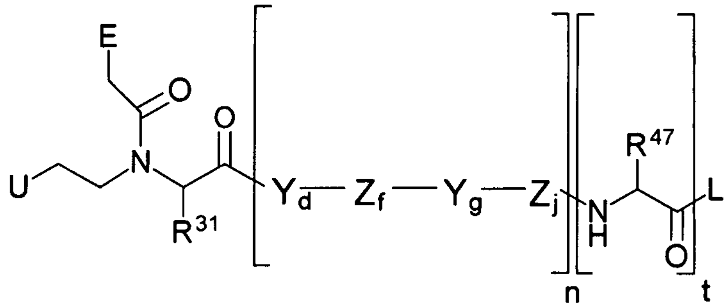

(VI)

wobei

E, Y, Z, d, f, g, j und n jeweils unabhängig wie in [23] definiert sind; mit der Maßgabe, dass die Summe aller Wiederholungseinheiten $Y_d$, $Z_f$, $Y_g$, und $Z_j$ in der allgemeinen Formel (VI) ≤ 40 ist und mindestens eine der Variablen f oder j eine ganze Zahl von 1 bis 5 darstellt;

$R^{31}$ ein H-Atom; eine Seitenkette der Aminosäure Alanin, Arginin, Asparagin, Asparaginsäure, Cystein, Glutamin, Glutaminsäure, Histidin, Isoleucin, Leucin, Lysin, Methionin, Ornithin, Phenylalanin, Prolin, Histidin, Serin, Threonin, Tryptophan, Tyrosin, oder Valin; oder eine Gruppe $-(CH_2)_m-NH-(CH_2)_h-CH_2-R^{12}$ darstellt; wobei $R^{12}$ eine Phosphonsäureester- oder eine Phosphonsäure-Gruppe ist; m eine ganze Zahl von 1 bis 5 darstellt; und h eine ganze Zahl von 0 bis 4 darstellt; mit der Maßgabe, dass für die Summe von m und h gilt: $2 \leq x \leq 5$;

$R^{47}$ jeweils unabhägig ein H-Atom; eine Seitenkette der Aminosäure Alanin, Arginin, Asparagin, Asparaginsäure, Cystein, Glutamin, Glutaminsäure, Histidin, Isoleucin, Leucin, Lysin, Methionin, Ornithin, Phenylalanin, Prolin, Histidin, Serin, Threonin, Tryptophan, Tyrosin, oder Valin; eine Gruppe der Formel (IXb):

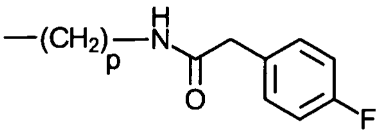

(IXb);

eine Gruppe der Formel (IXc):

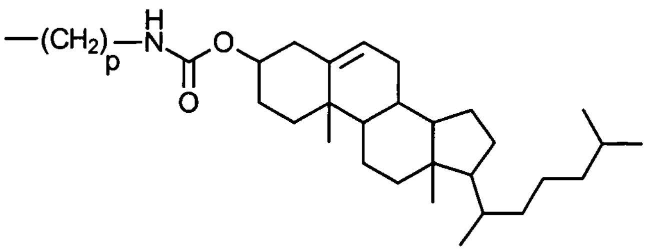

(IXc);

eine Gruppe der Formel (IXd):

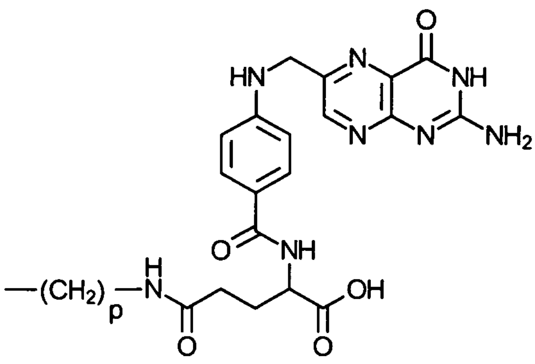

(IXd);

oder eine Gruppe der Formel (IXe):

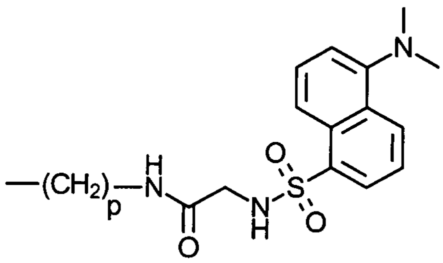

(IXe)

darstellt; p in den Formeln (IXb), (IXc), (IXd), und (IXe) die Zahl 3 oder 4 darstellt;

t eine ganze Zahl von 0 bis 10 darstellt;

L $-NR^DR^E$ $-NHNR^DR^E$ oder $-OR^F$ darstellt; wobei $R^D$, $R^E$ und $R^F$ jeweils unabhängig voneinander ein H-Atom; oder eine Alkyl-, Alkenyl-, Alkinyl-, Aryl-, Aralkyl-, Cycloalkyl-, oder Cycloalkenyl-Gruppe mit jeweils bis zu 20 C-Atomen darstellen;

U $-NR^AR^B$; $-N^{\oplus}R^AR^BR^C$; $-NR^A(CO)R^B$; $-NH\ (CO)\ NHR^B$; $-NH(CO)OR^B$; eine Gruppe der Formel (VIIIa):

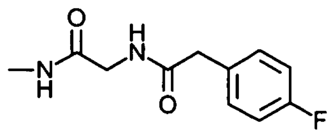

(VIIIa);

eine Gruppe der Formel (VIIIb):

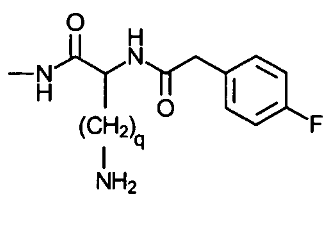

(VIIIb);

eine Gruppe der Formel (VIIIc):

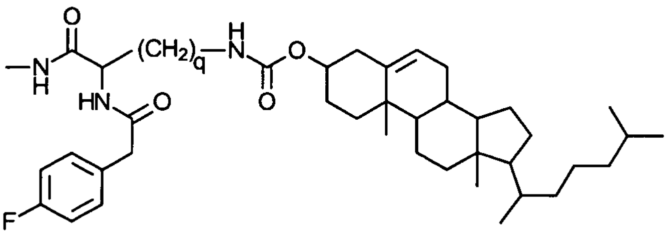

(VIIIc);

eine Gruppe der Formel (VIIId):

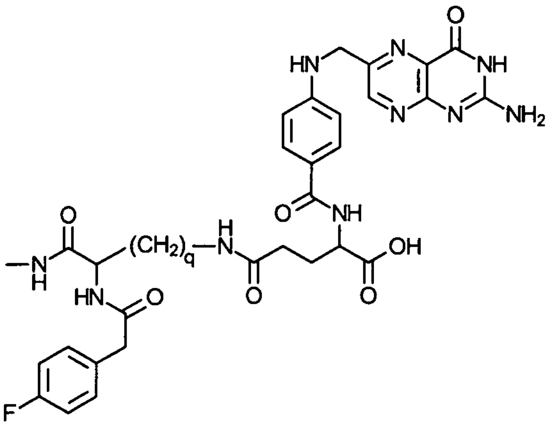

(VIIId);

eine Gruppe der Formel (VIIIe):

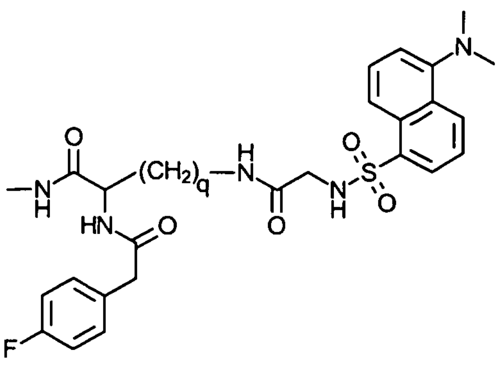

(VIIIe)

darstellt; q in den Formeln (VIIIb), (VIIIc), (VIIId), und (VIIIe) die Zahl 3 oder 4 darstellt;

oder eine Gruppe der allgemeinen Formel (VII):

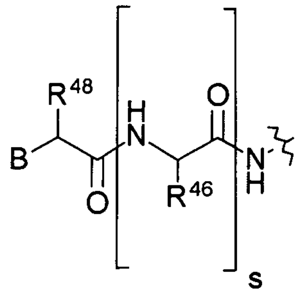

(VII)

darstellt; wobei

B ein H-Atom, $-NR^HR^I$, $-NR^{\oplus}R^HR^IR^J$,$-NR^H(CO)R^I$ -NH(CO)NHR$^I$, -NH(CO)OR$^I$, eine Phenylgruppe, oder eine substituierte Phenylgruppe, die mit 1 bis 3 Substituenten, ausgewählt aus der Gruppe, bestehend aus OH, F, Cl, Br, I und $NO_2$, substituiert ist, darstellt;

jedes $R^A$, $R^C$, $R^H$ und $R^J$ jeweils unabhängig voneinander ein H-Atom, eine Methyl-Gruppe oder eine Amino-Schutzgruppe darstellt; jedes $R^B$ und $R^I$ jeweils unabhängig ein H-Atom; eine Amino-Schutzgruppe; eine Alkyl-, Alkenyl-, Alkinyl-, Aryl-, Aralkyl-, Cycloalkyl-, Alkylcycloalkyl-, Cycloalkenyl-, Alkyloxy-, Alkenyloxy-, Alkinyloxy-, Alkyloxyaryl-, oder eine Cycloalkyloxy-Gruppe mit jeweils bis zu 40 C-Atomen darstellt; wobei in der Alkyl-, Alkenyl-, Alkinyl-, Aryl-, Aralkyl-, Cycloalkyl-, Alkylcycloalkyl-, Cycloalkenyl-, Alkyloxy-, Alkenyloxy-, Alkinyloxy-, Alkyloxyaryl-, oder Cycloalkyloxy-Gruppe ein oder mehrere Wasserstoffatom(e) jeweils unabhängig voneinander durch eine Phosphonsäureester- oder Phosphonsäure-Gruppe, F, Cl, Br, I, -OH, $O-CH_3$, $S-CH_3$, $NO_2$, =O, $NH_2$, $-S(O_2)NH-$, $-NHCH_3$, $-N(CH_3)_2$, eine $C_1$-$C_6$ Alkyl-, $C_2$-$C_6$ Alkenyl-, $C_2$-$C_6$ Alkinyl-, $C_3$-$C_{10}$ Cycloalkyl-, $C_6$-$C_{10}$ Aryl- oder eine $C_7$-$C_{12}$ Aralkyl-Gruppe ersetzt sein kann/können;

$R^{48}$ und jedes $R^{46}$ jeweils unabhängig voneinander ein H-Atom, oder eine Seitenkette der Aminosäure Alanin, Arginin, Asparagin, Asparaginsäure, Cystein, Glutamin, Glutaminsäure, Histidin, Isoleucin, Leucin, Lysin, Methionin, Ornithin, Phenylalanin, Prolin, Histidin, Serin, Threonin, Tryptophan, Tyrosin, oder Valin, oder eine Gruppe der Formel (IXb), (IXc), (IXd) oder (IXe) darstellen; und

s eine ganze Zahl von 0 bis 10 ist.

[25] Die in [23] oder [24] beschriebene Verbindung, mit der Maßgabe, dass die Summe aller Wiederholungseinheiten $Y_d$, $Z_f$, $Y_g$, und $Z_j$ in der allgemeinen Formel (III) oder (VI) $\leq 30$ ist.

[26] Die in [23] bis [25] beschriebene Verbindung, mit der Maßgabe, dass für die Summe aller Wiederholungseinheiten $Y_d$, $Z_f$, $Y_g$, und $Z_j$ in der allgemeinen Formel (III) oder (VI) gilt: $7 \leq x \leq 30$.

[27] Die in [23] bis [26] beschriebene Verbindung, mit der Maßgabe, dass für das Verhältnis (Summe der Wiederholungseinheiten $Z_f$ und $Z_j$): (Summe aller Wiederholungseinheiten $Y_d$, $Z_f$, $Y_g$, und $Z_j$) in der allgemeinen Formel (III) oder (VI) gilt: $0{,}1 \leq x \leq 1{,}0$.

[28] Die in [23] bis [26] beschriebene Verbindung, mit der Maßgabe, dass für das Verhältnis (Summe der Wiederholungseinheiten $Z_f$ und $Z_j$) : (Summe aller Wiederholungseinheiten $Y_d$, $Z_f$, $Y_g$, und $Z_j$) in der allgemeinen Formel (III) oder (VI) gilt: $0{,}1 \leq x \leq 0{,}8$.

[29] Die in [23] bis [26] beschriebene Verbindung, mit der Maßgabe, dass für das Verhältnis (Summe der Wiederholungseinheiten $Z_f$ und $Z_j$): (Summe aller Wiederholungseinheiten $Y_d$, $Z_f$, $Y_g$, und $Z_j$) in der allgemeinen Formel (III) oder (VI) gilt: $0{,}1 \leq x \leq 0{,}6$.

[30] Die in [23] bis [26] beschriebene Verbindung, mit der Maßgabe, dass für das Verhältnis (Summe der Wiederholungseinheiten $Z_f$ und $Z_j$): (Summe aller Wiederholungseinheiten $Y_d$, $Z_f$, $Y_g$, und $Z_j$) in der allgemeinen Formel (III) oder (VI) gilt: $0{,}1 \leq x \leq 0{,}5$.

[31] Die in [23] bis [26] beschriebene Verbindung, mit der Maßgabe, dass für das Verhältnis (Summe der Wieder-

holungseinheiten $Z_f$ und $Z_j$): (Summe aller Wiederholungseinheiten $Y_d$, $Z_f$, $Y_g$, und $Z_j$) in der allgemeinen Formel (III) oder (VI) gilt: $0{,}1 \leq x \leq 0{,}4$.

[32] Die in [23] bis [31] beschriebene Verbindung, wobei jedes E jeweils unabhängig eine Adeninyl-, Cytosinyl-, Pseudoisocytosinyl-, Guaninyl-, Thyminyl-, Uracilyl-, oder Phenyl-Gruppe darstellt.

[33] Die in [23] bis [32] beschriebene Verbindung, wobei jedes $R^{41}$ jeweils unabhängig ein H-Atom, oder eine Seitenkette der Aminosäure Lysin, Ornithin, Arginin, Histidin, Tryptophan, Tyrosin, Threonin oder Serin darstellt.

[34] Die in [23] bis [33] beschriebene Verbindung, wobei jedes $R^{41}$ jeweils unabhängig ein H-Atom, oder eine Seitenkette der Aminosäure Lysin, Ornithin, oder Arginin darstellt.

[35] Die in [23] bis [34] beschriebene Verbindung, wobei jedes $R^{41}$ jeweils ein H-Atom darstellt.

[36] Die in [23] bis [34] beschriebene Verbindung, wobei jedes $R^{12}$ jeweils unabhängig eine Phosphonsäureester-Gruppe der Formel $-P(=O)(OV)_2$ oder $-P(=O)(OV)(OH)$ darstellt; und jedes V jeweils unabhängig eine unsubstituierte $C_1$-$C_7$ Alkyl-, $C_3$-$C_7$ Cycloalkyl-, $C_4$-$C_7$ Alkylcycloalkyl-, Phenyl-, oder Benzyl-Gruppe darstellt.

[37] Die in [36] beschriebene Verbindung, wobei jedes V jeweils unabhängig eine Methyl-, Ethyl-, Cyclohexyl-, oder Benzyl-Gruppe darstellt.

[38] Die in [36] oder [37] beschriebene Verbindung, wobei V jeweils eine Ethyl-Gruppe darstellt.

[39] Die in [23] bis [38] beschriebene Verbindung, wobei jedes m jeweils unabhängig 1, 2, 3 oder 4 ist.

[40] Die in [23] bis [39] beschriebene Verbindung, wobei jedes h jeweils unabhängig 0, 1, 2, oder 3 ist.

[41] Die in [23] bis [40] beschriebene Verbindung, wobei jedes $R^{11}$ jeweils eine Gruppe der Formel $-CH_2-CH_2-CH_2-CH_2-NH-CH_2-CH_2-P=O(OEt)_2$, oder eine Gruppe der Formel $-CH_2-CH_2-CH_2-NH-CH_2-CH_2-P=O(OEt)_2$ darstellt.

[42] Die in [23] bis [41] beschriebene Verbindung, wobei jedes d jeweils unabhängig 0, 1, 2, 3 oder 4 ist

[43] Die in [23] bis [42] beschriebene Verbindung, wobei jedes f jeweils unabhängig 0, 1, 2, 3 oder 4 ist.

[44] Die in [23] bis [43] beschriebene Verbindung, wobei jedes g jeweils unabhängig 0, 1, 2, 3 oder 4 ist.

[45] Die in [23] bis [44] beschriebene Verbindung, wobei jedes j jeweils unabhängig 0, 1, 2, 3 oder 4 ist.

[46] Die in [23] bis [45] beschriebene Verbindung, wobei n = 0, 1, 2, 3, 4, 5, 6, 7 oder 8 ist.

[47] Die in [24] bis [46] beschriebene Verbindung, wobei $R^{31}$ ein H-Atom, eine Seitenkette der Aminosäure Lysin, Ornithin, Arginin, Histidin, Tryptophan, Tyrosin, Threonin oder Serin, eine Gruppe der Formel $-CH_2-CH_2-CH_2-CH_2-NH-CH_2-CH_2-P=O(OEt)_2$, oder eine Gruppe der Formel $-CH_2-CH_2-CH_2-NH-CH_2-CH_2-P=O(OEt)_2$ darstellt.

[48] Die in [24] bis [47] beschriebene Verbindung, wobei $R^{31}$ eine Gruppe der Formel $-CH_2-CH_2-CH_2-CH_2-NH-CH_2-CH_2-P=O(OEt)_2$, oder eine Gruppe der Formel $-CH_2-CH_2-CH_2-NH-CH_2-CH_2-P=O(OEt)_2$ darstellt.

[49] Die in [24] bis [47] beschriebene Verbindung, wobei $R^{31}$ ein H-Atom, oder eine Seitenkette der Aminosäure Lysin, Ornithin, oder Arginin darstellt.

[50] Die in [24] bis [47] oder [49] beschriebene Verbindung, wobei $R^{31}$ ein H-Atom darstellt.

[51] Die in [24] bis [50] beschriebene Verbindung, wobei $R^{47}$ jeweils unabhängig ein H-Atom; eine Seitenkette der Aminosäure Lysin, Ornithin, Arginin, Histidin, Tryptophan, Tyrosin, Threonin oder Serin; oder eine Gruppe der Formel (IXb), (IXc), (IXd) oder (IXe) darstellt.

[52] Die in [24] bis [51] beschriebene Verbindung, wobei $R^{47}$ jeweils unabhängig ein H-Atom; oder eine Seitenkette der Aminosäure Lysin, Ornithin, oder Arginin darstellt.

[53] Die in [52] beschriebene Verbindung, wobei $R^{47}$ jeweils ein H-Atom darstellt.

[54] Die in [52] beschriebene Verbindung, wobei $R^{47}$ jeweils unabhängig eine Seitenkette der Aminosäure Lysin, Ornithin, oder Arginin darstellt.

[55] Die in [24] bis [54] beschriebene Verbindung, wobei t = 0, 1, 2, 3, 4, 5, 6, 7 oder 8 ist.

[56] Die in [24] bis [51] beschriebene Verbindung, wobei $R^{47}$ jeweils unabhängig ein H-Atom; oder eine Gruppe der Formel (IXb), (IXc), (IXd) oder (IXe) darstellt; und t = 1, 2, 3, oder 4 ist.

[57] Die in [56] beschriebene Verbindung, wobei $R^{47}$ jeweils unabhängig eine Gruppe der Formel (IXb), (IXc), (IXd) oder (IXe) darstellt.

[58] Die in [24] bis [57] beschriebene Verbindung, wobei L -OH, - $NH_2$, -$NHNH_2$, eine -O($C_1$-$C_{10}$)Alkyl-, -O($C_2$-$C_{10}$)Alkenyl-, -O($C_2$-$C_{10}$)Alkinyl-, -O($C_3$-$C_{10}$) Cycloalkyl-, -O($C_4$-$C_{11}$)Alkylcycloalkyl-, - O($C_6$-$C_{10}$) Aryl-, -O($C_7$-$C_{12}$) Aralkyl, -NH- ($C_1$-$C_{10}$) Alkyl-, -NH($C_2$-$C_{10}$) Alkenyl-, -NH ($C_2$-$C_{10}$) Cycloalkenyl-, -NH ($C_3$-$C_{10}$) Cycloalkyl-,-NH($C_6$-$C_{10}$)Aryl-, oder eine -NH($C_7$-$C_{12}$)Aralkyl-Gruppe darstellt.

[59] Die in [24] bis [58] beschriebene Verbindung, wobei L -OH, -OEt, -$NH_2$ oder -$NHNH_2$ darstellt.

[60] Die in [24] bis [59] beschriebene Verbindung, wobei U -$NR^AR^B$; - $NR^A$(CO)$R^B$; -NH(CO)$NHR^B$; oder -NH(CO)$OR^B$ darstellt; $R^A$ jeweils ein H-Atom oder eine Methyl-Gruppe darstellt; und $R^B$ wie in [24] definiert ist.

[61] Die in [60] beschriebene Verbindung, wobei $R^B$ jeweils ein H-Atom, eine Alkyl-, Alkenyl-, Alkinyl-, Aryl-, Aralkyl-, Cycloalkyl-, Alkylcycloalkyl-, Cycloalkenyl-, Alkyloxy-, Alkenyloxy-, Alkinyloxy-, Alkyloxyaryl-, oder eine Cycloalkyloxy-Gruppe mit jeweils bis zu 30 C-Atomen darstellt; wobei in der Alkyl-, Alkenyl-, Alkinyl-, Aryl-, Aralkyl-, Cycloalkyl-, Alkylcycloalkyl-, Cycloalkenyl-, Alkyloxy-, Alkenyloxy-, Alkinyloxy-, Alkyloxyaryl-, oder Cycloalkyloxy-Gruppe ein oder mehrere Wasserstoffatom(e) jeweils unabhängig voneinander durch eine Phosphonsäureester- oder Phosphonsäure-Gruppe, F, Cl, Br, I, -OH, oder $NO_2$ ersetzt sein kann/können.

[62] Die in [60] beschriebene Verbindung, wobei $R^B$ jeweils ein H-Atom, eine Alkyl-, Alkenyl-, Alkinyl-, Aryl-, Aralkyl-, Cycloalkyl-, Alkylcycloalkyl-, Cycloalkenyl-, Alkyloxy-, Alkenyloxy-, Alkinyloxy-, Alkyloxyaryl-, oder eine Cycloalkyloxy-Gruppe mit jeweils bis zu 20 C-Atomen darstellt; wobei in der Alkyl-, Alkenyl-, Alkinyl-, Aryl-, Aralkyl-, Cycloalkyl-, Alkylcycloalkyl-, Cycloalkenyl-, Alkyloxy-, Alkenyloxy-, Alkinyloxy-, Alkyloxyaryl-, oder Cycloalkyloxy-Gruppe ein oder mehrere Wasserstoffatom(e) jeweils unabhängig voneinander durch eine Phosphonsäureester- oder Phosphonsäure-Gruppe, F, Cl, Br, I, -OH, oder $NO_2$ ersetzt sein kann/können.

[63] Die in [60] beschriebene Verbindung, wobei $R^B$ jeweils ein H-Atom, eine Alkyl-, Alkenyl-, Alkinyl-, Aryl-, Aralkyl-, Cycloalkyl-, Alkylcycloalkyl-, Cycloalkenyl-, Alkyloxy-, Alkenyloxy-, Alkinyloxy-, Alkyloxyaryl-, oder eine Cycloalkyloxy-Gruppe mit jeweils bis zu 12 C-Atomen darstellt; wobei in der Alkyl-, Alkenyl-, Alkinyl-, Aryl-, Aralkyl-, Cycloalkyl-, Alkylcycloalkyl-, Cycloalkenyl-, Alkyloxy-, Alkenyloxy-, Alkinyloxy-, Alkyloxyaryl-, oder Cycloalkyloxy-Gruppe ein oder mehrere Wasserstoffatom(e) jeweils unabhängig voneinander durch eine Phosphonsäureester- oder Phosphonsäure-Gruppe, F, Cl, Br, I, -OH, oder $NO_2$ ersetzt sein kann/können.

[64] Die in [24] bis [59] beschriebene Verbindung, wobei U eine Gruppe der allgemeinen Formel (VII) darstellt; B -$NR^HR^I$, -$NR^H$(CO)$R^I$, -NH(CO)$NHR^I$, oder -NH(CO)$OR^I$ darstellt; $R^{48}$ ein H-Atom ist; jedes $R^{46}$ jeweils unabhängig voneinander ein H-Atom, oder eine Seitenkette der Aminosäure Alanin, Arginin, Asparagin, Asparaginsäure, Cystein, Glutamin, Glutaminsäure, Histidin, Isoleucin, Leucin, Lysin, Methionin, Ornithin, Phenylalanin, Prolin, Histidin, Serin, Threonin, Tryptophan, Tyrosin, oder Valin darstellt; und $R^H$ und $R^I$ wie in [24] definiert sind.

[65] Die in [24] bis [59] beschriebene Verbindung, wobei U eine Gruppe der allgemeinen Formel (VII) darstellt; B -$NR^HR^I$, -$NR^H$(CO)$R^I$, -NH(CO)$NHR^I$, oder -NH(CO)$OR^I$ darstellt; $R^{48}$ eine Gruppe der Formel (IXb) bis (IXe) darstellt; jedes $R^{46}$ jeweils unabhängig voneinander ein H-Atom, oder eine Seitenkette der Aminosäure Alanin, Arginin, Asparagin, Asparaginsäure, Cystein, Glutamin, Glutaminsäure, Histidin, Isoleucin, Leucin, Lysin, Methionin, Ornithin, Phenylalanin, Prolin, Histidin, Serin, Threonin, Tryptophan, Tyrosin, oder Valin darstellt; und $R^H$ und $R^I$ wie in [24]

definiert sind.

[66] Die in [64] oder [65] beschriebene Verbindung, wobei $R^H$ ein H-Atom oder eine Methyl-Gruppe darstellt.

[67] Die in [64] bis [66] beschriebene Verbindung, wobei $R^I$ ein H-Atom, eine Alkyl-, Alkenyl-, Alkinyl-, Aryl-, Aralkyl-, Cycloalkyl-, Alkylcycloalkyl-, Cycloalkenyl-, Alkyloxy-, Alkenyloxy-, Alkinyloxy-, Alkyloxyaryl-, oder eine Cycloalkyloxy-Gruppe mit jeweils bis zu 30 C-Atomen darstellt; wobei in der Alkyl-, Alkenyl-, Alkinyl-, Aryl-, Aralkyl-, Cycloalkyl-, Alkylcycloalkyl-, Cycloalkenyl-, Alkyloxy-, Alkenyloxy-, Alkinyloxy-, Alkyloxyaryl-, oder Cycloalkyloxy-Gruppe ein oder mehrere Wasserstoffatom(e) jeweils unabhängig voneinander durch eine Phosphonsäureester- oder Phosphonsäure-Gruppe, F, Cl, Br, I, -OH, oder $NO_2$ ersetzt sein kann/können.

[68] Die in [64] bis [66] beschriebene Verbindung, wobei $R^I$ ein H-Atom, eine Alkyl-, Alkenyl-, Alkinyl-, Aryl-, Aralkyl-, Cycloalkyl-, Alkylcycloalkyl-, Cycloalkenyl-, Alkyloxy-, Alkenyloxy-, Alkinyloxy-, Alkyloxyaryl-, oder eine Cycloalkyloxy-Gruppe mit jeweils bis zu 20 C-Atomen darstellt; wobei in der Alkyl-, Alkenyl-, Alkinyl-, Aryl-, Aralkyl-, Cycloalkyl-, Alkylcycloalkyl-, Cycloalkenyl-, Alkyloxy-, Alkenyloxy-, Alkinyloxy-, Alkyloxyaryl-, oder Cycloalkyloxy-Gruppe ein oder mehrere Wasserstoffatom(e) jeweils unabhängig voneinander durch eine Phosphonsäureester- oder Phosphonsäure-Gruppe, F, Cl, Br, I, -OH, oder $NO_2$ ersetzt sein kann/können.

[69] Die in [64] bis [66] beschriebene Verbindung, wobei $R^I$ ein H-Atom, eine Alkyl-, Alkenyl-, Alkinyl-, Aryl-, Aralkyl-, Cycloalkyl-, Alkylcycloalkyl-, Cycloalkenyl-, Alkyloxy-, Alkenyloxy-, Alkinyloxy-, Alkyloxyaryl-, oder eine Cycloalkyloxy-Gruppe mit jeweils bis zu 12 C-Atomen darstellt; wobei in der Alkyl-, Alkenyl-, Alkinyl-, Aryl-, Aralkyl-, Cycloalkyl-, Alkylcycloalkyl-, Cycloalkenyl-, Alkyloxy-, Alkenyloxy-, Alkinyloxy-, Alkyloxyaryl-, oder Cycloalkyloxy-Gruppe ein oder mehrere Wasserstoffatom(e) jeweils unabhängig voneinander durch eine Phosphonsäureester- oder Phosphonsäure-Gruppe, F, Cl, Br, I, -OH, oder $NO_2$ ersetzt sein kann/können.

[70] Die in [24] bis [59] beschriebene Verbindung, wobei U eine Gruppe der allgemeinen Formel (VII) darstellt; B ein H-Atom, eine Phenylgruppe, oder eine substituierte Phenylgruppe, die mit 1 bis 3 Substituenten, ausgewählt aus der Gruppe, bestehend aus OH, F, Cl, Br, I und $NO_2$, substituiert ist, darstellt; $R^{48}$ ein H-Atom ist; und jedes $R^{46}$ jeweils unabhängig voneinander ein H-Atom, oder eine Seitenkette der Aminosäure Alanin, Arginin, Asparagin, Asparaginsäure, Cystein, Glutamin, Glutaminsäure, Histidin, Isoleucin, Leucin, Lysin, Methionin, Ornithin, Phenylalanin, Prolin, Histidin, Serin, Threonin, Tryptophan, Tyrosin, oder Valin darstellt.

[71] Die in [64] bis [70] beschriebene Verbindung, wobei jedes $R^{46}$ jeweils unabhängig voneinander ein H-Atom, oder eine Seitenkette der Aminosäure Alanin, Arginin, Asparagin, Glutamin, Histidin, Isoleucin, Leucin, Lysin, Methionin, Ornithin, Phenylalanin, Prolin, Histidin, Serin, Threonin, Tryptophan, Tyrosin, oder Valin darstellt.

[72] Die in [64] bis [70] beschriebene Verbindung, wobei jedes $R^{46}$ jeweils unabhängig voneinander ein H-Atom, oder eine Seitenkette der Aminosäure Arginin, Histidin, Lysin, Methionin, Ornithin, Histidin, Serin, Threonin, Tryptophan oder Tyrosin darstellt.

[73] Die in [64] bis [72] beschriebene Verbindung, wobei s = 0, 1, 2, 3, 4, 5, 6, 7 oder 8 ist.

[74] Die in [24] bis [59] beschriebene Verbindung, wobei U eine Gruppe der allgemeinen Formel (VII) darstellt; B ein H-Atom, eine Phenylgruppe, oder eine substituierte Phenylgruppe, die mit 1 bis 3 Substituenten, ausgewählt aus der Gruppe, bestehend aus OH, F, Cl, Br, I und $NO_2$, substituiert ist, darstellt; $R^{48}$ ein H-Atom ist; jedes $R^{46}$ jeweils unabhängig voneinander ein H-Atom, oder eine Gruppe der Formel (IXb) bis (IXe) darstellt; und s = 1, 2, 3 oder 4 ist.

[75] Die in [74] beschriebene Verbindung, wobei $R^{46}$ jeweils unabhängig eine Gruppe der Formel (IXb), (IXc), (IXd) oder (IXe) darstellt.

[76] Die in [24] bis [59] beschriebene Verbindung, wobei U eine Gruppe der Formel (VIIIa) bis (VIIIe) darstellt.

[77] Die in [24] bis [46] beschriebene Verbindung, wobei $R^{31}$ ein H-Atom, oder eine Gruppe $-(CH_2)_m-NH-(CH_2)_h-CH_2-R^{12}$ darstellt, wobei $R^{12}$ eine Phosphonsäureester-Gruppe der Formel $-P(=O)(OV)_2$ oder $P(=O)(OV)(OH)$ darstellt; jedes V unabhängig eine Methyl-, Ethyl-, Cyclohexyl-, oder Benzyl-Gruppe ist; m 1, 2, 3 oder 4 ist; und h 0, 1, 2, oder 3 ist; mit der Maßgabe, dass für die Summe von m und h gilt: $2 \leq x \leq 5$;

[78] Die in [77] beschriebene Verbindung, wobei $R^{47}$ jeweils unabhängig ein H-Atom; oder eine Seitenkette der Aminosäure Lysin, Ornithin, oder Arginin ist;

t = 0, 1, 2, 3, 4, 5, 6, 7 oder 8 ist;
L OH, OEt, $NH_2$ oder $-NHNH_2$ darstellt;
U eine Gruppe der allgemeinen Formel (VII) darstellt:

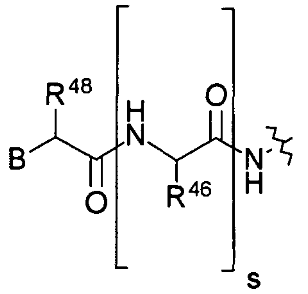

(VII)

wobei B ein H-Atom, eine Phenylgruppe, oder eine substituierte Phenylgruppe, die mit 1 bis 3 Substituenten, ausgewählt aus der Gruppe, bestehend aus OH, F, Cl, Br, I und $NO_2$, substituiert ist, darstellt; $R^{48}$ ein H-Atom ist; und

(i) jedes $R^{46}$ jeweils unabhängig voneinander ein H-Atom, oder eine Seitenkette der Aminosäure Alanin, Arginin, Asparagin, Asparaginsäure, Cystein, Glutamin, Glutaminsäure, Histidin, Isoleucin, Leucin, Lysin, Methionin, Ornithin, Phenylalanin, Prolin, Histidin, Serin, Threonin, Tryptophan, Tyrosin, oder Valin darstellt; und s 0, 1, 2, 3, 4, 5, 6, 7 oder 8 ist; oder

(ii) jedes $R^{46}$ jeweils unabhängig voneinander ein H-Atom, oder eine Gruppe der Formel (IXb) bis (IXe) darstellt; und s = 1, 2, 3 oder 4 ist.

[79] Die in [24], [25], und [27] bis [78] beschriebene Verbindung, mit der Maßgabe, dass für die Summe aller Wiederholungseinheiten $Y_d$, $Z_f$, $Y_g$, und $Z_j$ in der allgemeinen Formel (VI) gilt: $7 \leq x \leq 25$.

[80] Die in [24], [25], und [27] bis [78] beschriebene Verbindung, mit der Maßgabe, dass für die Summe aller Wiederholungseinheiten $Y_d$, $Z_f$, $Y_g$, und $Z_j$ in der allgemeinen Formel (III) oder (VI) gilt: $7 \leq x \leq 22$.

[81] Die in [77] bis [80] beschriebene Verbindung, wobei jedes $R^{11}$ jeweils eine Gruppe der Formel $-CH_2-CH_2-CH_2-CH_2-NH-CH_2-CH_2-P{=}O(OEt)_2$, oder eine Gruppe der Formel $-CH_2-CH_2-CH_2-NH-CH_2-CH_2-P{=}O(OEt)_2$ darstellt.

[82] Die in [77] bis [81] beschriebene Verbindung, wobei jedes $R^{31}$ jeweils ein H-Atom, oder eine Gruppe der Formel $-CH_2-CH_2-CH_2-CH_2-NH-CH_2-CH_2-P{=}O(OEt)_2$, oder eine Gruppe der Formel $-CH_2-CH_2-CH_2-NH-CH_2-CH_2-P{=}O(OEt)_2$ darstellt.

In den Verbindungen der allgemeinen Formel (III) und (VI), wie sie vorstehend in [23] bis [76] bzw. [77] bis [82] beschrieben sind, ist mindestens eine Gruppe Z enthalten. Entsprechend weisen die in [23] bis [76] bzw. [77] bis [82] beschriebenen erfindungsgemäßen Verbindungen der allgemeinen Formel (III) und (IV) an der Bindungsstelle von $R^{11}$ mit dem Grundgerüst mindestens 1 asymmetrisches Zentrum (#) auf. Bevorzugt besitzt dieses asymmetrische Zentrum (#) an der Bindungsstelle von $R^{11}$ mit dem Grundgerüst die R-Konfiguration.

Sind in den Verbindungen der allgemeinen Formel (III) und (VI), wie sie vorstehend in [23] bis [76] bzw. [77] bis [82] beschrieben sind, zwei oder mehr dieser asymmetrischen Zentren (#) vorhanden, können mindestens 50% der asymmetrischen Zentren (#), bevorzugt 66%, 70%, 75% oder 80%, stärker bevorzugt 85%, 90% oder 95%, und am stärksten bevorzugt 100% die R-Konfiguration aufweisen.

Alternativ können in den Verbindungen der allgemeinen Formel (III) und (VI) mit zwei oder mehr asymmetrischen Zentren (#), wie sie beispielsweise in [23] bis [76] bzw. [77] bis [82] beschrieben sind, mindestens 50%, bevorzugt 66%, 70%, 75% oder 80%, stärker bevorzugt 85%, 90% oder 95%, und am stärksten bevorzugt 100% dieser asymmetrischen Zentren (#) die S-Konfiguration aufweisen.

Ferner wird erfindungsgemäß eine pharmazeutische Zusammensetzung offenbart, die mindestens eine (oder

mehrere) erfindungsgemäße oligomere Verbindung(en), und fakultativ mindestens einen Trägerstoff, gegebenenfalls in Kombination mit üblichen pharmakologisch tolerierten Hilfs- und/oder Füllstoffen, und/oder mindestens ein Adjuvanz enthält.

Auch die Verwendung einer erfindungsgemäßen oligomeren Verbindung als Medikament oder Arzneimittel ist Gegenstand der vorliegenden Erfindung. Im Allgemeinen werden erfindungsgemäße Verbindungen unter Anwendung der bekannten und akzeptablen Modi, entweder einzeln oder in Kombination mit einem beliebigen anderen therapeutischen Mittel verabreicht. Die Verabreichung kann z.B. auf einem der folgenden Wege erfolgen: oral, z.B. als Dragees, überzogene Tabletten, Pillen, Halbfeststoffe, weiche oder harte Kapseln, Lösungen, Emulsionen oder Suspensionen; parenteral, z.B. als injizierbare Lösung; rektal als Suppositorien; durch Inhalation, z.B. als Pulverformulierung oder Spray, transdermal oder intranasal. Zur Herstellung solcher Tabletten, Pillen, Halbfeststoffe, überzogenen Tabletten, Dragees und harten Gelatinekapseln kann das therapeutisch verwendbare Produkt mit pharmakologisch inerten, anorganischen oder organischen Arzneimittelträgersubstanzen vermischt werden, z.B. mit Lactose, Sucrose, Glucose, Gelatine, Malz, Silicagel, Stärke oder Derivaten derselben, Talkum, Stearinsäure oder ihren Salzen, Trockenmagermilch und dgl. Zur Herstellung von weichen Kapseln kann man Arzneimittelträgerstoffe wie z.B. pflanzliche Öle, Petroleum, tierische oder synthetische Öle, Wachse, Fette, Polyole einsetzen. Zur Herstellung von flüssigen Lösungen und Sirups kann man Arzneimittelträgerstoffe wie z.B. Wasser, Alkohole, wässrige Salzlösung, wässrige Dextrose, Polyole, Glycerin, pflanzliche Öle, Petroleum, tierische oder synthetische Öle verwenden. Für Suppositorien kann man Arzneimittelträgerstoffe wie z.B. pflanzliche Öle, Petroleum, tierische oder synthetische Öle, Wachse, Fette und Polyole verwenden. Für Aerosol-Formulierungen kann man komprimierte Gase, die für diesen Zweck geeignet sind, wie z.B. Sauerstoff, Stickstoff, Fluorchlorkohlenwasserstoffe, Fluorkohlenwasserstoffe, Chlorkohlenwasserstoffe und Kohlendioxid einsetzen. Die pharmazeutisch verwendbaren Mittel können auch Zusatzstoffe zur Konservierung, Stabilisierung, Emulgatoren, Süßstoffe, Aromastoffe, Salze zur Veränderung des osmotischen Drucks, Puffer, Umhüllungszusatzstoffe und Antioxidantien enthalten.

Durch ihre Fähigkeit, an komplementäre Nukleinsäuresequenzen zu binden, kann sich eine erfindungsgemäße oligomere Verbindung oder pharmazeutische Zusammensetzung zur Verwendung bei der Vorbeugung und/oder Behandlung von vielen verschiedenen Erkrankungen eignen. Beispiele für solche Erkrankungen, denen mit den erfindungsgemäßen oligomeren Verbindungen vorgebeugt werden kann, oder die mit diesen behandelt werden können, sind, zum Beispiel: Krankheiten, verursacht durch Viren, wie beispielsweise humanes Immundefizienz-Virus (HIV), Hepatitis B-Virus und Hepatitis C-Virus oder humane Papillomviren (HPV); Krebs, wie beispielsweise Hautkrebs, Lungenkrebs, Leberkrebs, Prostatakrebs, Leukämie, oder Gehirntumore; seltene neuromuskuläre Erkrankungen, wie beispielsweise Muskeldystrophie vom Typ Duchenne oder spinale Muskelatrophie; Entzündungskrankheiten wie beispielsweise Asthma, rheumatoide Arthritis, oder Schuppenflechte; Autoimmunerkrankungen, wie beispielsweise Morbus Crohn oder Multiple Sklerose; neurologische Krankheiten, wie beispielsweise Parkinson; oder metabolische Krankheiten, wie beispielsweise erhöhte Cholesterin-Werte oder Fettleibigkeit.

Die erfindungsgemäßen oligomeren Verbindungen, d.h. oligomere Verbindungen der allgemeinen Formel (III) oder (VI) (hierin auch als N-Phos-Oligomere bezeichnet), zeigen im Vergleich zu den aus EP 2041161 bekannten oligomeren Verbindungen mit Alkyl-Phosphonsäureester-Gruppen (im Folgenden bezeichnet als: EP2041161-Oligomere) überraschende und verbesserte Eigenschaften, wie beispielsweise eine deutlich verbesserte Bioverfügbarkeit sowie eine längere Halbwertszeit in verschiedenen therapeutisch relevanten Organen. Dies wurde beispielsweise bei einer vergleichenden Gewebsverteilungs-Studie, bei der Mäusen die jeweiligen oligomeren Verbindungen verabreicht und deren Menge zu verschiedenen Zeitpunkten in 18 therapeutisch relevanten Organen gemessen wurde, gefunden (siehe Beispiel 14 und Abb. 1 und 2).

Ferner wurde festgestellt, dass die erfindungsgemäßen N-Phos-Oligomere im Vergleich zu den EP 2041161-Oligomeren eine stärkere Bindung an Blut-Plasma-Proteine besitzen (siehe Beispiel 15); dies ist vorteilhaft für die Bioverfügbarkeit und verlängert die Halbwertszeit.

Zudem weisen die erfindungsgemäßen N-Phos-Oligomere im Vergleich zu den EP 2041161-Oligomeren eine deutlich verbesserte, sequenzunabhängige Wasserlöslichkeit auf (siehe Beispiel 16).

Darüber hinaus wurde festgestellt, dass die erfindungsgemäßen N-Phos-Oligomere verbesserte Bindungseigenschaften an DNA (erhöhter Schmelzpunkt) besitzen (siehe Beispiel 17).

Weiterhin zeigen die erfindungsgemäßen N-Phos-Oligomere einen überraschend deutlich stärkeren Effekt auf die Modulation der Genexpression im Vergleich zu den EP 2041161-Oligomeren. Der stärkere Effekt auf die Modulation der Genexpression zeigt sich beispielsweise bei der Down-Regulation von NFkB in HeLa-Zellen (siehe Beispiel 18) sowie bei der Splice Site Modulation des TNFR2-Gens in THP1-Zellen (siehe Beispiel 19).

Ein Wirksamkeitsvergleich zwischen einem erfindungsgemäßen N-Phos-Oligomer, einem EP 2041161-Oligomer und einem US5719262-Oligomer bei der Splice Site Modulation des Targets TNFR2 in THP1 Zellen bestätigte den deutlich stärkeren Effekt der erfindungsgemäßen N-Phos-Oligomere auf die Modulation der Genexpression. Das US5719262-Oligomer war nahezu wirkungslos, was mit den Beobachtungen von R. Corradini et al. (Current Topics in Medicinal Chemistry, 11 (12), pp. 1535-1554, (2011)), dass die US5719262-Oligomere eine starke Tendenz zeigen,

sich innerhalb von Zellen in Vesikeln anzureichern und damit nicht in ausreichender Menge für einen Antisense-Effekt am Wirkort, der mRNA im Cytosol oder im Nukleus, zur Verfügung zu stehen, übereinstimmt (siehe Beispiel 21 und Abb. 3).

Der starke Effekt der N-Phos-Oligomere auf die Modulation der Genexpression auch im lebenden Organismus wird anhand der Splice Site Modulation des TNFR2-Gens in der Milz und in den Lymphkonten der Maus deutlich (siehe Beispiel 20). Auch in der Lunge (siehe Beispiel 22 und Abb. 4 sowie Beispiel 23 und Abb. 5), der Niere (siehe Beispiel 23 und Abb. 5 sowie Beispiel 24 und Abb. 6), der Leber (siehe Beispiel 23 und Abb. 5) und im Muskel (siehe Beispiel 25 und Abb. 7) konnte für verschiedene erfindungsgemäße N-Phos-Oligomere ein starker Effekt beobachtet werden. In der Niere der Maus zeigen erfindungsgemäße N-Phos-Oligomere beispielsweise einen bis zu 12,6-fach stärkeren Effekt bei der Splice Site Modulation des TNFR2-Gens im Vergleich zu den EP 2041161-Oligomeren (siehe Beispiel 24 und Abb. 6). Im Muskel der Maus konnte ein starker Effekt der erfindungsgemäßen N-Phos-Oligomere auf die Modulation der Genexpression des Dystrophin-Gens (Skipping von Exon 23) gezeigt werden (siehe Beispiel 25 und Abb. 7).

Die Targets NF-kappaB und TNFR2 spielen eine wichtige Rolle im TNF-$\alpha$ Signaltransduktionsweg in Immunzellen. Milz und Lymphknoten sind wichtige Organe des Immunsystems. Damit eignen sich die erfindungsgemäßen oligomeren Verbindungen (N-Phos-Oligomere) für einen therapeutischen Einsatz bei Immunsystem-vermittelten Krankheiten wie beispielsweise Entzündungskrankheiten, Autoimmunerkrankungen oder Krebs.

Die erfindungsgemäßen Monomere der allgemeinen Formel (I) können mittels dem Fachmann bekannter Reaktionen hergestellt werden. Beispielsweise kann ein erfindungsgemäßes Monomer der allgemeinen Formel (I), wobei das asymmetrische Zentrum (#) die R-Konfiguration aufweist und $R^3$ eine Cbz-Schutzgruppe ist, nach folgendem Synthese-Schema hergestellt werden (für eine detailliertere Darstellung siehe Beispiele 1-10):

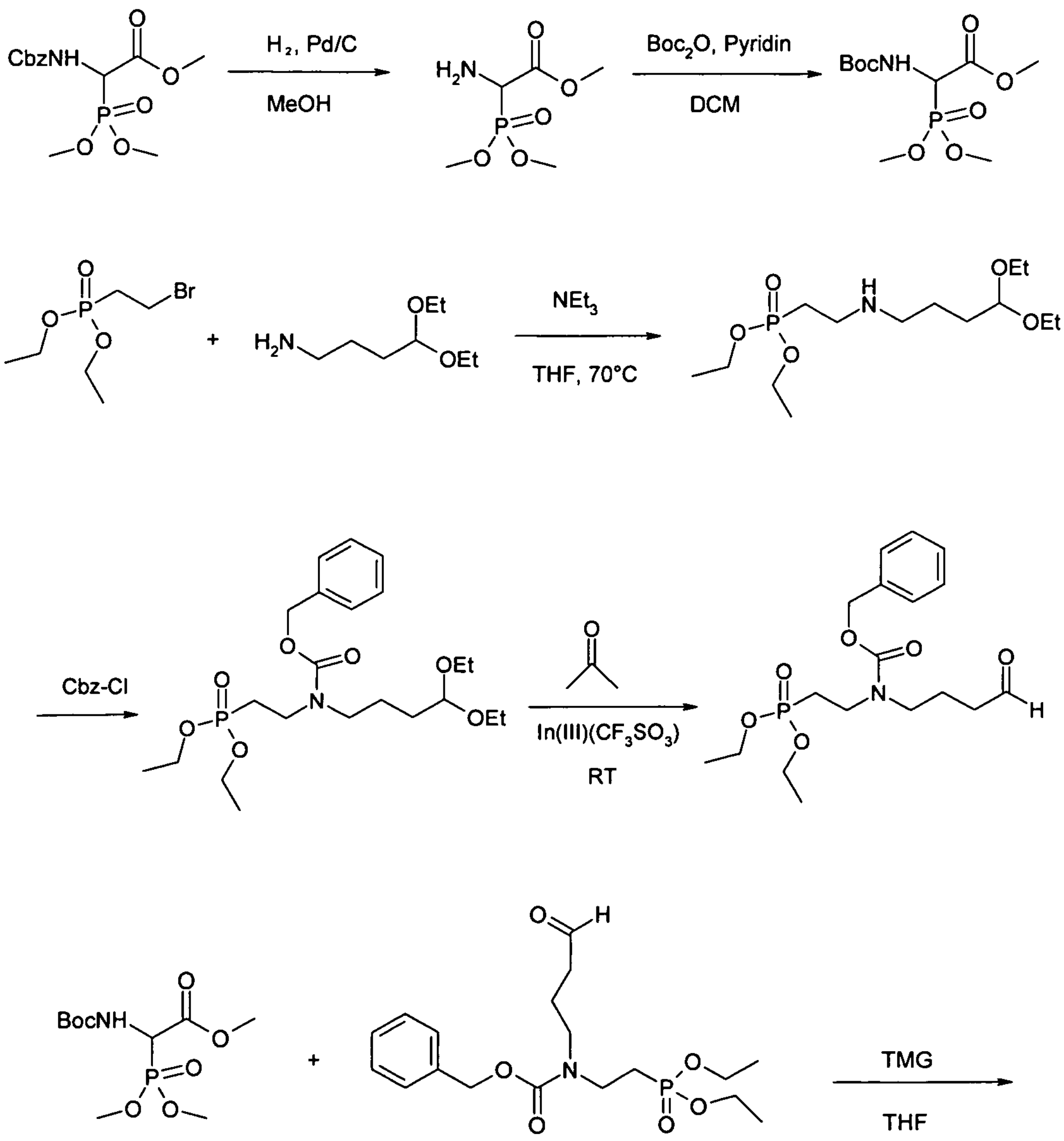

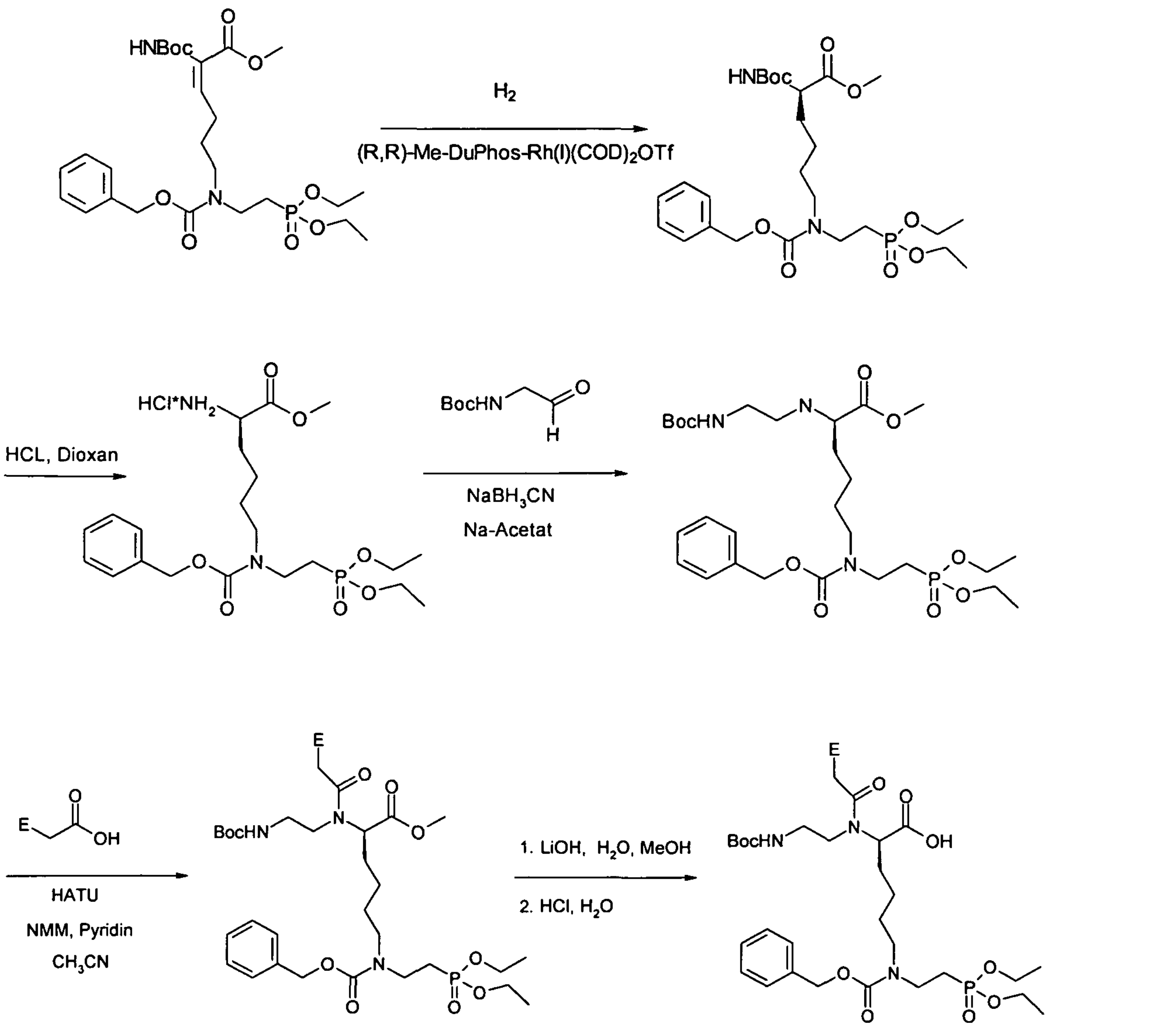


Für die Herstellung eines erfindungsgemäßen Monomers der allgemeinen Formel (I) (hierin auch als N-Phos-Monomer bezeichnet) mit einer S-Konfiguration am asymmetrischen Zentrum (#), wird bei der Hydrierung statt des (R,R)-Me-DuPhos-Rh(I)(OD)$_2$OTf-Katalysators der (S,S)-Me-DuPhos-Rh(I)(COD)$_2$OTf-Katalysator verwendet.

Die erfindungsgemäßen oligomeren Verbindungen gemäß der allgemeinen Formel (III) oder (VI), lassen sich beispielsweise mittels in der Literatur beschriebener Verfahren durch Umsetzung von erfindungsgemäßen Monomeren der allgemeinen Formel (I), oder gegebenenfalls weiteren PNA-Monomeren oder Aminosäuren in an sich bekannter Weise (z.B. L. Christensen, R. Fitzpatrick, B. Gildea, K.H. Petersen, H.F. Hansen, T. Koch, M. Egholm, O. Buchardt, P.E. Nielsen, J. Coull, R.H. Berg, J.Pept.Sci. 3, 1995, 175-183. T. Koch, H.F. Hansen, P. Andersen, T. Larsen, H.G. Batz, K. Otteson, H. Örum, J.Pept.Res. 49, 1997, 80-88. F. Bergmann, W. Bannwarth, S. Tam, Tetrahedron Lett. 36, 1995, 6823-6826) herstellen. Nach der Festphasensynthese werden die Schutzgruppen abgespalten, so dass man erfindungsgemäße oligomere Verbindungen, z.B. Verbindungen der allgemeinen Formel (IV), erhält.

## Beschreibung der Abbildungen

Abbildung 1: Bioverfügbarkeit eines $^3$H markierten N-Phos-Oligomers gemäß der vorliegenden Erfindung sowie eines $^3$H markierten EP2041161-Oligomers in verschiedenen Geweben über den Zeitraum von 14 Tagen. Wie aus der Abbildung ersichtlich, ist die Bioverfügbarkeit des $^3$H markierten N-Phos-Oligomers gegenüber der Bioverfügbarkeit $^3$H markierten EP2041161-Oligomers über den Zeitraum von 14 Tagen in allen Geweben um das 1,7-4,6 fache erhöht.

Abbildung 2: Halbwertszeit eines $^3$H markierten N-Phos-Oligomers gemäß der vorliegenden Erfindung sowie eines $^3$H markierten EP2041161-Oligomers über den Zeitraum von 14 Tagen. Aus Abbildung 2 wird ersichtlich, dass die Halbwertszeit des $^3$H markierten N-Phos-Oligomers gegenüber der Halbwertszeit des $^3$H markierten EP2041161-Oli-

gomers über den Zeitraum von 14 Tagen in den meisten Geweben, in der Milz sogar um das 2-fache, erhöht ist.

Abbildung 3: Wirksamkeitsvergleich zwischen einem erfindungsgemäßen N-Phos-Oligomer, einem EP 2041161-Oligomer und einem US5719262-Oligomer bei der Splice Site Modulation des Targets TNFR2 (Skipping von Exon 7) in THP1 Zellen. Aus Abb. 3 wird ersichtlich, dass das N-Phos-Oligomer im Vergleich zu dem EP 2041161-Oligomer einen 2,6-fach stärkeren Effekt bei der Splice Site Modulation des Targets TNFR2 in THP1 Zellen zeigt, während die Modulation des Targets TNFR2 in THP1 Zellen durch das US5719262-Oligomer nahezu Null ist.

Abbildung 4: Wirkung von erfindungsgemäßen N-Phos-Oligomeren mit unterschiedlichen Resten U auf die Splice Site Modulation des Targets TNFR2 (Skipping von Exon 7) in der Lunge von Mäusen. Aus Abb. 4 wird ersichtlich, dass bei Verwendung von erfindungsgemäßen N-Phos-Oligomeren gemäß der Formel (VI) mit einem Rest U gemäß der allgemeinen Formel VII und einer Gruppe der Formel IXc (Cholesterin-Derivat) bzw. IXd (Folsäure-Derivat) als $R^{46}$ der Effekt auf die Genexpression bei der Splice Site Modulation um das 560-fache (Cholesterin-Derivat) bzw. um das 378-fache (Folsäure-Derivat) gegenüber der PBS-Negativ-Kontrolle erhöht ist.

Abbildung 5: Wirkung der erfindungsgemäßen N-Phos-Oligomere N-Phos 23-1, N-Phos 23-2, N-Phos 23-3 und N-Phos 23-4 auf die Splice Site Modulation des Targets TNFR2 (Skipping von Exon 7) in der Niere, Leber und Lunge von Mäusen. Die erfindungsgemäßen N-Phos-Oligomere N-Phos 23-1, N-Phos 23-2, N-Phos 23-3 und N-Phos 23-4 unterscheiden sich in der Nukleobasen-Sequenz, den Resten U, sowie in der Anzahl und Position der Gruppen der allgemeinen Formel (IV) und (V) gemäß der allgemeinen Formel (VI). Aus Abb. 5 wird ersichtlich, dass die erfindungsgemäßen N-Phos-Oligomere N-Phos 23-1, N-Phos 23-2, N-Phos 23-3 und N-Phos 23-4 in verschiedenen Maus-Geweben (Niere, Leber und Lunge) sehr starke Effekte auf die Genexpression der mRNA Isoform ohne Exon 7 zeigen. In der Niere ist beispielsweise der Effekt von N-Phos 23-1 um 1983-fach gegenüber der PBS-Negativ-Kontrolle erhöht.

Abbildung 6: Wirksamkeitsvergleich zwischen erfindungsgemäßen N-Phos-Oligomeren und EP 2041161-Oligomeren bei der Splice Site Modulation des Targets TNFR2 (Skipping von Exon 7) in der Niere von Mäusen. Die getesteten Oligomere variieren zum einen in der Summe aller Wiederholungseinheiten Yd, Zf, Yg, und Zj (15 bzw. 14) und zum anderen in der Anzahl und Position der Gruppen der allgemeinen Formel (IV) und (V). Aus Abb. 6 wird ersichtlich, dass die Wirkung der N-Phos-Oligomere auf die Genexpression der mRNA Isoform ohne Exon 7 im direkten Vergleich mit den EP2041161-Oligomeren 12,6 bzw. 6,7-fach stärker ist.

Abbildung 7: *In vivo* Wirkung eines erfindungsgemäßen N-Phos-Oligomeren mit 20 Bausteinen (Summe aller Wiederholungseinheiten Yd, Zf, Yg, und Zj gemäß der allgemeinen Formel (VI) = 19) auf die Splice Site Modulation des Targets Dystrophin (Skipping von Exon 23) im Muskel von mdx-Mäusen. Aus Abb. 7 wird ersichtlich, dass das erfindungsgemäße N-Phos-Oligomer schon in einem Kurzzeitexperiment über 15 Tage mit nur 3 Injektionen im Muskel eine 9-fache stärkere Wirkung auf die Genexpression der mRNA Isoform ohne Exon 23 im Vergleich zur PBS-Kontroll-Gruppe zeigt.

## Beispiele

### Beispiel 1: Herstellung von Verbindung 1

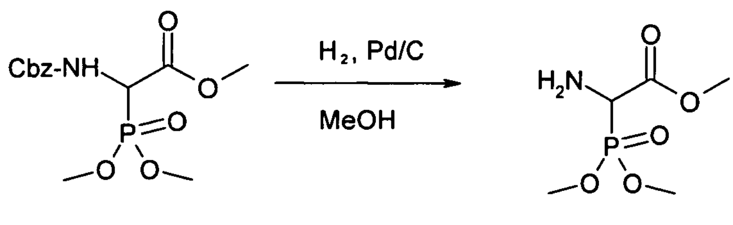


Verbindung 1

Beschreibung: 66,52 g (200 mmol) 2-N-Cbz-Amino-2-(dimethoxyphosphoryl)-essigsäuremethylester in 300 ml Methanol werden mit 2, 13 g Pd/C 10% (entsprechend 1 Mol% Pd) versetzt und unter einem Wasserstoffdruck von 2 bar 24 Stunden bei Raumtemperatur gerührt. Der Katalysator wird über Celite abfiltriert, dann vom Filtrat das Lösemittel abrotiert. Als Produkt erhält man ein leicht gelbes Öl, das beim Stehenlassen zu einem wachsartigen Feststoff wird. Ausbeute: 39 g, 99%. **$^1$H-NMR** (DMSO-$d_6$): 4.01 (d, 1H), 3.66-3.73 (m, 9H), 2.4-2.5 (s, br, 2H). **$^{31}$P-NMR** (DMSO-$d_6$): 23.6 ppm.

**Beispiel 2: Herstellung von Verbindung 2**

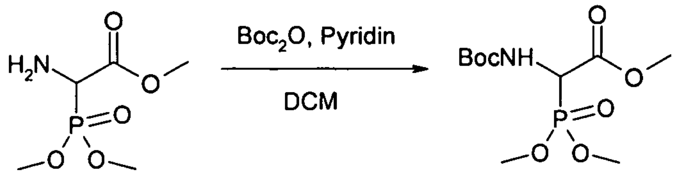


Verbindung 2

Beschreibung: 39 g (198 mmol, 1 eq) Verbindung 1 werden in 1000 ml Dichormethan gelöst. Dann werden 56,178 g (257 mmol, 1,3 eq) $BOC_2O$ sowie 16,1 ml (198 mmol, 1 eq) Pyridin zugefügt. Die Mischung wird 48 Stunden bei Raumtemperatur gerührt. Das Lösemittel wird am Rotationsverdampfer entfernt, der Rückstand in Essigester aufgenommen und mit 5%iger Zitronensäurelösung, gesättigter Natriumcarbonatlösung und gesättigter Kochsalzlösung gewaschen. Anschließend wird mit Magnesiumsulfat getrocknet und einrotiert. Das zurückbleibende Produkt wird per FlashChromatographie (Silicagel, Hexan/Essigester 1:5) gereinigt. Es resultiert ein gelbes Öl.
Ausbeute: 43,5 g (146 mmol, 74%). **$^1$H-NMR** ($CDCl_3$): 5.35 (d, br, 1H), 4.88 (dd, 1H), 3.80-3.86 (m, 9H), 1.46 (s, 9H). **$^{31}$P-NMR** ($CDCl_3$): 20.1 ppm

**Beispiel 3: Herstellung von Verbindung 3**

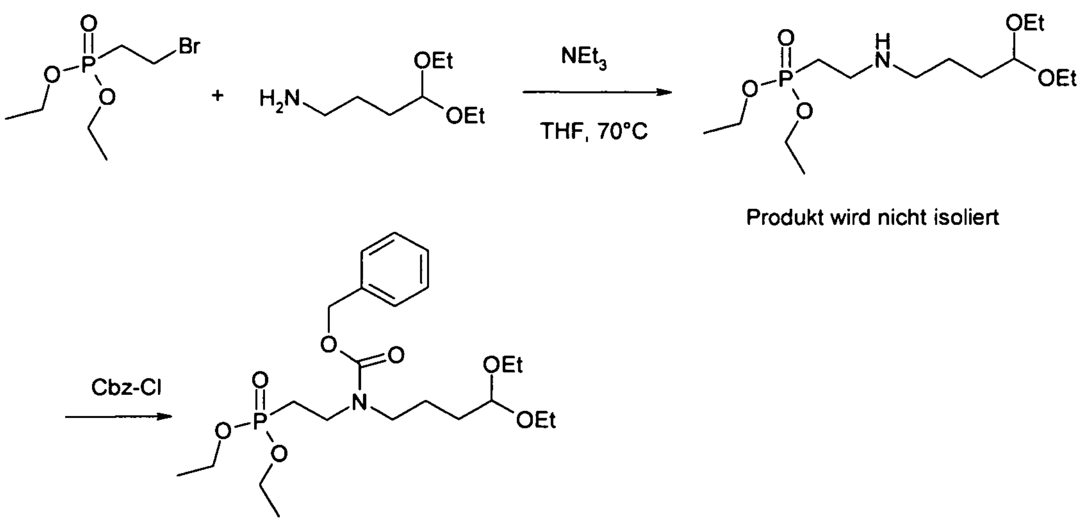


Produkt wird nicht isoliert

Verbindung 3

Beschreibung: 50 g (319 mmol, 1eq) 4-Aminobutyraldehyddiethylacetal werden bei Raumtemperatur in 200 ml THF und 51,5 ml (372 mmol, 1,2 eq) Triethylamin gelöst, dann werden 75,99 g (310 mmol, 1eq) Diethyl-2-bromethylphosphonat zugetropft. Anschließend wird die Lösung auf 70°C erhitzt und bei dieser Temperatur 24 Stunden gerührt. Das Lösemittel wird am Rotationsverdampfer entfernt. Der Rückstand wird mit Ether heftig geschüttelt und abfiltriert. Der dabei zurückbleibende Feststoff wird noch zweimal mit Ether extrahiert. Die Ether-Filtrate werden vereint und abrotiert. Das resultierende Produkt ist ein gelbes Öl, das ohne weitere Aufreinigung in die nächste Reaktion eingesetzt wird. **$^1$H-NMR** (DMSO-$d_6$): 4.45 (t, 1H); 3.98 (m, 4H); 3.54 and 3.42 (2m, 2 x 2H); 2.5-2.8 (m, 4H); 1.90 (m, 2H); 1.25-1.60 (m, 4H); 1.22 (t, 6H); 1.10 (2t, 6H). **$^{31}$P-NMR** (DMSO-$d_6$): 30 ppm.

Das im ersten Schritt erhaltene Zwischenprodukt wird in 400 ml THF gelöst, mit 85,94 ml (620 mmol, 2eq) Triethylamin versetzt und auf 0°C gekühlt. Dann werden 66,11 ml (465 mmol, 1,5 eq) Chlorameisensäurebenzylester zugetropft, die Kühlung wird entfernt und es wird über Nacht bei Raumtemperatur gerührt. Anschließend wird das Reaktionsgemisch mit 1 M Salzsäure neutralisiert und das Lösemittel wird abrotiert. Der Rückstand wird mit Ether

geschüttelt und über Nacht im Kühlschrank aufbewahrt. Der dabei ausgefallene Feststoff wird abgetrennt und noch zweimal gründlich mit Ether nachgewaschen. Die Etherlösungen werden vereinigt und abrotiert. Der Rückstand wird durch Chromatographie über eine Kieselgelsäule gereinigt. Dabei werden zunächst alle Verunreinigungen mit Hexan:Essigester 2:1 und dann das Produkt mit Essigester eluiert.
Ausbeute: 85,752 g (60,2%) farbloses viskoses Öl. **$^1$H-NMR** (DMSO-$d_6$): 7.34 (m, 5H); 5.07 (s, 2H); 4.44 (m,1H); 3.94 (m, 4H); 3.6-3.2 (m, 8H); 2.02 (m, 2H); 1.46 (m, 4H); 1.19 (m, 6H); 1.09 (m, 6H). **$^{31}$P-NMR** (DMSO-$d_6$):: 28.93 und 28.57 ppm.

**Beispiel 4: Herstellung von Verbindung 4**

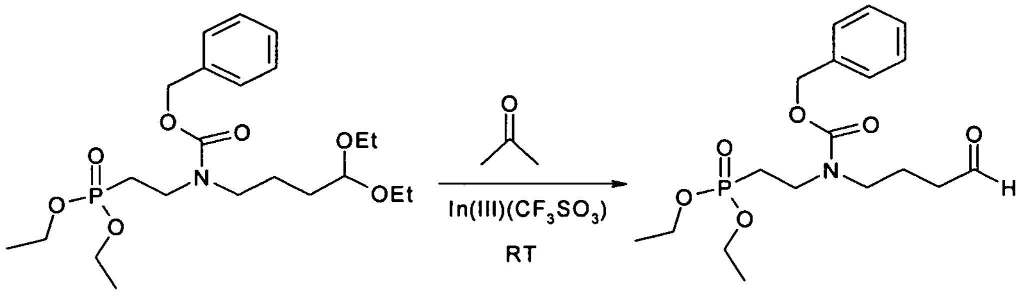


Verbindung 4

Beschreibung: 80 g der Verbindung 3 in 1000 ml Aceton werden mit 0,98 g (1,74 mmol, 1Mol%) Indium(III)-triflat bei Raumtemperatur gerührt. Der Fortschritt der Gleichgewichtsreaktion wird per HPLC ($RP_{18}$, Methanol/Wasser 80:20) verfolgt. Von Zeit zu Zeit wird das Lösungsmittel abrotiert und durch frisches Aceton ersetzt. Dies geschieht so lange, bis die Reaktion zu mehr als 95% abgelaufen ist. Dann wird das Lösemittel abrotiert. Die Substanz, ein gelbes Öl, wird kurz im Hochvakuum getrocknet und sofort weiterverwendet.
**$^1$H-NMR** (DMSO-$d_6$): 9.62 (d, 1H); 7.36 (m, 5H); 5.07 (s, 2H); 3.94 (m, 4H); 3.37 und 3.24 (2m, 4H); 2.41 (m, 2H), 2.04 (m, 2H); 1.73 (m, 2H); 1.22 (t, 6H). **$^{31}$P-NMR**(DMSO-$d_6$): 29.51, 29.14 ppm

**Beispiel 5: Herstellung von Verbindung 5**

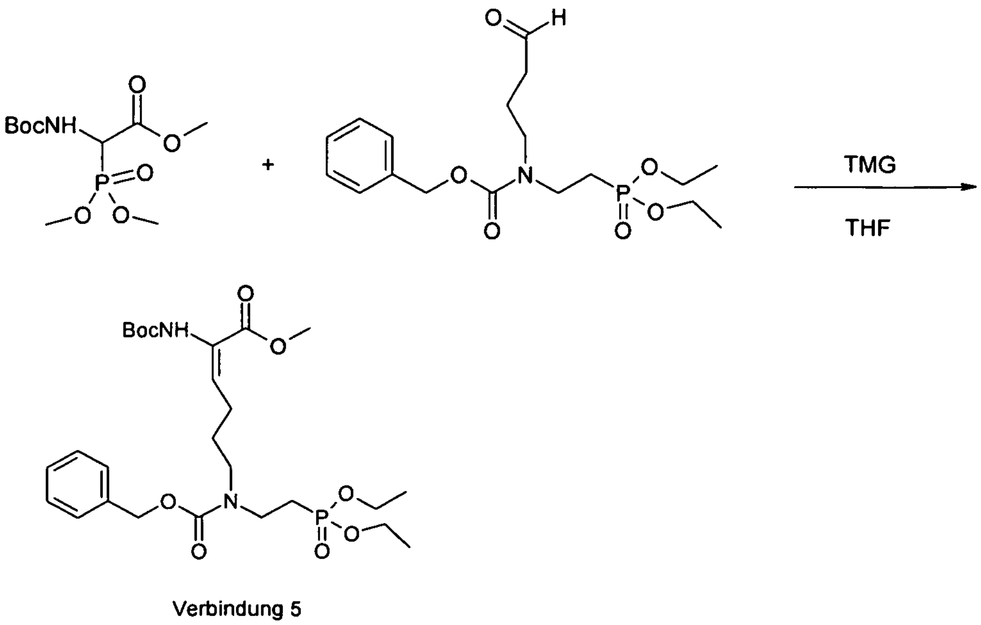


Verbindung 5

Beschreibung: In Argon-Atmosphäre werden 29,438 g Verbindung 2 in 350 ml THF auf -70°C gekühlt, dann

werden 12,96 ml (103 mmol, 1,04 eq) N,N,N'N'-Tetramethylguanidin zugetropft. Nachdem 10 Minuten bei -70°C gerührt wurde, werden 38,170 g (99,04 mmol, 1 eq) Verbindung 4 in 60 ml THF zugetropft. Es wird noch eine Stunde bei -70°C gerührt, dann lässt man den Ansatz langsam auf Raumtemperatur kommen und rührt über Nacht.

Das Lösemittel wird abrotiert. Der Rückstand wird in etwa 400 ml Essigester aufgenommen, zweimal mit 5%iger Zitronensäurelösung und einmal mit gesättigter NaCl-Lösung gewaschen, mit Magnesiumsulfat getrocknet und einrotiert. Man erhält ein gelbes Öl.

Ausbeute: 53,228 g, 95,6mmol, 96,5%. **$^1$H-NMR** ($CDCl_3$): 7.33 (s, 5H); 6.48 (t, 1H); 5.13 (s, 2H); 4.08 (m, 4H) ; 3.81 und 3.76 (2s, gesamt 3H); 3.49 (m, 2H); 3.30 (t, 2H); 2.21 (m, 2H); 2.04 (m, 2H); 1.71 (m, 2H); 1.48 und 1.46 (2s, gesamt 9H); 1.28 (m, 6H). **$^{31}$P-NMR** ($CDCl_3$): 29.72 und 29.15 ppm.

**Beispiel 6: Herstellung von Verbindung 6**

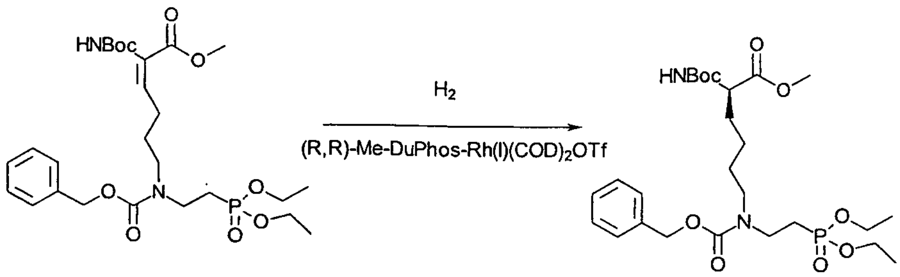


Verbindung 6

Beschreibung: In der Reaktionsflasche einer Parr-Hydrierapparatur werden unter Argon 450 mg (0,96 mmol, 1Mol%) Bis(1,5-cyclooctadienyl)rhodium(I)-trifluormethansulfonat sowie 306 mg (0,96 mmol, 1Mol%) (-)-1,2-Bis-[(2R,5R)-2,5-dimethyl-phospholano]-benzol in etwa 50 ml Methanol gelöst, dann werden 53,228 g (96 mmol) Verbindung 5, gelöst in 250 ml Methanol, zugegeben. Die Flasche wird in die Hydrierapparatur eingebaut, dreimal evakuiert und mit Wasserstoff gefüllt. Schließlich wird ein Wasserstoffdruck von 4,5-5 bar eingestellt und die Flasche 24 Stunden geschüttelt.

Der überschüssige Wasserstoff wird abgelassen, die Flasche wird ausgebaut und die Reaktionslösung wird über Celite filtriert. Das Filtrat wird abrotiert und im Vakuum getrocknet. Man erhält ein hellbraunes Öl.

Ausbeute:53,712 g, 96 mmol, quantitativ. **$^1$H-NMR** ($CDCl_3$): 7.35 (m, 5H); 6.48 und 6.35 (2m, gesamt 1H); 5.13 (s, 2H); 4.27 (m, 1H); 4.07 (m, 4H); 3.73 (s, 3H); 3.48 (m, 2H); 3.27 (m, 2H); 2.25-1.95 (m, 4H); 1.75-1.55 (m, 4H); 1.45 (s, 9H); 1.28 (m, 6H). **$^{31}$P-NMR** ($CDCl_3$): 29.78 und 29.23 ppm.

**Beispiel 7: Herstellung von Verbindung 7**

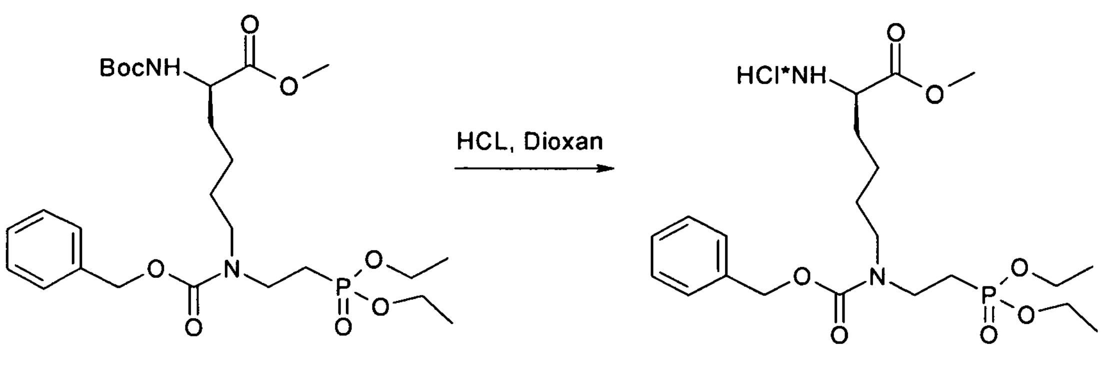


Verbindung 7

Beschreibung: Zu 53,94 g (96,6 mmol) Verbindung 6 in 120 ml THF werden 240 ml einer 4M Lösung HCl in Dioxan getropft. Anschließend wird bei Raumtemperatur gerührt. Der Reaktionsverlauf wird mittels HPLC verfolgt (Methanol/Wasser 70:30). Nach 2-3 Stunden ist die Boc-Abspaltung vollständig. Das Lösemittel wird abrotiert und der Rückstand mit Diethylether gewaschen und getrocknet. Man erhält ein zähes braunes Öl. Ausbeute: 47,83 g, quantitativ. **$^1$H-NMR** (DMSO-$d_6$): 8.64 (s, br, 2H); 7.36 (m,, 5H); 5.07 (s, 2H); 3.97 (m, 5H); 3.73 (s, 3H); 3.38 (m, 2H); 3.22 (m, 2H); 2.04 (m, 2H); 1.80 (m, 2H); 1.48-1.35 (m, 4H); 1.22 (m, 6H). **$^{31}$P-NMR** (DMSO-$d_6$): 29.012 und 28.62 ppm.

**Beispiel 8: Herstellung von Verbindung 8**

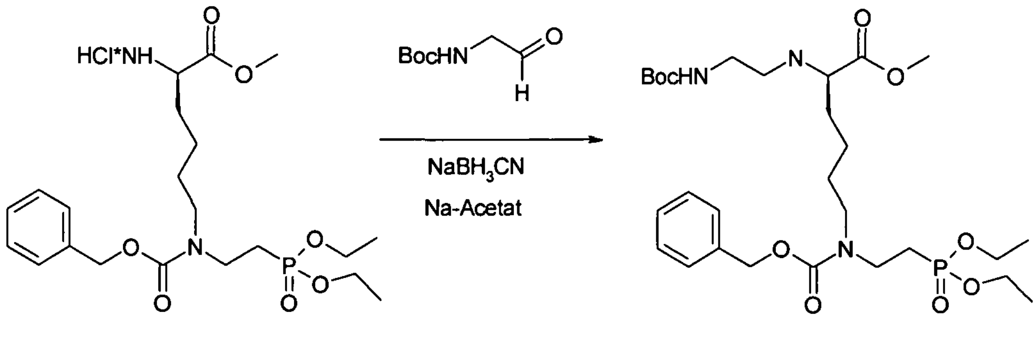


Verbindung 8

Beschreibung: 16,65 g (29,8 mmol, 1eq.) Verbindung 7 in 100 ml Methanol werden auf 0 °C gekühlt und mit 5,414 g (66 mmol, 2,2 eq) Natriumacetat versetzt. Dann werden 5,218 g (32,8 mmol, 1,1 eq) N-Boc-aminoacetaldehyd in 150 ml Methanol zugetropft. Es wird eine Stunde bei 0°C gerührt, dann werden 2,074 g (33 mmol, 1,1 eq) Natriumcyanoborhydrid portionsweise zugegeben. Wenn die Gasentwicklung nachgelassen hat, wird das Kühlbad entfernt und über Nacht bei Raumtemperatur gerührt.

Das Lösungsmittel wird am Rotationsverdampfer entfernt, der Rückstand in Essigester aufgenommen und mit Natriumhydrogencarbonat-Lösung (halbgesättigt) sowie gesättigter Natriumchlorid-Lösung gewaschen. Die organische Phase wird mit Magnesiumsulfat getrocknet, einrotiert und Vakuum-getrocknet. Das Rohprodukt wird über eine Kieselgelsäule aufgereinigt (Dichlormethan/Methanol (5%, v/v)). Man erhält ein viskoses gelbes Öl.

Ausbeute: 15,379 g (25,6 mmol, 86%). **$^1$H-NMR** (DMSO-$d_6$): 7.36 (m, 5H); 6.69 (m, 1H); 5.06 (s, 2H); 4.02 (m, 4H); 3.4-3.15 (3m, gesamt 7H); 2.95 (m, 1H); 2.38 (m, 1H); 2.01 (m, 2H); 1.60-1.30 (m, 6H); 1.38 (s, 9H); 1.20 (m, 6H). **$^{31}$P-NMR** (DMSO-$d_6$) : 29.00 und 28.60 ppm.

**Beispiel 9: Herstellung von Verbindung 9**

E = Cbz-A, Cbz-C, Cbz-G, T

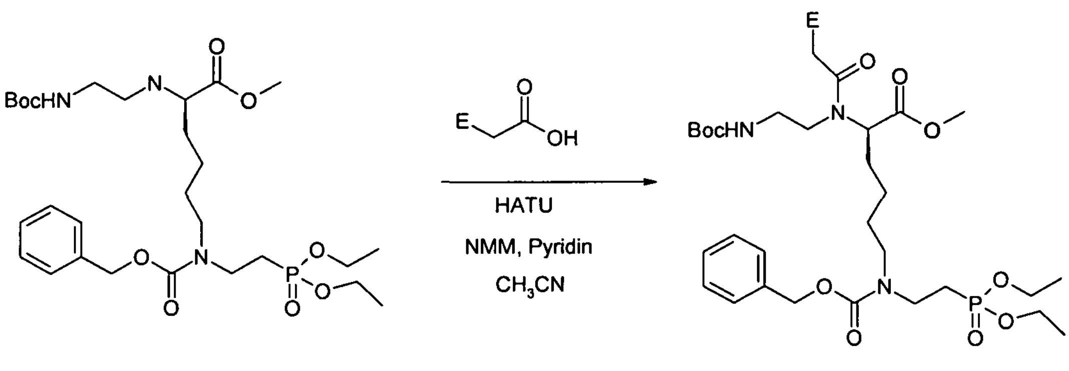


Verbindung 9

Beschreibung: 20,25 mmol (1,5 eq) der Essigsäurekomponente (N6-Cbz-Adenin-9-yl-essigsäure, N4-Cbz-Cytosin-1-yl-essigsäure, N2-Cbz-Guanin-9-yl-essigsäure bzw. Thymidin-1-yl-essigsäure) in 70 ml Acetonitril, 20 ml Pyridin sowie 4,5 ml (40, 5 mmol, 3eq) N-Methylmorpholin werden auf 0°C gekühlt und mit 7,186 g (18,9 mmol, 1,4 eq) HATU

versetzt. Die Kühlung wird entfernt, und es wird 10 Minuten bei Raumtemperatur gerührt. Dann wird das Gemisch langsam zu 8,1 g (13,5 mmol, 1eq) der Verbindung 8, gelöst in 50 ml Acetonitril, gegeben. Es wird 1 Stunde bei Raumtemperatur, dann über Nacht bei 40°C gerührt.

Man kühlt das Gemisch wieder auf Raumtemperatur ab und versetzt es mit 20 ml Wasser. Nach halbstündigem Rühren wird das Lösemittel abrotiert. Anschließend wird der Rückstand noch zweimal mit Dichlormethan abrotiert, um möglichst viel Pyridin zu entfernen. Dann wird der Rückstand wieder in Dichlormethan aufgenommen und über Nacht in den Kühlschrank gestellt. Der dabei ausfallende Feststoff wird abfiltriert, das Filtrat einrotiert und mittels Flash-Chromatographie (Kieselgel, 2-5% Methanol in Dichlormethan) gereinigt, wobei das Produkt als weiß-gelb gefärbter Schaum erhalten wird.

Verbindung 9, E = Cbz-A:
Ausbeute: 62 %. **$^1$H-NMR** (DMSO-$d_6$): 10.65 (s, 1H); 8.59 (s, 1H); 8.35 und 8.29 (2s, gesamt 1H); 1.47-7.24 (m, 10H); 6.90 und 6.72 (2m, gesamt 1H); 5.42-5.00 (m, 6H); 3.92 (m, 5H); 3.56 und 3.49 (2s, gesamt 3H); 3.50-2.95 (m, 8H); 2.12-1.30 (m, 8H); 1.38 und 1.35 (2s, gesamt 9H); 1.16 (m, 6H) ; **$^{31}$P-NMR** (DMSO-$d_6$): 29.52 und 29.16 ppm.

Verbindung 9, E=Cbz-C:
Ausbeute: 59%. **$^1$H-NMR** (DMSO-$d_6$): 10.77 (s, br, 1H); 7.94 (d, 1H), 7.42-7.33 (m, 10H); 7.01(d, 1H); 6.91 und 6.80 (2m, gesamt 1H); 5.19 (s, 2H); 5.07 (s, 2H); 4.80-4.6 (m, 2H); 4.36 (m, 1H); 3.94 (m, 4H); 3.7 und 3.59 (2s, gesamt 3H); 3.5-2.8 (m, 8H); 2.05-1.3 (m, 8H); 1.38 und 1.37 (2s, gesamt 9H); 1.19 (m, 6H). **$^{31}$P-NMR** (DMSO-$d_6$): 29.02 und 28.63 ppm.

Verbindung 9, E = Cbz-G:
Ausbeute: 77,5 %. **$^1$H-NMR** (DMSO-$d_6$): 11.33 (s, br, 2H); 7.85 (s, 1H); 7.45-7.30 (m, 10 H); 6.99 und 6.81 (2m, gesamt 1H); 5.26 (s, 2H); 5.06 (m, 4H); 4.58 und 4.31 (2m, gesamt 1H); 3.934 (m, 4H); 3.56 (s, 3H); 3.31-3.19 (m, 8H); 2.21-1.30 (m, 8H); 1.36 und 1.35 (2s, gesamt 9H); 1.19 (m, 6H). **$^{31}$P-NMR** (DMSO-$d_6$): 28.98 und 28.59 ppm.

Verbindung 9, E = T:
Ausbeute: 78 % **$^1$H-NMR** (DMSO-$d_6$): 11.26 (s, br, 1H); 7.35 (m, 6H); 6.90 und 6.79 (2m, gesamt 1H); 5.07 (s, 2H); 4.63-4.49 (m, 2H); 4.31 (m, 1H); 3.94 (m, 4H); 3.70 und 3.58 (2s, gesamt 3H); 3.46-3.12 (m, 8H); 2.11-1.30 (m, 8H); 1.76 (s, 3H); 1.38 und 1.35 (2s, gesamt 9H); 1.19 (m, 6H). **$^{31}$P-NMR** (DMSO-$d_6$): 29.61 und 29.25 ppm.

**Beispiel 10: Herstellung von**

E = Cbz-A, Cbz-C, Cbz-G, T

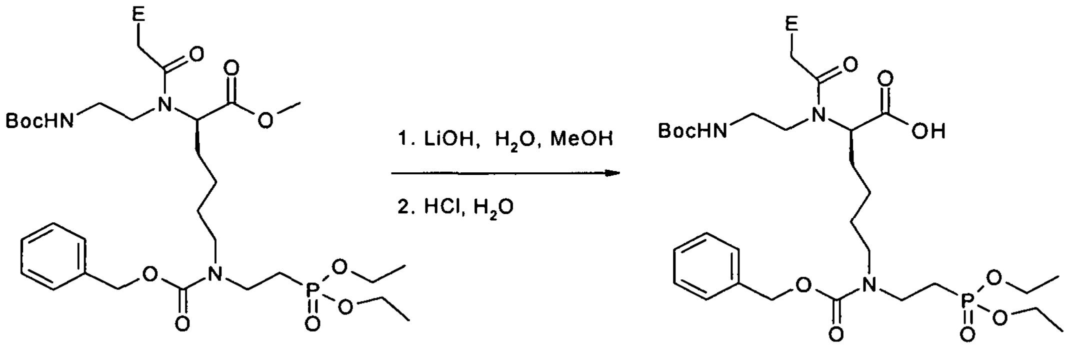


Verbindung 10

Beschreibung: 2,31 mmol der Verbindung 9 (E = Cbz-A, Cbz-C, Cbz-G oder T) werden in 12 ml Wasser/Methanol 1:1 auf 0°C gekühlt, dann werden 12 ml einer 2N NaOH-Lösung zugetropft. Es wird 15 Minuten bei 0°C, dann bei Raumtemperatur gerührt, bis die Verseifung gemäß DC-Kontrolle (Kieselgel, 10% Methanol in Dichlormethan) vollständig ist (Dauer: ca. 1 Stunde). Dann wird mit etwas Wasser verdünnt und evtl. vorhandene ungelöste Substanzen werden kurz abzentrifugiert. Die klare Lösung wird auf etwa 300 ml mit Wasser verdünnt und wieder auf 0°C gekühlt. Mit 1M HCl-Lösung wird ein pH von 2,5 eingestellt, dabei fällt ein weißer Feststoff aus. Die Lösung wird solange mit Dichlormethan extrahiert (ca. 5mal), bis kein Produkt mehr in die organische Phase übergeht (DC-Kontrolle).Die vereinigten organischen Phasen werden mit Magnesiumsulfat getrocknet, einrotiert und vakuumgetrocknet. Man erhält das Produkt als weiß-gelben Feststoff.

Verbindung 10, E = Cbz-A:
Ausbeute: 72 %. **$^1$H-NMR** (DMSO-$d_6$): 10.68 (s, br, 1H); 8.59 (s, br, 1H); 8.35 und 8.29 (2s, br, gesamt 1H); 7.49-7.33 (m, 10 H); 6.98 und 6.85 (2m, gesamt 1H); 5.35-5.04 (m 6H); 4.62 und 4,22 (2m, gesamt 1H,); 3.93 (m, 4H,); 3.50-2.85

(m, 8H); 2.20-1.30 (m, 8H); 1.39 und 1.36 (2s, gesamt 9H); 1.17 (m, 6H). **$^{31}$P-NMR** (DMSO-$d_6$): 29.55 und 29.19 ppm.

Verbindung 10, E = Cbz-C:
Ausbeute: 59%. **$^{1}$H-NMR** (DMSO-$d_6$): 7.95 (d, 1H); 7.42-7.28 (m, 10H); 7.01 (d, 1H); 6.90 und 6.85 (2m, gesamt 1H); 5.19 (s, 2H); 5.07 (s, 2H); 4.81-4.65 (m, 2H); 4.33 (m, 1H); 3.933 (m, 4H); 3.45-3.15 (m, 8H); 2.15-1.30 (m, 8H); 1.38 und 1.35 (2s, gesamt 9H); 1.20 (m, 6H). **$^{31}$P-NMR** (DMSO-$d_6$): 29.03 und 28.66 ppm.

Verbindung 10, E = Cbz-G:
Ausbeute: 62%. **$^{1}$H-NMR** (DMSO-$d_6$): 11.43 (s, br, 1H); 11.33 (s, br, 1H); 7.85 (s, 1H); 7.44-7.31 (m, 10H); 6.97 und 6.81 (2m, gesamt 1H); 5.24 (s, 2H); 5.10-5.00 (m, 4H); 4.51 und 4.28 (2m, gesamt 1H); 3.91 (m, 4H); 3.52-3.08 (m, 8H); 2.12-1.28 (m, 8H); 1.36 und 1.34 (2s, gesamt 9H); 1.15 (m, 6H). **$^{31}$P-NMR** (DMSO-$d_6$): 29.61 und 29.21 ppm.

Verbindung 10, E = Cbz-T:
Ausbeute: 72%. **$^{1}$H-NMR** (DMSO-$d_6$): 11.27 (s, br, 1H); 7.35 (m, 6H); 6.88 (m, 1H); 5.07 (s, 2H); 4.65-4.48 (m, 2H); 4.37 und 4.38 (2m, gesamt 1H); 3.94 (m, 4H) ; 3.45-3.17 (m, 8H); 2.11-1.30 (m, 8h); 1.75 (s, 3H); 1.38 und 1.36 (2s, gesamt 9H); 1.19 (m, 6H). **$^{31}$P-NMR** (DMSO-$d_6$): 29.64 und 29.26 ppm.

### Beispiel 11: Herstellung erfindungsgemäßer oligomerer Verbindungen der allgemeinen Formeln (III) oder (VI)

Durch sequenzielle Verknüpfung von entsprechenden Verbindungen der allgemeinen Formel (I) mit Monomereinheiten ausgewählt aus der Gruppe, bestehend aus N-Acetyl-N-(2-aminoethyl)glycin-Bausteinen, Aminosäuren, Aminosäurederivaten, und einer Gruppe B-CH($R^{48}$)COOH, mittels Festphasenpeptidsynthese werden erfindungsgemäße oligomere Verbindungen hergestellt.

Zur einfacheren Darstellung der Einheiten Z gemäß der allgemeinen Formel (V) in oligomeren Verbindungen der allgemeinen Formel (IV) werden beispielsweise folgende Abkürzungen verwendet: $T^R$, $C^R$, $G^R$, $A^R$, $P^R$, $T^S$, $C^S$, $G^S$, $A^S$ und $P^S$. Dabei steht T, C, G, A, und P (Phenyl) jeweils für die Nukleobase der jeweiligen Monomereinheit und das hochgestellte R bzw. S steht für die R- bzw. S-Konfiguration am asymmetrischen Zentrum (#) der Einheit Z gemäß der allgemeinen Formel (V).

Monomere, bestehend aus N-Acetyl-N-(2-aminoethyl)glycin-Bausteinen, werden zu den oben beschriebenen Monomeren der allgemeinen Formel (V) analog abgekürzt, mit dem Unterschied, dass anstatt dem Großbuchstaben für die Nukleobase und dem hochgestellten Buchstaben für die Konfiguration (z.B. $A^R$) der entsprechende Kleinbuchstabe a verwendet wird. Beispielsweise wird ein Monomer mit C als Nukleobase als c abgekürzt.

### Beispiel 12: Oligomere Verbindungen der allgemeinen Formeln (III) oder (VI) mit 4-Fluorphenyl-acetat-Substituent als Gruppe B-CH($R^{48}$)COOH:

Zur Herstellung der erfindungsgemäßen Verbindungen mittels Festphasenpeptidsynthese wird folgendes Syntheseprotokoll verwendet:

Schritt 1: 3 h Vorquellen von 10 mg Harz (MBHA-Harz, Novabiochem, Low Loaded ca. 0.5-06 mmol/g) in Dichlormethan.
Schritt 2: Beginn des Synthesezyklus: 4x Waschen mit Dichlormethan.
Schritt 3: Neutralisieren des Harzes: 3x Waschen mit Dichlormethan/DIPEA (5%).
Schritt 4: 5x Waschen mit Dichlormethan.
Schritt 5: 5x Waschen mit NMP.
Schritt 6: 1 min Voraktivierung von 4 Äquivalenten der entsprechenden geschützten Verbindung (Verbindung der allgemeinen Formel (I)/PNA-Monomer/Aminosäure/Aminosäurederivat) mit 3,8 Äquivalenten HATU und 9 Äquivalenten NMM in NMP/Pyridin (2:1).
Schritt 7: Reaktion der aktivierten geschützten Verbindung (Verbindung der allgemeinen Formel (I)/PNA-Monomer/Aminosäure/Aminosäurederivat) mit der festen Phase (1. Kupplung; Dauer: 60 min).
Schritt 8: 4x Waschen mit NMP.
Schritt 9: Wiederholung der Schritte 6 bis 8 (2.Kupplung).
Schritt 10: Überprüfung der Kupplungseffizienz mit Ninhydrin (Kaiser-Test; Wenn der Kaiser-Test positiv ist, müssen die Schritte 6 bis 8 mit der entsprechenden geschützten Verbindung (Verbindung der allgemeinen Formel (I)/PNA-Monomer/Aminosäure/Aminosäurederivat) wiederholt werden).
Schritt 11: Nach negativem Kaiser-Test, 1x Capping mit einer Lösung aus $Ac_2O$/NMP/Pyridin (1:25:25) für 10 min.
Schritt 12: 5x Waschen mit NMP.
Schritt 13: Umstellen des Lösungsmittels auf Dichlormethan: 5x Waschen mit DCM.
Schritt 14: Boc-Abspaltung durch Reaktion mit TFA/m-Kresol (95:5). Reaktionsdauer: 2x je 3 min
Schritt 15: 5x Waschen mit DCM.
Schritt 16: Umstellen des Lösungsmittels auf NMP. 5x Waschen mit NMP.

Schritt 17: Wiederholen des Synthesezyklus (Schritte 6 bis 16) bis zur Kupplung mit der letzten entsprechenden geschützten Verbindung (Verbindung der allgemeinen Formel (I)/PNA-Monomer/Aminosäure/Aminosäurederivat). Anschließend gegebenenfalls Wiederholen des Synthesezyklus (Schritte 6 bis 16) bis zur Kupplung mit der letzten entsprechend geschützten Verbindung (Verbindung der allgemeinen Formel (I)/PNA-Monomer/Aminosäure/Aminosäurederivat).
Schritt 18: 5x Waschen mit Dichlormethan.
Schritt 19: Boc-Abspaltung durch Reaktion mit TFA/m-Kresol (95:5). Reaktionsdauer: 2x je 3 min
Schritt 20: 5x Waschen mit Dichlormethan.
Schritt 21: 5x Waschen mit NMP.
Schritt 22: 1 min Voraktivierung von 6 Äquivalenten 4-Fluorphenyl-Essigsäure mit 5,7 Äquivalenten HATU und 13 Äquivalenten NMM in NMP/Pyridin (2:1).
Schritt 23: Reaktion von aktivierten 4-Fluorphenyl-Essigsäure mit der festen Phase (Dauer: 60 min).
Schritt 24: 4x Waschen mit NMP.
Schritt 25: Eventuell Wiederholung der Schritte 23 bis 24 (2.Kupplung).
Schritt 26: 5x Waschen mit Dichlormethan.
Schritt 27: zur Trocknung: 5x Waschen mit Diethylether.

Man erhält eine Verbindung der allgemeinen Formeln (III) oder (VI), die am C-terminalen Ende an das Harz gebunden ist.

Abspaltung der erfindungsgemäßen Verbindung der allgemeinen Formeln (III) oder (VI) vom Harz:
Das Harz mit der erfindungsgemäßen Verbindung wird in einer Lösung aus Trifluoressigsäure, Trifluormethansulfonsäure, Thioanisol und Ethandithiol (85/12,5/1,7/0,8, v/v/v/v) für 2 Stunden geschüttelt. Die flüssige Phase wird abfiltriert und das Rohprodukt durch Zugabe von kaltem Ether ausgefällt. Das Rohprodukt wird durch Size-Exclusion-Chromatografie entsalzt. Das Rohprodukt wird durch präparative HPLC über eine RP-$C_{18}$-Säule mit Methanol / Wasser gereinigt. Man erhält die erfindungsgemäße Verbindung als farblosen Feststoff in ca. 50%-iger Ausbeute. Die Masse der erfindungsgemäßen Verbindung wird mit HPLC-ESI charakterisiert.

**Beispiel 13: Beispiele von hergestellten Verbindungen der allgemeinen Formeln (III) oder (VI)**

Durch Durchführung der allgemeinen Synthesevorschrift aus Beispiel 12 erhält man oligomere Verbindungen der allgemeinen Formeln (III) oder (VI), die wie folgt abgekürzt werden:
Beispielsweise wird eine erfindungsgemäße oligomere Verbindung aus Monomeren der allgemeinen Formel (I) mit einem asymmetrischen Zentrum mit R-Konfiguration sowie weiteren, PNA-Monomeren und der Aminosäure L-Lysin (Abkürzung: $Lys^L$) hergestellt und im letzten Schritt die $\alpha$-Amino-Funktion des Lysins mit Acetyl gecappt und zuletzt die oligomere Verbindung danach als primäres Amid vom Harz abgespalten, abgekürzt als Ac-$Lys^L$-c$C^R$g$G^R$g$G^R$tcgca$G^R$c-$T^R$g$G^R$-$NH_2$. Beispielsweise wird eine erfindungsgemäße oligomere Verbindung aus Monomeren der allgemeinen Formel (I) mit einem asymmetrischen Zentrum mit S-Konfiguration sowie weiteren, PNA-Monomeren und der Aminosäure Glycin (Abkürzung: Gly) hergestellt und im letzten Schritt die $\alpha$-Amino-Funktion des Lysins mit Phenylacetat (Abkürzung: Pac) gecappt und die oligomere Verbindung zuletzt als primäres Amid vom Harz abgespalten, abgekürzt als Pac-Gly-agccc$T^S$aac$T^S$gcac$T^ST^S$cca$T^S$-$NH_2$.
Beispielsweise wird eine erfindungsgemäße oligomere Verbindung aus Monomeren der allgemeinen Formel (I) mit einem asymmetrischen Zentrum mit R-Konfiguration sowie weiteren PNA-Monomeren und der Aminosäure D-Lysin (Abkürzung: $Lys^D$) hergestellt und im letzten Schritt die $\alpha$-Amino-Funktion des Lysins mit 4-Fluor-Phenylacetat (Abkürzung: FluPac) gecappt und die oligomere Verbindung zuletzt als primäres Amid vom Harz abgespalten, und danach noch die $\varepsilon$-Amino-Funktion des Lysins mit dem Fluoreszenz-Farbstoff ATTO647 gekoppelt, abgekürzt als FluPac-$Lys^D$(ATTO647)-c$C^R$g$G^R$g$G^R$tcgca$G^R$c$T^R$g$G^R$-$NH_2$.

Weitere Fluoreszenz-Farbstoffe sind beispielsweise ATTO, MegaRed, Alexa, BODIPY und TAMRA

Es wurden beispielsweise folgende erfindungsgemäße Verbindungen der allgemeinen Formel (IV) hergestellt:

Pac-$Lys^L$-agccc$T^R$aac$T^R$gcac$T^RT^R$cca$T^R$-$NH_2$
Pac-$Lys^L$-$T^RT^R$cca$T^R$cc$T^RT^R$ggagc$T^RT^R$ggc$T^R$-$NH_2$
Pac-$Lys^L$-c$T^R$aac$T^R$gcac$T^RT^R$cca$T^R$cc$T^RT^R$-$NH_2$
Pac-$Lys^L$-$T^RT^R$cccagccct$^R$aac$T^R$gcac$T^R$-$NH_2$
Pac-$Lys^L$-gaccc$T^RT^R$cccagccc$T^R$aac$T^R$-$NH_2$
Pac-$Lys^L$-gg$T^R$agaccc$T^RT^R$cccagccc$T^R$-$NH_2$
Pac-$Lys^L$-$T^RT^R$cg$T^R$cca$T^R$ggccgggg$T^R$cc-$NH_2$
Pac-$Lys^L$-$T^RT^R$cg$T^R$ccag$T^R$gccgggg$T^R$cc-$NH_2$
FluPac-$Lys^L$-c$C^R$g$G^R$g$G^R$tcgca$G^R$c$T^R$g$G^R$-$NH_2$

FluPac-Lys$^L$-cC$^R$gG$^R$gG$^R$tcccgG$^R$gG$^R$gC$^R$-NH$_2$
FluPac-Lys$^L$-CC$^R$aT$^R$gG$^R$ccgggG$^R$tC$^R$cC$^R$-NH$_2$
FluPac-Lys$^L$-GT$^R$tC$^R$gT$^R$ccatgG$^R$cC$^R$gG$^R$-NH$_2$
FluPac-Lys$^L$-GG$^R$gG$^R$gA$^R$acagtT$^R$cG$^R$tC$^R$-NH$_2$
FluPac-LyS$^L$-GA$^R$GG$^R$gG$^R$ggaaCaG$^R$tT$^R$cG$^R$-NH$_2$
FluPac-Lys$^L$-CG$^R$gG$^R$aA$^R$gatgaG$^R$gG$^R$gG$^R$-NH$_2$
FluPac-Lys$^L$-AG$^R$aG$^R$gC$^R$ctgggC$^R$tG$^R$gC$^R$-NH$_2$
FluPac-Lys$^L$-cc$^R$aC$^R$aT$^R$aggggC$^R$CA$^R$gA$^R$-NH$_2$
FluPac-Lys$^L$-tT$^R$gG$^R$gC$^R$tgctcA$^R$aT$^R$gA$^R$-NH$_2$
FluPac-Lys$^L$-cG$^R$cG$^R$gA$^R$gcgccC$^R$cT$^R$cG$^R$-NH$_2$
FluPac-Lys$^L$-tG$^R$gG$^R$gT$^R$gggtcT$^R$tG$^R$gT$^R$-NH$_2$
FluPac-Lys$^L$-gT$^R$cG$^R$cT$^R$gtctcC$^R$gC$^R$tT$^R$-NH$_2$
FluPac-Lys$^L$-aG$^R$cT$^R$gA$^R$ccctgA$^R$aG$^R$tT$^R$-NH$_2$
FluPac-Lys$^L$-aG$^R$cT$^R$gA$^R$cctgcA$^R$aG$^R$tT$^R$-NH$_2$
FluPac-Lys$^L$-cC$^R$gG$^R$gG$^R$tcgcaG$^R$cT$^R$GA$^R$-NH$_2$
FluPac-Lys$^L$-gC$^R$cG$^R$gG$^R$gtcgcA$^R$gC$^R$tG$^R$-NH$_2$
FluPac-Lys$^L$-cC$^R$aT$^R$gG$^R$tcaggG$^R$tC$^R$cC$^R$-NH$_2$
FluPac-Lys$^L$-gC$^R$cT$^R$gG$^R$gctggC$^R$tC$^R$tG$^R$-NH$_2$
FluPac-Lys$^L$-cC$^R$aC$^R$aT$^R$aaggcC$^R$cA$^R$gA$^R$-NH$_2$
FluPac-Lys$^L$-tG$^R$gT$^R$gG$^R$tatctG$^R$tG$^R$cT$^R$-NH$_2$
FluPac-Lys$^L$-tT$^R$gA$^R$tC$^R$ttgatG$^R$gT$^R$gG$^R$-NH$_2$
FluPac-Lys$^L$-tG$^R$gG$^R$gT$^R$gggtcT$^R$tG$^R$gT$^R$-NH$_2$
FluPaC-Lys$^L$-CC$^R$tatC$^R$aC$^R$gA$^R$ttagcA$^R$tT$^R$aA$^R$-NH$_2$
Flupac-Lys$^L$-cC$^R$catG$^R$gA$^R$aT$^R$tcagtT$^R$cT$^R$CA$^R$-NH$_2$
FluPac-Lys$^L$-cC$^R$tatC$^R$AC$^R$gA$^R$tatgcT$^R$aT$^R$aA$^R$-NH$_2$
Ac-Lys$^L$-cC$^R$gG$^R$gG$^R$tcgcaG$^R$cT$^R$gG$^R$-NH$_2$
TML-Lys$^L$-cC$^R$gG$^R$gG$^R$tcgcaG$^R$cT$^R$gG$^R$-NH$_2$ (TML = ε-Trimethyl-lysin)
FluPac-Lys$^L$-gT$^R$cG$^R$cT$^R$gtctcC$^R$gC$^R$tT$^R$-NH$_2$
Ac-Lys$^L$-gT$^R$cG$^R$cT$^R$gtctcC$^R$gC$^R$tT$^R$-NH$_2$
FluPac-Lys$^L$-cC$^R$gG$^R$gG$^R$tgccaG$^R$cT$^R$gG$^R$-NH$_2$
FluPac-Lys$^L$-cC$^R$ggG$^R$gtgccA$^R$gctG$^R$g-NH$_2$
FluPac-Lys$^L$-cc$^R$ac$^R$aA$^R$tcagtC$^R$cT$^R$aG$^R$-NH$_2$
FluPac-Lys$^L$-cA$^R$gT$^R$cC$^R$tagaaA$^R$gA$^R$aA$^R$-NH$_2$
FluPac-Lys$^L$-aC$^R$tT$^R$tT$^R$cacctG$^R$gG$^R$tC$^R$-NH$_2$
FluPac-Lys$^L$-cA$^R$aT$^R$aC$^R$tattgC$^R$aC$^R$tG$^R$-NH$_2$
FluPac-Lys$^L$-gC$^R$tT$^R$tG$^R$acaatA$^R$cT$^R$aT$^R$-NH$_2$
FluPac-Lys$^L$-tG$^R$aC$^R$aA$^R$tactaT$^R$tG$^R$cA$^R$-NH$_2$
FluPac-Lys$^L$-aG$^R$tA$^R$tT$^R$ggaccC$^R$tT$^R$aC$^R$-NH$_2$
FluPac-Lys$^L$-gA$^R$aC$^R$aG$^R$tattgG$^R$aC$^R$cC$^R$-NH$_2$
FluPac-Lys$^L$-gA$^R$acaG$^R$tA$^R$tT$^R$ggaccC$^R$tT$^R$aC$^R$-NH$_2$
FluPac-Lys$^L$-tC$^R$aG$^R$tC$^R$tgataA$^R$gC$^R$tA$^R$-NH$_2$
FluPac-Lys$^L$-tC$^R$aA$^R$CA$^R$tcagtC$^R$tG$^R$aT$^R$-NH$_2$
FluPac-Lys$^L$-aC$^R$aT$^R$cA$^R$gtctgA$^R$tA$^R$aG$^R$-NH$_2$
FluPac-Lys$^L$-cC$^R$gG$^R$gG$^R$tC$^R$gC$^R$aG$^R$cT$^R$gG$^R$-NH$_2$
FluPac-Lys$^L$(ATTO647)-cC$^R$gG$^R$gG$^R$tcgcaG$^R$cT$^R$gG$^R$-NH$_2$
FluPac-Lys$^L$(MegaRed)-cC$^R$gG$^R$gG$^R$tcgcaG$^R$cT$^R$gG$^R$-NH$_2$
FluPac-Lys$^L$(ATTO647)-cC$^R$gG$^R$gG$^R$tC$^R$gC$^R$aG$^R$cT$^R$gG$^R$-NH$_2$
FluPac-Lys$^L$(MegaRed)-cC$^R$gG$^R$gG$^R$tC$^R$gC$^R$aG$^R$cT$^R$gG$^R$-NH$_2$
FluPac-gG$^R$ccaA$^R$aC$^R$cT$^R$cggctT$^R$aC$^R$cT$^R$-NH$_2$
FluPac-gG$^R$ccaA$^R$aC$^R$cT$^R$gcgctT$^R$aC$^R$cT$^R$-NH2
FluPac-Lys$^L$(ATTO)-cC$^R$gG$^R$gG$^R$tcgcaG$^R$cT$^R$gG$^R$-NH$_2$
FluPac-Lys$^L$(ATTO)-cC$^R$gG$^R$gG$^R$tC$^R$gC$^R$aG$^R$cT$^R$gG$^R$-NH$_2$
FluPac-Lys$^L$(ATTO)-cC$^R$gG$^R$gG$^R$tgccaG$^R$cT$^R$gG$^R$-NH$_2$
Ac-Lys$^L$(ATTO)-cC$^R$gG$^R$gG$^R$tcgcaG$^R$cT$^R$gG$^R$-NH$_2$
Ac-Lys$^L$(ATTO)-cC$^R$gG$^R$gG$^R$tcgcaG$^R$CT$^R$g-NH$_2$
FluPac-Lys$^L$(ATTO)-gT$^R$cG$^R$cT$^R$gtctcC$^R$gC$^R$tT$^R$-NH$_2$
FluPac-Lys$^L$(ATTO)-cC$^R$ggG$^R$gtgccA$^R$gctG$^R$g-NH$_2$
FluPac-Lys$^L$(ATTO)-C$^R$cgggG$^R$tC$^R$gC$^R$agctgG$^R$-NH$_2$

FluPac-Lys$^{L}$(Alexa)-cC$^{R}$gG$^{R}$gG$^{R}$tcgcaG$^{R}$cT$^{R}$gG$^{R}$-$NH_2$

FluPac-Lys$^{L}$(BODIPY)-cC$^{R}$gG$^{R}$gG$^{R}$tcgcaG$^{R}$cT$^{R}$gG$^{R}$-$NH_2$

FluPac-Lys$^{L}$-G$^{R}$gC$^{R}$caaA$^{R}$cctcgG$^{R}$cttacC$^{R}$tG$^{R}$aA$^{R}$aT$^{R}$-$NH_2$

FluPac-Lys$^{L}$-G$^{R}$gC$^{R}$CaaA$^{R}$cctgcG$^{R}$cttacC$^{R}$tG$^{R}$aA$^{R}$aT$^{R}$-$NH_2$

FluPac-Lys$^{L}$-A$^{R}$cC$^{R}$aT$^{R}$agcgaG$^{R}$gT$^{R}$gA$^{R}$-$NH_2$

FluPac-Lys$^{L}$-G$^{R}$aC$^{R}$gA$^{R}$accatA$^{R}$gC$^{R}$gA$^{R}$-$NH_2$

FluPac-Lys$^{L}$-G$^{R}$aG$^{R}$gC$^{R}$agacgA$^{R}$aC$^{R}$cA$^{R}$-$NH_2$

FluPac-Lys$^{L}$-A$^{R}$aG$^{R}$cA$^{R}$gccccA$^{R}$gA$^{R}$gG$^{R}$-$NH_2$

FluPac-Lys$^{L}$-A$^{R}$gC$^{R}$gG$^{R}$tcagcA$^{R}$aG$^{R}$cA$^{R}$-$NH_2$

FluPac-Lys$^{L}$-G$^{R}$aT$^{R}$gG$^{R}$acagcG$^{R}$gT$^{R}$cA$^{R}$-$NH_2$

FluPac-Lys$^{L}$-C$^{R}$tA$^{R}$aA$^{R}$aacagA$^{R}$aT$^{R}$tG$^{R}$-$NH_2$

FluPac-Lys$^{L}$-G$^{R}$aC$^{R}$cT$^{R}$aaaaaC$^{R}$aG$^{R}$aA$^{R}$-$NH_2$

FluPac-Lys$^{L}$-G$^{R}$aT$^{R}$gG$^{R}$acctaA$^{R}$aA$^{R}$aC$^{R}$-$NH_2$

FluPac-Lys$^{L}$-T$^{R}$gG$^{R}$tT$^{R}$ctggaT$^{R}$gG$^{R}$aC$^{R}$-$NH_2$

FluPac-Lys$^{L}$-T$^{R}$tT$^{R}$tC$^{R}$tctgcA$^{R}$tG$^{R}$cA$^{R}$-$NH_2$

FluPac-Lys$^{L}$-C$^{R}$tG$^{R}$tT$^{R}$tttctC$^{R}$tG$^{R}$cA$^{R}$-$NH_2$

FluPac-Lys$^{L}$-A$^{R}$gG$^{R}$tA$^{R}$ctgttT$^{R}$tT$^{R}$cT$^{R}$-$NH_2$

FluPac-Lys$^{L}$-A$^{R}$tT$^{R}$gG$^{R}$agaagA$^{R}$aG$^{R}$cC$^{R}$-$NH_2$

FluPac-Lys$^{L}$-T$^{R}$gA$^{R}$cA$^{R}$tttttC$^{R}$gA$^{R}$aA$^{R}$-$NH_2$

FluPac-Lys$^{L}$-T$^{R}$gT$^{R}$cC$^{R}$aagggT$^{R}$gA$^{R}$cA$^{R}$-$NH_2$

FluPac-Lys$^{L}$-C$^{R}$tT$^{R}$gT$^{R}$ccaagG$^{R}$gT$^{R}$gA$^{R}$-$NH_2$

FluPac-Lys$^{L}$-A$^{R}$cC$^{R}$tT$^{R}$gtccaA$^{R}$gG$^{R}$gT$^{R}$-$NH_2$

FluPac-Lys$^{L}$-A$^{R}$tA$^{R}$cC$^{R}$ttgtcC$^{R}$aA$^{R}$gG$^{R}$-$NH_2$

FluPac-Lys$^{L}$-C$^{R}$tT$^{R}$aT$^{R}$accttG$^{R}$tC$^{R}$cA$^{R}$-$NH_2$

FluPac-Lys$^{L}$-A$^{R}$cT$^{R}$gC$^{R}$aacctC$^{R}$cA$^{R}$cC$^{R}$-$NH_2$

FluPac-Lys$^{L}$-T$^{R}$cA$^{R}$cT$^{R}$gcaacC$^{R}$tC$^{R}$cA$^{R}$-$NH_2$

FluPac-Lys$^{L}$-C$^{R}$aG$^{R}$cT$^{R}$cactgC$^{R}$aA$^{R}$cC$^{R}$-$NH_2$

FluPac-Lys$^{L}$-C$^{R}$tC$^{R}$aG$^{R}$ctcacT$^{R}$gC$^{R}$aA$^{R}$-$NH_2$

FluPac-Lys$^{L}$-A$^{R}$tC$^{R}$tC$^{R}$agctcA$^{R}$cT$^{R}$gC$^{R}$-$NH_2$

FluPac-Lys$^{L}$-C$^{R}$gA$^{R}$tC$^{R}$tcagcT$^{R}$cA$^{R}$cT$^{R}$-$NH_2$

FluPac-Lys$^{L}$-C$^{R}$gC$^{R}$gA$^{R}$tctcaG$^{R}$cT$^{R}$cA$^{R}$-$NH_2$

FluPac-Lys$^{L}$-G$^{R}$gC$^{R}$gC$^{R}$gatctC$^{R}$aG$^{R}$cT$^{R}$-$NH_2$

FluPac-Lys$^{L}$-G$^{R}$tG$^{R}$gC$^{R}$gcgatC$^{R}$tC$^{R}$aG$^{R}$-$NH_2$

FluPac-Lys$^{L}$-C$^{R}$tC$^{R}$aG$^{R}$ctcacT$^{R}$gC$^{R}$aA$^{R}$-$NH_2$

FluPac-Lys$^{L}$-G$^{R}$aT$^{R}$gG$^{R}$caaacA$^{R}$gG$^{R}$aT$^{R}$-$NH_2$

FluPac-Lys$^{L}$-C$^{R}$aC$^{R}$cA$^{R}$agaggA$^{R}$tG$^{R}$gC$^{R}$-$NH_2$

FluPac-Lys$^{L}$-A$^{R}$gA$^{R}$cC$^{R}$agcaCC$^{R}$aA$^{R}$gA$^{R}$-$NH_2$

FluPac-Lys$^{L}$-A$^{R}$cT$^{R}$cA$^{R}$ctgatA$^{R}$aA$^{R}$gA$^{R}$-$NH_2$

FluPac-Lys$^{L}$-C$^{R}$cT$^{R}$gA$^{R}$ggactC$^{R}$aC$^{R}$tG$^{R}$-$NH_2$

FluPac-Lys$^{L}$-T$^{R}$cC$^{R}$cC$^{R}$acctgA$^{R}$gG$^{R}$aC$^{R}$-$NH_2$

FluPac-Lys$^{L}$-T$^{R}$gG$^{R}$CC$^{R}$accttT$^{R}$tC$^{R}$tA$^{R}$-$NH_2$

FluPac-Lys$^{L}$-T$^{R}$tC$^{R}$tT$^{R}$ggccaC$^{R}$cT$^{R}$tT$^{R}$-$NH_2$

FluPac-Lys$^{L}$-T$^{R}$tg$^{R}$GC$^{R}$ttcttG$^{R}$gC$^{R}$cA$^{R}$-$NH_2$

FluPac-Lys$^{L}$-T$^{R}$tG$^{R}$gT$^{R}$tggctT$^{R}$CT$^{R}$tG$^{R}$-$NH_2$

FluPac-Lys$^{L}$-T$^{R}$aC$^{R}$cT$^{R}$tattgG$^{R}$tT$^{R}$gG$^{R}$-$NH_2$

FluPac-Lys$^{L}$-C$^{R}$cT$^{R}$aC$^{R}$cttatT$^{R}$gG$^{R}$tT$^{R}$-$NH_2$

FluPac-Lys$^{L}$-T$^{R}$gA$^{R}$cC$^{R}$tacctT$^{R}$aT$^{R}$tG$^{R}$-$NH_2$

FluPac-Lys$^{L}$-G$^{R}$gG$^{R}$tG$^{R}$acctaC$^{R}$cT$^{R}$tA$^{R}$-$NH_2$

FluPac-Lys$^{L}$-aG$^{R}$aG$^{R}$cagaaC$^{R}$cT$^{R}$-$NH_2$

FluPac-Lys$^{L}$-aG$^{R}$aG$^{R}$cA$^{R}$gaaccT$^{R}$tA$^{R}$c-$NH_2$

FluPac-Lys$^{L}$-aG$^{R}$agC$^{R}$agaacC$^{R}$ttaC$^{R}$t-$NH_2$

FluPac-Lys$^{L}$-agagcA$^{R}$gA$^{R}$aC$^{R}$cT$^{R}$tact-$NH_2$

FluPac-Lys$^{L}$-gC$^{R}$tA$^{R}$tT$^{R}$accttA$^{R}$aC$^{R}$cC$^{R}$-$NH_2$

FluPac-Lys$^{L}$-cA$^{R}$aT$^{R}$cA$^{R}$gacctA$^{R}$gG$^{R}$aA$^{R}$-$NH_2$

FluPac-Lys$^{L}$-tT$^{R}$cT$^{R}$gC$^{R}$tctcgT$^{R}$cC$^{R}$tG$^{R}$-$NH_2$

FluPac-Lys$^{L}$-gT$^{R}$cG$^{R}$cG$^{R}$agacaC$^{R}$gC$^{R}$tT$^{R}$-$NH_2$

FluPac-LyS$^{L}$-aG$^{R}$aG$^{R}$cA$^{R}$gaacct$^{R}$tA$^{R}$cT$^{R}$-$NH_2$

FluPac-Lys$^{L}$-gT$^{R}$cG$^{R}$cT$^{R}$gtctcC$^{R}$gC$^{R}$tT$^{R}$-$NH_2$

FluPac-Lys$^{L}$-cC$^{R}$tatC$^{R}$aC$^{R}$gA$^{R}$tatgcT$^{R}$aT$^{R}$aA$^{R}$-NH$_2$
MN-Lys$^{L}$-tG$^{R}$cC$^{R}$tA$^{R}$ggactC$^{R}$cA$^{R}$gC$^{R}$-NH$_2$
MN = 4-Hydroxy-3-nitro-phenylacetat-Rest
MN-Lys$^{L}$(TAMRA)-tG$^{R}$cC$^{R}$tA$^{R}$ggactC$^{R}$cA$^{R}$gC$^{R}$-NH$_2$
MN-Lys$^{L}$(ATTO)-tG$^{R}$cC$^{R}$tA$^{R}$ggactC$^{R}$cA$^{R}$gC$^{R}$-NH$_2$

FluPac-Gly-aG$^{R}$aG$^{R}$cA$^{R}$gaaccT$^{R}$tA$^{R}$c-NH$_2$
FluPac-Lys$^{D}$-cT$^{R}$gA$^{R}$aA$^{R}$ttttcG$^{R}$aA$^{R}$gT$^{R}$-NH$_2$
FluPac-Lys$^{D}$-tT$^{R}$aC$^{R}$cT$^{R}$gaaatT$^{R}$tT$^{R}$cG$^{R}$-NH$_2$
FluPac-Lys$^{D}$-CG$^{R}$gC$^{R}$tT$^{R}$acctgA$^{R}$aA$^{R}$tT$^{R}$-NH$_2$
FluPac-Lys$^{D}$-CT$^{R}$cG$^{R}$gC$^{R}$ttaccT$^{R}$gA$^{R}$aA$^{R}$-NH$_2$
FluPac-Lys$^{D}$-aC$^{R}$cT$^{R}$cG$^{R}$gcttaC$^{R}$cT$^{R}$gA$^{R}$-NH$_2$
FluPac-Lys$^{D}$-aA$^{R}$aC$^{R}$cT$^{R}$cggctT$^{R}$aC$^{R}$cT$^{R}$-NH$_2$
FluPac-Lys$^{D}$-cC$^{R}$aA$^{R}$aC$^{R}$ctcggC$^{R}$tT$^{R}$aC$^{R}$-NH$_2$
FluPac-Lys$^{D}$-aA$^{R}$gG$^{R}$cC$^{R}$aaaccT$^{R}$cG$^{R}$gC$^{R}$-NH$_2$
FluPac-Gly-aG$^{R}$aG$^{R}$cA$^{R}$gaaccT$^{R}$tA$^{R}$cT$^{R}$-NH$_2$
FluPac-Gly-aG$^{R}$agcA$^{R}$gaaccttA$^{R}$ct-NH$_2$
FluPac-Gly-aG$^{R}$aA$^{R}$gA$^{R}$cgttcC$^{R}$aA$^{R}$CT$^{R}$-NH$_2$
FluPac-Gly-aG$^{R}$cG$^{R}$aA$^{R}$gcataT$^{R}$aT$^{R}$cC$^{R}$-NH$_2$
FluPac-Gly-aC$^{R}$aG$^{R}$gA$^{R}$cgagaG$^{R}$cA$^{R}$gA$^{R}$-NH$_2$
FluPac-Gly-aC$^{R}$cT$^{R}$tA$^{R}$cttttC$^{R}$cT$^{R}$cT$^{R}$-NH$_2$
FluPac-Gly-caC$^{R}$aG$^{R}$atgacA$^{R}$tT$^{R}$aG$^{R}$-NH$_2$
FluPac-Gly-cA$^{R}$aT$^{R}$cA$^{R}$gacctA$^{R}$gG$^{R}$aA$^{R}$-NH$_2$
FluPac-Gly-aC$^{R}$aC$^{R}$cC$^{R}$acaatC$^{R}$aG$^{R}$tC$^{R}$-NH$_2$
FluPac-Gly-aC$^{R}$CC$^{R}$aC$^{R}$aatCaG$^{R}$tC$^{R}$cT$^{R}$-NH$_2$
FluPac-Gly-aC$^{R}$aA$^{R}$tC$^{R}$agtccT$^{R}$aG$^{R}$aA$^{R}$-NH$_2$
FluPac-Gly-aA$^{R}$tC$^{R}$aG$^{R}$tcctaG$^{R}$aA$^{R}$aG$^{R}$-NH$_2$
FluPac-Gly-tC$^{R}$aG$^{R}$tC$^{R}$ctagaA$^{R}$aG$^{R}$aA$^{R}$-NH$_2$
FluPac-Gly-aG$^{R}$tC$^{R}$cT$^{R}$agaaaG$^{R}$aA$^{R}$aA$^{R}$-NH$_2$
FluPac-Gly-gG$^{R}$aT$^{R}$gG$^{R}$actctT$^{R}$aC$^{R}$tT$^{R}$-NH$_2$
FluPac-Gly-aT$^{R}$gG$^{R}$aC$^{R}$tcttaC$^{R}$tT$^{R}$tT$^{R}$-NH$_2$
FluPac-Gly-aC$^{R}$tC$^{R}$tT$^{R}$actttT$^{R}$cA$^{R}$cC$^{R}$-NH$_2$
FluPac-Gly-tC$^{R}$tT$^{R}$aC$^{R}$ttttcA$^{R}$cC$^{R}$tG$^{R}$-NH$_2$
FluPac-Gly-tT$^{R}$aC$^{R}$tT$^{R}$ttcacC$^{R}$tG$^{R}$gG$^{R}$-NH$_2$
FluPac-Gly-tT$^{R}$tT$^{R}$cA$^{R}$cctggG$^{R}$tC$^{R}$aT$^{R}$-NH$_2$
FluPac-Gly-tC$^{R}$cA$^{R}$aC$^{R}$aatcaG$^{R}$aC$^{R}$cT$^{R}$-NH$_2$
FluPac-Gly-cA$^{R}$aC$^{R}$aA$^{R}$tcagaC$^{R}$cT$^{R}$aG$^{R}$-NH$_2$
FluPac-Gly-aC$^{R}$aA$^{R}$tC$^{R}$agaccT$^{R}$aG$^{R}$gA$^{R}$-NH$_2$
FluPac-Gly-aA$^{R}$tC$^{R}$aG$^{R}$acctaG$^{R}$gA$^{R}$aA$^{R}$-NH$_2$
FluPac-Gly-tC$^{R}$aG$^{R}$aC$^{R}$ctaggA$^{R}$aA$^{R}$aC$^{R}$-NH$_2$
FluPac-Gly-aG$^{R}$aC$^{R}$cT$^{R}$aggaaA$^{R}$ac$^{R}$gG$^{R}$-NH$_2$
FluPac-Gly-aC$^{R}$gA$^{R}$gA$^{R}$gcagaA$^{R}$cC$^{R}$tT$^{R}$-NH$_2$
FluPac-Gly-gA$^{R}$gA$^{R}$gC$^{R}$agaacC$^{R}$tT$^{R}$aC$^{R}$-NH$_2$
FluPac-Gly-gA$^{R}$gC$^{R}$aG$^{R}$aacctT$^{R}$aC$^{R}$tT$^{R}$-NH$_2$
FluPac-Gly-gC$^{R}$aG$^{R}$aA$^{R}$ccttaC$^{R}$tT$^{R}$tT$^{R}$-NH$_2$
FluPac-Gly-aG$^{R}$aA$^{R}$cC$^{R}$ttactT$^{R}$tT$^{R}$cC$^{R}$-NH$_2$
FluPac-Gly-aA$^{R}$cC$^{R}$tT$^{R}$actttT$^{R}$cC$^{R}$tC$^{R}$-NH$_2$
FluPac-Gly-cA$^{R}$gT$^{R}$cC$^{R}$tagaaA$^{R}$gA$^{R}$aA$^{R}$-NH$_2$
FluPac-Gly-aA$^{R}$aC$^{R}$cT$^{R}$CggCtT$^{R}$aC$^{R}$cT$^{R}$-NH$_2$
FluPac-Lys$^{D}$-cC$^{R}$gG$^{R}$gG$^{R}$tcgcaG$^{R}$cT$^{R}$gG$^{R}$-NH$_2$
Ac-Lys$^{D}$-cC$^{R}$gG$^{R}$gG$^{R}$tcgcaG$^{R}$cT$^{R}$gG$^{R}$-NH$_2$
FluPac-Lys$^{D}$-cC$^{R}$ggggtcgC$^{R}$aG$^{R}$cT$^{R}$gG$^{R}$-NH$_2$
FluPac-Lys$^{D}$-aG$^{R}$aG$^{R}$cA$^{R}$gaaccT$^{R}$tA$^{R}$cT$^{R}$-NH$_2$
AC-Lys$^{D}$-aG$^{R}$aG$^{R}$CA$^{R}$gaaccT$^{R}$tA$^{R}$cT$^{R}$-NH$_2$
FluPac-Lys$^{D}$-aG$^{R}$agcagaaC$^{R}$cT$^{R}$tA$^{R}$cT$^{R}$-NH$_2$
FluPac-LyS$^{D}$-cC$^{R}$aC$^{R}$aA$^{R}$tcagtC$^{R}$cT$^{R}$aG$^{R}$-NH$_2$
Ac-LysD-cC$^{R}$aC$^{R}$aA$^{R}$tcagtC$^{R}$cT$^{R}$aG$^{R}$-NH$_2$
FluPac-Lys$^{D}$-cC$^{R}$acaatcaG$^{R}$tC$^{R}$aT$^{R}$aG$^{R}$-NH$_2$

FluPac-Gly-aC$^{R}$aT$^{R}$cA$^{R}$gtctgA$^{R}$tA$^{R}$aG$^{R}$-$NH_2$

FluPac-Gly-gG$^{R}$gG$^{R}$tC$^{R}$atcaaG$^{R}$gG$^{R}$tG$^{R}$-$NH_2$

FluPac-Gly-cC$^{R}$aC$^{R}$aG$^{R}$atgacA$^{R}$tT$^{R}$aG$^{R}$-$NH_2$

FluPac-Gly-C$^{R}$caC$^{R}$aA$^{R}$tcagtC$^{R}$cT$^{R}$aG$^{R}$-$NH_2$

FluPac-Gly-C$^{R}$caC$^{R}$aA$^{R}$tcagtC$^{R}$cT$^{R}$ag-$NH_2$

FluPac-Gly-ccaC$^{R}$aA$^{R}$tcagtC$^{R}$cT$^{R}$aG$^{R}$-$NH_2$

FluPac-Gly-ccaC$^{R}$aA$^{R}$tcagtC$^{R}$cT$^{R}$ag-$NH_2$

FluPac-Gly-cC$^{R}$aC$^{R}$aA$^{R}$tcagtC$^{R}$cT$^{R}$aG$^{R}$-$NH_2$

FluPac-Lys$^{D}$(ATTO)-cC$^{R}$gG$^{R}$gG$^{R}$tcgcaG$^{R}$cT$^{R}$gG$^{R}$-$NH_2$

Ac-LyS$^{D}$(ATTO)-cC$^{R}$gG$^{R}$gG$^{R}$tcgcaG$^{R}$cT$^{R}$gG$^{R}$-$NH_2$

FluPac-Lys$^{D}$(ATTO)-cC$^{R}$ggggtcgC$^{R}$aG$^{R}$cT$^{R}$gG$^{R}$-$NH_2$

FluPac-Lys$^{D}$(ATTO)-aG$^{R}$aG$^{R}$cA$^{R}$gaaccT$^{R}$tA$^{R}$cT$^{R}$-$NH_2$

Ac-LyS$^{D}$(ATTO)-aG$^{R}$aG$^{R}$cA$^{R}$gaaccT$^{R}$tA$^{R}$cT$^{R}$-$NH_2$

FluPac-Lys$^{D}$(ATTO)-aG$^{R}$agcagaaC$^{R}$CT$^{R}$tA$^{R}$CT$^{R}$-$NH_2$

FluPac-Lys$^{D}$(ATTO)-cC$^{R}$aC$^{R}$aA$^{R}$tcagtC$^{R}$cT$^{R}$aG$^{R}$-$NH_2$

Ac-LyS$^{D}$(ATTO)-cC$^{R}$aC$^{R}$aA$^{R}$tcagtC$^{R}$cT$^{R}$aG$^{R}$-$NH_2$

FluPac-Lys$^{D}$(ATTO)-cC$^{R}$acaatcaG$^{R}$tC$^{R}$cT$^{R}$aG$^{R}$-$NH_2$

FluPac-Lys$^{D}$(Chol)-gA$^{R}$gA$^{R}$gC$^{R}$agaacC$^{R}$tT$^{R}$aC$^{R}$-$NH_2$

Chol =

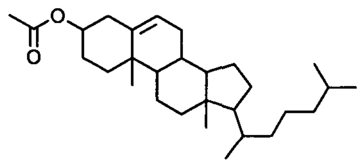

FluPac-Lys$^{D}$(Fol)-gA$^{R}$gA$^{R}$gC$^{R}$agaacCC$^{R}$tT$^{R}$aC$^{R}$-$NH_2$

Fol =

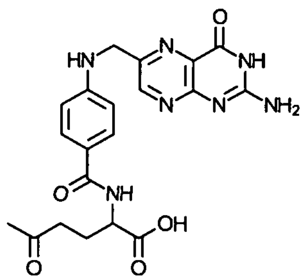

FluPac-Lys$^{D}$(Dans)-gA$^{R}$gA$^{R}$gC$^{R}$agaacC$^{R}$tT$^{R}$aC$^{R}$-$NH_2$

Dans =

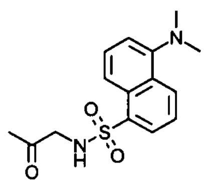

FluPac-Gly-aG$^{R}$aG$^{R}$cA$^{R}$gaaccT$^{R}$tA$^{R}$c-$NH_2$

FluPac-Gly-aG$^{R}$aG$^{R}$Ca$^{R}$gaaccT$^{R}$tA$^{R}$cT$^{R}$-$NH_2$

FluPac-Lys$^{D}$-gA$^{R}$gC$^{R}$aG$^{R}$aacctT$^{R}$aC$^{R}$tT$^{R}$-$NH_2$

FluPac-Lys$^{D}$-gA$^{R}$gaG$^{R}$cagaaC$^{R}$ctta$^{R}$c-$NH_2$

AC-Lys$^{D}$-gA$^{R}$gaG$^{R}$cagaaC$^{R}$ctta$^{R}$c-$NH_2$

FluPac-Gly-gA$^{R}$gcA$^{R}$gaaccT$^{R}$tacT$^{R}$t-$NH_2$

FluPac-Gly-cagtccT$^{R}$aG$^{R}$aA$^{R}$agaaa-$NH_2$

FluPac-Gly-gG$^{R}$ccaA$^{R}$aC$^{R}$cT$^{R}$cggctT$^{R}$aC$^{R}$T$^{R}$-$NH_2$

FluPac-Gly-cagtcc$T^R$a$G^R$a$A^R$agaaa-$NH_2$

**Beispiel 14: Verbesserte Bioverfügbarkeit und verlängerte Halbwertszeit in verschiedenen Organen/Geweben**

Ein $^3H$ markiertes N-Phos-Oligomer sowie ein $^3H$ markiertes EP2041161-Oligomer werden jeweils in PBS (pH 7.1) gelöst und in einer Konzentration von 10mg/kg Mäusen mittels einer intravenösen Bolusinjektion verabreicht. Nach verschiedenen Zeitpunkten (20 min, 1,5 Stunden, 3 Stunden, 6 Stunden, 24 Stunden, 2 Tagen, 4 Tagen, 8 Tagen und 14 Tagen) werden den Mäusen Blut sowie 18 verschiedene Organe/Gewebe (Niere, Leber, Milz, Knochenmark, Lymphknoten, Lunge, Dickdarm, Dünndarm, Bauchspeicheldrüse, Harnblase, Herz, Thymusdrüse, Magen, Muskel, Großhirn, Kleinhirn, Prostata und Haut) entnommen und die $^3H$-Konzentration in dem jeweiligen Gewebe gemessen. Die pharmakokinetische Analyse wurde mittels der validierten professionellen WinNonlin Software, Version 4.0.1 (Pharsight Corporation, Mountain View, USA), durchgeführt. Die Radioaktivität in den jeweiligen Organen/Geweben (Mittelwert von drei Tieren pro Probeentnahme-Punkt) im Verlauf der Zeit wurde ohne angenommene Kompartimente evaluiert, um die Bioverfügbarkeit (ausgedrückt als Fläche unter der Kurve) und die Halbwertszeiten in Organen/Geweben zu berechnen.
Es wurde gefunden, dass die Bioverfügbarkeit des $^3H$ markierten N-Phos-Oligomers gegenüber der Bioverfügbarkeit $^3H$ markierten EP2041161-Oligomers über den Zeitraum von 14 Tagen in allen Geweben um das 1,7-4,6 fache erhöht ist. Die Ergebnisse sind in Abbildung 1 dargestellt.
Weiterhin wurde gefunden, dass die Halbwertszeit des $^3H$ markierten N-Phos-Oligomers gegenüber der Halbwertszeit des $^3H$ markierten EP2041161-Oligomers über den Zeitraum von 14 Tagen in den meisten Geweben, in der Milz sogar um das 2-fache, erhöht ist. Die Ergebnisse sind in Abbildung 2 dargestellt.

**Beispiel 15: Erhöhte Bindung an Plasmaproteine**

Die Bindung an Humanes Serum Albumin wurde auf einer 5 cm HPLC Säule von Chromtech (4.0 x 50mm, 5$\mu$m) ermittelt. Auf dieser Säule ist Humanes Serum Albumin immobilisiert, so dass über die Retentionzeiten die Bindungsaffinität zu Humanen Serum Albumin ermittelt werden kann. Als Eluent wurde ein 30 % Isoprop/Ammoniumacetatpuffer (pH 7) verwendet.
Die Affinitätskonstante wird nach Angaben des Herstellers mit folgender Formel berechnet:

$$k' = (tr - tm)/tm$$

tr = Retentionszeit der aufgetragenen Probe
tm = Retentionszeit von Acetaminophen

Mit diesem Wert errechnet sich die Bindung P (in %) an Humanes Serum Albumin zu:

$$P = 100(k'/(k' + 1))$$

Ein höherer Wert ist ein Indikator für eine vorteilhafte Verteilung *in vivo.* Die anschließende Tabelle zeigt eine deutlich stärkere Bindung des N-Phos-Oligomers an Serum Albumin im Vergleich zum EP2041161-Oligomer.

| Tabelle zum Vergleich der Plasma-Protein-Bindung von N-Phos-Oligomeren mit EP2041161-Oligomeren | | |
|---|---|---|
| | Plasma-Protein-Bindung (%) | |
| Sequenz: | N-Phos-Oligomer[a] | EP2041161-Oligomer[b] |
| FluPac-Gly-c$A^R$g$T^R$c$C^R$tagaa$A^R$g$A^R$a$A^R$-$NH_2$ | 94 | 71,5 |
| [a]N-Phos-Oligomer: $R^1 = -CH_2-CH_2-CH_2-CH_2-NH-CH_2-CH_2-P{=}O(OEt)_2$<br>[b]EP2041161-Oligomer: $R^1 = -CH_2-CH_2-CH_2-P{=}O(OEt)_2$ | | |

**Beispiel 16: Verbesserte und sequenzunabhängige Wasserlöslichkeit**

Verschiedene N-Phos-Oligomere sowie EP2041161-Oligomere werden eingewogen und so viel PBS (pH 7.2) hinzugegeben, dass theoretisch eine 100$\mu$M Lösung entsteht. Anschließend werden die OD-Werte der resultierenden Lösungen gemessen. Dann werden die OD-Werte der N-Phos-Oligomere sowie der EP2041161-Oligomere miteinander

verglichen, wobei ein hoher OD-Wert einer höheren Anzahl von gelösten Molekülen und damit einer höheren Löslichkeit entspricht. Während sich aus den EP2041161-Oligomeren, deren Sequenz eine größere Anzahl von Guanin- und Cytosin-Basen umfasst, keine 100μM Lösung in PBS herstellen lässt, in der die oligomeren Verbindungen vollständig gelöst sind, sind die N-Phos-Oligomere bei der Herstellung einer 100μM Lösung hingegen vollständig in PBS gelöst. Die anschließende Tabelle zeigt, dass sequenzunabhängig die Wasserlöslichkeit der N-Phos-Oligomere im Vergleich zu den EP2041161-Oligomeren überraschend deutlich erhöht ist.

| Tabelle zum Vergleich der Wasserlöslichkeit von N-Phos-Oligomeren mit EP2041161-Oligomeren | | | | |
|---|---|---|---|---|
| | N-Phos-Oligomere[a] | | EP2041161-Oligomere[b] | |
| Sequenz | m[c] | OD-Wert[d] | m[c] | OD-Wert[d] |
| FluPac-Lys$^L$-cC$^R$gG$^R$gG$^R$tcgcaG$^R$cT$^R$gG$^R$-$NH_2$ | 2,6 | 70,4 | 2,4 | 1,4 |
| FluPac-Lys$^L$-cC$^R$gG$^R$gG$^R$tcgcaG$^R$cT$^R$gC$^R$-$NH_2$ | 2,7 | 75,7 | 2,6 | 8,2 |
| FluPac-Lys$^L$-cC$^R$aT$^R$gG$^R$ccgggG$^R$tC$^R$cC$^R$-$NH_2$ | 2,9 | 81,6 | 2,8 | 2 |
| FluPac-Lys$^L$-gT$^R$tC$^R$gT$^R$ccatgG$^R$cC$^R$gG$^R$-$NH_2$ | 2,4 | 77,4 | 2,6 | 3 |
| FluPac-Lys$^L$-gG$^R$gG$^R$gA$^R$acagtT$^R$cG$^R$tC$^R$-$NH_2$ | 2,7 | 72,8 | 2,8 | 8,6 |
| FluPac-Lys$^L$-gA$^R$gG$^R$gG$^R$gaacaG$^R$tT$^R$cG$^R$-$NH_2$ | 2,7 | 70,6 | 2,4 | 2,1 |
| FluPac-Lys$^L$-cG$^R$gG$^R$aA$^R$gatgaG$^R$gG$^R$gG$^R$-$NH_2$ | 2,5 | 63,8 | 2,3 | 4,4 |
| FluPac-Lys$^L$-aG$^R$aG$^R$gC$^R$ctgggC$^R$tG$^R$gC$^R$-$NH_2$ | 2,4 | 86 | 2,4 | 1,8 |
| FluPac-Lys$^L$-cc$^R$ac$^R$aT$^R$aggggc$^R$cA$^R$gA$^R$-$NH_2$ | 2,6 | 66,9 | 2,5 | 1,3 |
| FluPac-Lys$^L$-tT$^R$gG$^R$gC$^R$tgctcA$^R$aT$^R$gA$^R$-$NH_2$ | 3 | 77,5 | 2,3 | 11,2 |
| FluPac-Lys$^L$-cG$^R$cG$^R$gA$^R$gcgccC$^R$cT$^R$cG$^R$-$NH_2$ | 2,9 | 78,8 | 2,8 | 15,8 |
| FluPac-Lys$^L$-tG$^R$gG$^R$gT$^R$gggtcT$^R$tG$^R$gT$^R$-$NH_2$ | 2,4 | 76,6 | 1 | 5 |

[a]N-Phos-Oligomere: $R^1$ = $-CH_2-CH_2-CH_2-CH_2-NH-CH_2-CH_2-P{=}O(OEt)_2$
[b]EP2041161-Oligomere: $R^1$ = $-CH_2-CH_2-CH_2-P{=}O(OEt)_2$
[c]m = Einwaage [mg]
[d]OD-Wert = Gemessener OD-Wert nach Herstellung einer theoretisch 100 μM Lösung in PBS;

**Beispiel 17: Starke Bindung an komplementäre DNA**

Ein N-Phos-Oligomer bzw. ein EP2041161-Oligomer und das sequenzkomplementäre DNA-Oligomer werden im äquimolaren Verhältnis in Magnesium- und Kalzium-freien physiologischen PBS-Puffer gelöst. Die Lösung wird soweit verdünnt, dass im UV-Spektrometer ein OD-Wert von 0.8 gemessen wird. Mittels eines Heizbades werden die Küvetten im UV-Spektrometer stufenweise in 1 °C-Schritten von Raumtemperatur auf 95 °C erhitzt. Nach jedem 1 °C Schritt wird der OD-Wert bestimmt. Der Schmelzpunkt ergibt sich aus dem Wendepunkt der resultierenden Kurve.
Die anschließende Tabelle zeigt, dass das N-Phos-Oligomer einen höheren Schmelzpunkt als das entsprechende EP2041161-Oligomer hat. Das N-Phos-Oligomer bildet also eine stabilere Bindung zum sequenzkomplementären DNA-Oligomer aus.

| Tabelle zum Vergleich der Höhe der Schmelzpunktes N-Phos-Oligomeren mit EP2041161-Oligomeren | | |
|---|---|---|
| | Schmelzpunkt (°C) | |
| Sequenz: FluPac-Gly-cA$^R$gT$^R$cC$^R$tagaaA$^R$gA$^R$aA$^R$-$NH_2$ | N-Phos-Oligomere[a] | EP2041161-Oligomere[b] |
| DNA-Sequenz, 100% Match: 5'-TTTCTTTCTAGGACTG-3' | 73 | 69 |

[a]N-Phos-Oligomer: $R^1$ = $-CH_2-CH_2-CH_2-CH_2-NH-CH_2-CH_2-P{=}O(OEt)_2)$
[b]EP2041161-Oligomer: $R^1$ = $-CH_2-CH_2-CH_2-P{=}O(OEt)_2)$

**Beispiel 18: Down-Regulation von NFkB in HeLa-Zellen**

Zwei Tage nach der Kultivierung von HeLa Zellen (Quelle DSMZ), ausgesät in Greiner μClear 384w Platten

mit einer Dichte von 800 Zellen/well, wurden in PBS gelöste N-Phos-Oligomere bzw. EP2041161-Oligomere hinzugegeben, so dass jeweils finale Konzentrationen von 0.5, 2.5, und 10μM in Vollmedium erhalten wird. Am Tag 5 nach der Zell-Aussaat werden 20 μl/well frisches Vollmedium hinzugegeben. Am Tag 7 findet ein Wechsel auf ein Hungermedium bei 0.1% FCS statt. Am Tag 8 wird, zur Stimulation der Zellen, 10 ng/ml TNFa (Peprotech) im Medium (0.1% FCS, keine Antibiotika) hinzugegeben, die Zellen nach 30 Minuten für die morphologische Analyse fixiert (4% PFA) und zur Darstellung der wichtigsten subzellulären Strukturen wie Zellkern und Zytoplasma mit entsprechenden Farbstoffen und Antikörpern gefärbt. Die Bildverabeitung findet mit einem ImageXPress Micro automatisierten Microskop (MDC) statt. Die Bildanalyse wird visuell mit der Metamorph (MDC) Software und dann quantitativ mit der automatisierten Bildanalyse-Software Definiens XD (Definiens) unter Verwendung spezifischer Algorhithmen durchgeführt. Auf diese Weise wird anhand der Färbung mit einem Hoechst Farbstoff die Anzahl der Zellkerne bestimmt, welche als Surrogat für das Ausmaß der Zellproliferation und damit für die Down-Regulation von NFkB durch die oligomeren Verbindungen dient.
Die anschließende Tabelle zeigt anhand der niedrigeren Werte der Anzahl der Zellkerne, vor allem bei den Konzentrationen von 2,5μM und 10 μM, dass der Effekt auf die Genexpression der N-Phos-Oligomere anhand der erhöhten Down-Regulation von NFkB im Vergleich zu den EP2041161-Oligomeren verbessert ist.

Tabelle zum Vergleich der Down-Regulation von NFkB von N-Phos-Oligomeren mit EP-2041161-Oligomeren anhand der Bestimmung der Zellproliferation durch Messung der Anzahl der Zellkerne.

| Sequenz | Anzahl Zellkerne (Mittelwert normiert) | | | | | |
|---|---|---|---|---|---|---|
| | N-Phos-Oligomer[a] | | | EP-2041161-Oligomer[b] | | |
| | 0,5 μM | 2,5 μM | 10 μM | 0,5 μM | 2,5 μM | 10 μM |
| FluPac-Lys$^L$-cC$^R$gG$^R$gG$^R$tcgcaG$^R$cT$^R$gG$^R$-$NH_2$ | 97,1 | 92,0 | 68,7 | 99,3 | 102,4 | 111,5 |
| FluPac-Lys$^L$-cC$^R$gG$^R$gG$^R$tcccgG$^R$gG$^R$gC$^R$-$NH_2$ | 92,1 | 92,7 | 79,7 | 106,2 | 106,7 | 114,5 |
| FluPac-Lys$^L$-CC$^R$aT$^R$gG$^R$ccgggG$^R$tC$^R$cC$^R$-$NH_2$ | 92,3 | 83,7 | 66,6 | 105,0 | 111,8 | 119,2 |

[a]N-Phos-Oligomere: $R^1$ = $-CH_2-CH_2-CH_2-CH_2-NH-CH_2-CH_2-P{=}O(OEt)_2$
[b]EP2041161-Oligomere: $R^1$ = $-CH_2-CH_2-CH_2-P{=}O(OEt)_2$

**Beispiel 19: Stärkere Splice Site Modulation des Targets TNFR2 (Skipping von Exon 7) in THP1 Zellen**

Die Wirksamkeitstests für die oligomeren Verbindungen werden in THP1 Zellkultur von ATCC (ATCC TIB-202®™) durchgeführt. THP1 ist eine menschliche Monozyten-Zelllinie von einem akuten Monozyten-Leukämie-Patienten. THP1 Zellkulturen (in RPMI 1640-Medium mit 10% FCS) werden ohne Einsatz von Antibiotika durchgeführt. Mycoplasma-Tests (mit Venor GeM-Kit von Minerva) werden regelmäßig durchgeführt.
Am Tag 1 werden THP1-Zellen unter Zugabe von PMA bei 13.000 Zellen/Well in Greiner Kollagen I 384W Platten (#781956) mit einem Multidrop-Dispenser angesetzt. In diesem Schritt werden die Zellen in Vollmedium mit 10% Serum und Penicillin/Streptomycin behandelt. Nach dem Aussäen wird PMA (Sigma #P8139) bei 100nM zugegeben. Am Tag 4 nach Austausch des Kulturmediums werden die Oligomere in den Konzentrationen von 0,2, 2 und 20 μM hinzugefügt. An Tag 6 werden die Zellen durch Zugabe von THP1 INFg bei 100 U/ml (Peprotech) und ferner mit IFN-γ für 24h konditioniert. Am Tag 7, nach Austausch des Zellkulturmediums, werden 5 μg LPS (Sigma) im sog. Hungermedium (0,1% FCS) hinzugegeben und die Kultur für 24h stimuliert. Am Tag 8 werden THP1-Zellen in Lysepuffer Stratec S (#7061311700) lysiert und das Lysat bei -80°C gelagert. Am Tag 9 wird unter Verwendung von RNA Stratec InviTrap RNA-Extraktions-Kits Zelle (#7061300400) extrahiert und bei -80°C zur weiteren Analyse gelagert.
Die RT-Reaktion wird unter Verwendung der LifeTech High Capacity cDNA Kit (#4368813) mit RNase-Inhibitor (#N8080119) ausgeführt. Die qPCR wird bei 11 μl Reaktionsvolumen, unter Verwendung der Bioline SensiMix Sybr qPCR Mastermix (#QT605-20), mit spezifischen Primern für die mRNAs der menschlichen TNFR2 Isoformen mit und ohne Exon 7, durchgeführt.
Die qPCR-Reaktionen werden auf einem ABI PRISM 7900HT System durchgeführt. Die RT-qPCR-Daten werden manuell auf Amplifikationskurven in jedem Well überprüft. Relative mRNA des Zielgens wird auf die mRNA-Menge des Rpl13a Referenz-Gens normalisiert.
Für jede getestete Konzentration der oligomeren Verbindungen wird das Verhältnis (ausgedrückt in Prozent) der Expression der induzierten TNFR2-Isoform ohne Exon 7 in Bezug auf die Expression der TFNR2 Isoform mit Exon 7, jeweils immer relativ zur Expression des Referenz Gens Rpl13a, bestimmt. Die equipotent wirksame Konzentration,

EC50, wird auf der Grundlage der Kurvenfunktion mit dem besten statistischen Fit zu den einzelnen Konzentrationsdaten (quadratische Anpassung) mit Hilfe des Excel-Add- ins XLfit.5 (IDBS) berechnet.

Die anschließende Tabelle zeigt, dass die N-Phos-Oligomere bei der Splice Site Modulation von TNFR2 einen deutlich niedrigeren EC50-Wert als die EP2041161-Oligomere besitzen.

| Tabelle zum Vergleich des Effekts auf die Genexpression von N-Phos-Oligomeren mit EP2041161-Oligomeren am Beispiel der Splice Site Modulation des Targets TNFR2 in THP1 Zellen | | |
|---|---|---|
| | EC50 Wert (μM) | |
| Sequenz | N-Phos-Oligomere[a] | EP2041161-Oligomere[b] |
| FluPac-Gly-CA$^R$gT$^R$cC$^R$tagaaA$^R$gA$^R$aA$^R$-$NH_2$ | 28,0 | 216,6 |
| FluPac-Gly-cA$^R$gT$^R$cC$^R$tagaaA$^R$gA$^R$aa-$NH_2$ | 84,6 | 274,7 |
| [a]N-Phos-Oligomere: $R^1$ = $-CH_2-CH_2-CH_2-CH_2-NH-CH_2-CH_2-P{=}O(OEt)_2$<br>[b]EP2041161-Oligomere: $R^1$ = $-CH_2-CH_2-CH_2-P{=}O(OEt)_2$ | | |

**Beispiel 20: Splice Site Modulation des Targets TNFR2 (Skipping von Exon 7) in Mäusen**

Je eine Behandlungsgruppe mit jeweils 5 Mäusen des Stammes BalB/C (Jackson Labs) wird an den Tagen 1, 3 und 5 intravenös mit entweder 50mg/kg N-Phos-Oligomer oder PBS im gleichen Volumen injiziert. Am Tag 8 findet eine Stimulation einer Entzündungsreaktion 15mg/kg LPS (Phenol-LPS von E. coli -Serotyp Ø127: B8, Sigma Cat #L3129 mit einem Endotoxin-Wert von nicht weniger als 500.000 EU -Endotoxin Einheiten/mg) statt. 3 Stunden nach der LPS-Stimulation werden die Tiere getötet und je ca. 30mg von Milz und mesenterischen Lymphknoten präpariert und sofort in flüssigem Stickstoff eingefroren. Die Gewebeproben werden bei - 80°C im Gefrierschrank bis zur Weiterverarbeitung aufbewahrt. Für die Extraktion von RNA werden die Gewebestücke etwas unter 30mg (nach Entfernung von übermäßig vorhandenem Gewebe mit einem Skalpell) sofort in ein Röhrchen mit 300 μl QIAzol Reagenz und Edelstahl-Perlen (Qiagen cat # 69989) zur Lyse übertragen. Die Extraktion der RNA aus den Gewebeproben wird mit dem Qiagen RNeasy 96 Universal-Tissue Kit (Qiagen #74881) nach dem Protokoll des Herstellers durchgeführt. Die erhaltene RNA wird bei -80 ° C bis zur weiteren Verwendung gelagert. Bei der qPCR-Analyse wird für die RT-Reaktion der High Capacity cDNA Reverse Transkription-Kit mit RNase Inhibitor, Invitrogen (cat #4374966) gemäß Protokoll des Herstellers verwendet. Die qPCR Reaktionsansätze werden mit dem Bioline SensiMix SYBR Mastermix (#QT605-20), 11 ul Reaktionsvolumen, mit SybrGreen-basierter Erkennung und vorvalidierten Transkript-spezifischen Primer-Paaren als Triplikate angesetzt. Die Echtzeit-PCR-Reaktionen werden mit einem ABI PRISM 7900HT System durchgeführt. Die RT-qPCR-Daten wurden manuell überprüft und die Menge der Ziel-mRNA wird auf die Basis der Menge der mRNA des RNA Referenzgens Rpl13a normalisiert. Das Expressionsniveau der Ziel-mRNA, der mRNA Isoform des TNFR2 Gens der Maus ohne das Exon 7, wurde als Median der 5 Mediane der Triplikat-Messung der Milz oder der mesenterischen Lymphknoten, normalisiert auf Rpl13a jeweils einer Maus aus der Versuchsgruppe von 5 Mäusen, bestimmt.

| Tabelle zur Darstellung des starken Effekts auf die Genexpression von N-Phos-Oligomeren am Beispiel der Splice Site Modulation des Targets TNFR2 in Mäusen | | |
|---|---|---|
| | Median der Expression der TNFR2 mRNA Isoform ohne Exon 7 (ausgedrückt als Vielfaches des RNA Standards Rpl13a) | |
| PBS bzw. N-Phos-Oligomer[a] | Mesenterische Lymphknoten | Milz |
| PBS | 0.000834 | 0.001097 |
| FluPac-Gly-aG$^R$aG$^R$cA$^R$gaaccT$^R$tA$^R$cT$^R$-$NH_2$ | 0.017613 | 0.027815 |
| FluPac-Gly-gA$^R$gA$^R$cA$^R$agaacC$^R$tT$^R$aC$^R$-$NH_2$ | 0.038645 | 0.032936 |
| [a]N-Phos-Oligomere: $R^1$ = $-CH_2-CH_2-CH_2-CH_2-NH-CH_2-CH_2-P{=}O(OEt)_2$ | | |

**Beispiel 21: Wirksamkeitsvergleich zwischen einem erfindungsgemäßen N-Phos-Oligomer, einem EP 2041161-Oligomer und einem US5719262-Oligomer bei der Splice Site Modulation des Targets TNFR2 (Skipping von Exon 7) in THP1 Zellen**

Die Durchführung des Experiments erfolgte wie in Beispiel 19 beschrieben. Die Ergebnisse sind in der nachfolgenden Tabelle und Abb. 3 gezeigt.

Das erfindungsgemäße N-Phos-Oligomer zeigt im Vergleich zu dem EP 2041161-Oligomer einen 2,6-fach stärkeren Effekt bei der Splice Site Modulation des Targets TNFR2 in THP1 Zellen, während die Modulation des Targets TNFR2 in THP1 Zellen durch das US5719262-Oligomer nahezu Null ist.

| Tabelle zum Vergleich des Effekts auf die Genexpression von N-Phos-Oligomeren mit EP2041161-Oligomeren und US5719262-Oligomeren am Beispiel der Splice Site Modulation des Targets TNFR_2 in THP1 Zellen bei einer Konzentration von 10μM. | | | |
|---|---|---|---|
| | Verhältnis TNFR2 mRNA Isoform ohne Exon 7 zu TNFR2 mRNA gesamt in % | | |
| Sequenz | N-Phos-Oligomer[a] | EP 2041161-Oligomer[b] | US5719262-Oligomer[c] |
| FluPac-Gly-cagtcc$T^R$a$G^R$a$A^R$agaaa-$NH_2$ | 3,4 | 1,3 | 0,3 |
| [a]N-Phos-Oligomer: $R^1$ = $-CH_2-CH_2-CH_2-CH_2-NH-CH_2-CH_2-P{=}O(OEt)_2$ [b]EP2041161-Oligomer: $R^1$ = $-CH_2-CH_2-CH_2-P{=}O(OEt)_2$<br>[c]US5719262-Oligomer: $R^1$ = $-CH_2-CH_2-CH_2-CH_2-NH_2$ | | | |

**Beispiel 22: Wirkung von erfindungsgemäßen N-Phos-Oligomeren mit unterschiedlichen Resten U auf die Splice Site Modulation des Targets TNFR2 (Skipping von Exon 7) in der Lunge von Mäusen**

Die Durchführung des Experiments erfolgte wie in Beispiel 20 beschrieben.

Die getesteten erfindungsgemäßen N-Phos-Oligomeren gemäß der Formel (VI) mit einem Rest U gemäß der allgemeinen Formel VII und einer Gruppe der Formel IXc (Cholesterin-Derivat) bzw. IXd (Folsäure-Derivat) als $R^{46}$ zeigen einen starken Effekt auf die Genexpression bei der Splice Site Modulation des Targets TNFR2 in Mäusen.

Beispielsweise ist der Effekt in der Lunge bei Verwendung des Cholesterin-Derivats um das 560-fache und beim Folsäure-Derivat um das 378-fache gegenüber der PBS-Negativ-Kontrolle erhöht. Die Ergebnisse sind in Abb. 4 gezeigt.

**Beispiel 23: Wirkung von erfindungsgemäßen N-Phos-Oligomeren mit unterschiedlicher Nukleobasen-Sequenz, Resten U, sowie Unterschieden in der Anzahl und Position der Gruppen der allgemeinen Formel (IV) und (V) gemäß der allgemeinen Formel (VI) auf die Splice Site Modulation des Targets TNFR2 (Skipping von Exon 7) in der Niere, Leber und Lunge von Mäusen.**

Das Experiment wurde mit den erfindungsgemäßen N-Phos-Oligomeren N-Phos 23-1, N-Phos 23-2, N-Phos-23-4 (vgl. Abb. 5) durchgeführt. Die Durchführung des Experiments erfolgte wie in Beispiel 20 beschrieben.

Alle getesteten erfindungsgemäßen N-Phos-Oligomere zeigen in verschiedenen Maus-Geweben (Niere, Leber und Lunge) sehr starke Effekte auf die Genexpression der mRNA Isoform ohne Exon 7. In der Niere ist beispielsweise der Effekt von N-Phos 23-1 um das 1983-fache gegenüber der PBS-Negativ-Kontrolle erhöht. Die Ergebnisse sind in Abb. 5 gezeigt.

**Beispiel 24: Wirksamkeitsvergleich zwischen erfindungsgemäßen N-Phos-Oligomeren und EP 2041161-Oligomeren bei der Splice Site Modulation des Targets TNFR2 (Skipping von Exon 7) in der Niere von Mäusen.**

Es wurden 2 Varianten von N-Phos-Oligomeren (Variante 1: Summe aller Wiederholungs-einheiten Yd, Zf, Yg, und Zj gemäß der allgemeinen Formel (VI) = 15 bzw. 14; Variante 2: Anzahl und Position der Gruppen der allgemeinen Formel (IV und (V) gemäß der allgemeinen Formel (VI)) und die entsprechenden Varianten von EP2041161-Oligomeren getestet. Die getesteten Varianten sind in Abb. 6 gezeigt. Die Durchführung des Experiments erfolgte wie in Beispiel 20 beschrieben, mit dem Unterschied, dass in diesem Experiment die Tiere bereits 2 Stunden nach der LPS-Stimulation getötet wurden.

Die Wirkung der N-Phos-Oligomere auf die Genexpression der mRNA Isoform ohne Exon 7 ist im direkten Vergleich mit den EP2041161-Oligomeren 12,6 bzw. 6,7-fach stärker. Die Ergebnisse sind Abb. 6 gezeigt.

**Beispiel 25: *In vivo* Wirkung eines erfindungsgemäßen N-Phos-Oligomeren mit 20 Bausteinen auf die Splice Site Modulation des Targets Dystrophin (Skipping von Exon 23) im Muskel von mdx-Mäusen.**

Das Experiment wurde mit einem erfindungsgemäßen N-Phos-Oligomer mit 20 Bausteinen (Summe aller Wiederholungseinheiten Yd, Zf, Yg, und Zj gemäß der allgemeinen Formel (VI) = 19) durchgeführt. Die getestete Verbindung ist in Abb. 7 gezeigt. Die Durchführung dieses Experiments erfolgte wie in Beispiel 20 beschrieben, mit den Unterschieden, dass in diesem Experiment Mäuse des Stammes C57BL/10ScSn-Dmdmdx/J (Jackson Labs) verwendet wurden, die Tiere nicht mit LPS stimuliert wurden, und am Tag 15 getötet wurden. Das Expressionsniveau der Ziel-mRNA, der mRNA Isoform des Dystrophin-Gens der Maus ohne das Exon 23, wurde als Median der 5 Mediane der Triplikat-Messung des Muskels, normalisiert auf Rpl13a bestimmt.

Das erfindungsgemäße N-Phos-Oligomer mit 20 Bausteinen zeigt im Muskel eine 9-fache stärkere Wirkung *in vivo* auf die Genexpression der mRNA Isoform ohne Exon 23 im Vergleich zur PBS-Kontroll-Gruppe. Das Ergebnis ist in Abb. 7 gezeigt.

## Patentansprüche

1. Verbindung der allgemeinen Formel (I):

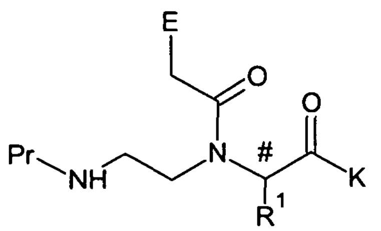

(I)

wobei

K eine Carbonsäure-Aktivester-Gruppe oder -O-$R_M$ darstellt; wobei $R_M$ ein H-Atom, eine Methyl-, Ethyl-, Allyl-, Benzyl-, Phenyl-, tert.-Butyl-, oder eine Trimethylsilyl-Gruppe darstellt;
Pr ein H-Atom oder eine Amino-Schutzgruppe darstellt;
# ein asymmetrisches C-Atom kennzeichnet;
E eine gegebenenfalls mit einer Nukleobasen-Schutzgruppe substituierte Adeninyl-, Cytosinyl-, Pseudoisocytosinyl-, Guaninyl-, Thyminyl-, Uracilyl- oder Phenyl-Gruppe darstellt;
$R^1$ eine Gruppe ist, die durch die allgemeine Formel (II) dargestellt ist:

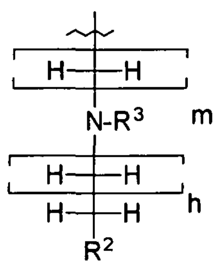

(II)

wobei

$R^2$ eine Phosphonsäureester- oder eine Phosphonsäure-Gruppe ist;
$R^3$ ein H-Atom, oder eine Amino-Schutzgruppe ist;
m 1, 2, 3 oder 4 ist; und
h 0, 1, 2, oder 3 ist;
mit der Maßgabe, dass für die Summe von m und h in der allgemeinen Formel (II) gilt: $2 \leq x \leq 5$.

2. Verbindung nach Anspruch 1, wobei E eine Thyminyl-, Uracilyl-, Phenyl-, N2-Acetyl-guaninyl-, N2-Isobutyryl-guaninyl-, N2-Benzyloxycar-bonyl-guaninyl-, N2-(4-Methoxyphenyl)-diphenylmethyl-guaninyl-, N2-Benzhydryloxycarbonyl-guaninyl-, N2-Di-benzhydryloxycarbonyl-guaninyl-, N2-tert-Butyloxycarbonyl-guaninyl, N2-Di-tert-butyloxycarbonyl-guaninyl-, N6-Benzyloxycarbonyl-adeninyl-, N6-(4-Methoxyphenyl)-diphenylmethyl-adeninyl-, N6-Anisoyl-adeninyl-, N6-Benzhydryloxycarbonyl-adeninyl-, N6-Di-benzhydryloxycarbonyl-adeninyl-, N6-tert-Butyloxycarbonyl-adeninyl-, N6-Di-tert-butyloxycarbonyl-adeninyl-, O6-Benzylguaninyl-, N2-Acetyl-O6-diphenylcar-bamoyl-guaninyl-, N2-Isobutyryl-O6-diphenylcarbamoyl-guaninyl-, N2-Benzyloxycarbonyl-O6-diphenylcarbamoyl-guaninyl-, N2-(4-Methoxyphenyl)-diphenylmethyl-O6-diphenylcarbamoyl-guaninyl-, N2-Benzhydryloxycarbonyl-O6-diphenyl-carbamoyl-guaninyl-, N4-Benzyloxycarbonyl-cytosinyl-, N4-(4-Methoxyphenyl)-diphenylmethyl-cytosinyl-, N4-4-tert.Butylbenzoyl-cytosinyl-, N4-Benzhydryloxycarbonyl-cytosinyl-, N4-Di-benzhydryloxycarbonylcytosinyl-, N4-tert-Butyloxycarbonyl-cytosinyl-, N4-Di-tert-butyloxycarbonyl-cytosinyl-, N2-Benzyloxycarbonyl-pseudoisocytosinyl-, N2-(4-Methoxyphenyl)-diphenylmethyl-pseudoisocytosinyl-, N2-4-tert.-Butylbenzoyl-pseudoisocytosinyl-, N2-Benzhydryloxycarbonyl-pseudoisocytosinyl-, N2-Dibenzhydryloxycarbonyl-pseudoisocytosinyl-, N2-tert-Butyloxycarbonyl-pseudoisocytosinyl- oder eine N2-Di-tert-butyloxycarbonyl-pseudoisocytosinyl-Gruppe darstellt.

3. Verbindung nach Anspruch 2, wobei E eine Thyminyl-, Uracilyl-, Phenyl-, N2-Benzyloxycar-bonyl-guaninyl-, N2-Benzhydryloxycarbonyl-guaninyl-, N2-tert-Butyloxycarbonylguaninyl-, N2-Benzyloxycarbonyl-O6-diphenylcarbamoyl-guaninyl-, N2-Benzhydryloxycarbonyl-O6-diphenylcarbamoyl-guaninyl-, N6-Benzyloxycarbonyl-adeninyl-, N6-Benzhydryloxycarbonyl-adeninyl-, N6-tert-Butyloxycarbonyl-adeninyl-, N6-Di-tert-Butyloxycarbonyl-adeninyl-, N4-Benzyloxycarbonyl-cytosinyl-, N4-Benzhydryloxycarbonyl-Cytosinyl-, N4-Di-tert-butyloxycarbonyl-cytosinyl-, N2-Benzyloxycarbonyl-pseudo-isocytosinyl-, N2-Benzhydryloxycarbonyl-pseudoisocytosinyl- oder eine N2-tert-Butyloxycarbonyl-pseudoisocytosinyl-Gruppe darstellt.

4. Verbindung nach einem der Ansprüche 1 bis 3, wobei $R^2$ eine Phosphonsäureester-Gruppe der Formel $-P(=O)(OV)_2$ oder $-P(=O)(OV)(OH)$ darstellt; und jedes V unabhängig eine Methyl-, Ethyl-, Cyclohexyl-, oder Benzyl-Gruppe darstellt.

5. Verbindung nach einem der Ansprüche 1 bis 4, wobei $R^3$ ein H-Atom ist.

6. Verbindung nach einem der Ansprüche 1 bis 4, wobei $R^3$ eine Oxocarbamat-, Thiocarbamat-, oder eine Mmt-Schutzgruppe darstellt.

7. Verbindung, dargestellt durch die allgemeine Formel (VI):

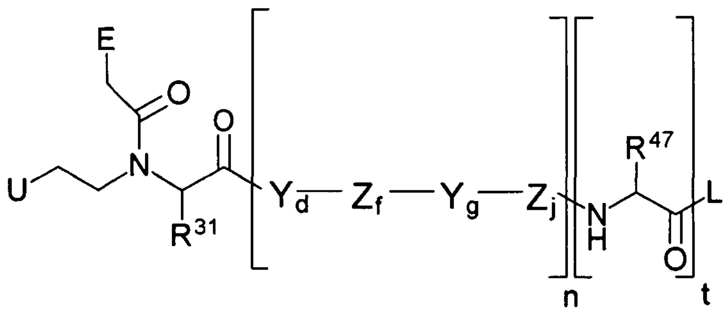

(VI)

wobei
jedes Y jeweils unabhängig eine Gruppe der allgemeinen Formel (IV) darstellt:

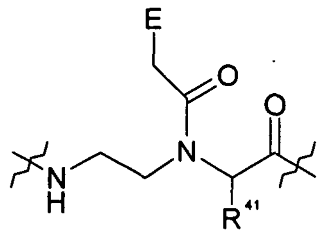

(IV);

jedes Z jeweils unabhängig eine Gruppe der allgemeinen Formel (V) darstellt:

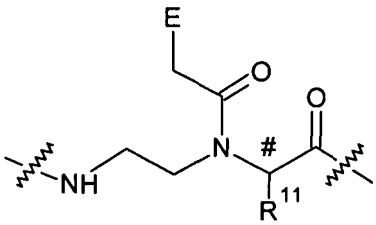

(V);

wobei

jedes E jeweils unabhängig eine Adeninyl-, Cytosinyl-, Pseudoisocytosinyl-, Guaninyl-, Thyminyl-, Uracilyl-, oder Phenyl-Gruppe darstellt;
# ein asymmetrisches C-Atom kennzeichnet;
jedes $R^{41}$ jeweils ein H-Atom darstellt;
jedes $R^{11}$ eine Gruppe $-(CH_2)_m-NH-(CH_2)_h-CH_2-R^{12}$ darstellt;
wobei $R^{12}$ jeweils unabhängig eine Phosphonsäureester-Gruppe der Formel $-P(=O)(OV)_2$ oder $-P(=O)(OV)(OH)$ darstellt; und jedes V jeweils unabhängig eine Methyl-, Ethyl-, Cyclohexyl-, oder Benzyl-Gruppe darstellt;
m jeweils unabhängig 1, 2, 3 oder 4 und h jeweils unabhängig 0, 1, 2, oder 3 ist; mit der Maßgabe, dass für die Summe von m und h gilt: $2 \le x \le 5$;
d jeweils unabhängig 0, 1, 2, 3 oder 4 ist;
f jeweils unabhängig 0, 1, 2, 3 oder 4 ist;
g jeweils unabhängig 0, 1, 2, 3 oder 4 ist;
j jeweils unabhängig 0, 1, 2, 3 oder 4 ist;
n = 0, 1, 2, 3, 4, 5, 6, 7 oder 8 ist;
mit der Maßgabe, dass für die Summe aller Wiederholungseinheiten Yd, Zf, Yg, und Zj in der allgemeinen Formel (VI) gilt: $7 \le x \le 30$; und mindestens eine der Variablen f oder j eine ganze Zahl von 1 bis 5 darstellt;
mit der Maßgabe, dass für das Verhältnis (Summe der Wiederholungseinheiten Zf und Zj): (Summe aller Wiederholungseinheiten Yd, Zf, Yg, und Zj) in der allgemeinen Formel (VI) gilt: $0{,}1 \le x \le 0{,}5$;
$R^{31}$ ein H-Atom; eine Seitenkette der Aminosäure Alanin, Arginin, Asparagin, Asparaginsäure, Cystein, Glutamin, Glutaminsäure, Histidin, Isoleucin, Leucin, Lysin, Methionin, Ornithin, Phenylalanin, Prolin, Histidin, Serin, Threonin, Tryptophan, Tyrosin, oder Valin; oder eine Gruppe $-(CH_2)_m-NH-(CH_2)_h-CH_2-R^{12}$ darstellt; wobei $R^{12}$ eine Phosphonsäureester- oder eine Phosphonsäure-Gruppe ist; m eine ganze Zahl von 1 bis 5 darstellt; und h eine ganze Zahl von 0 bis 4 darstellt; mit der Maßgabe, dass für die Summe von m und h gilt: $2 \le x \le 5$;
$R^{47}$ jeweils unabhängig ein H-Atom; oder eine Seitenkette der Aminosäure Lysin, Ornithin, oder Arginin ist;
t = 0, 1, 2, 3, 4, 5, 6, 7 oder 8 ist;
L OH, OEt, $NH_2$ oder $-NHNH_2$ darstellt;
U eine Gruppe der allgemeinen Formel (VII) darstellt:

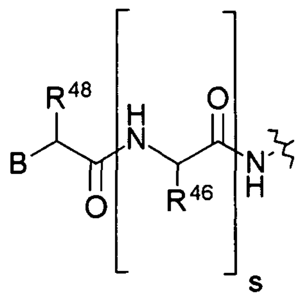

(VII)

wobei B ein H-Atom, eine Phenylgruppe, oder eine substituierte Phenylgruppe, die mit 1 bis 3 Substituenten, ausgewählt aus der Gruppe, bestehend aus OH, F, Cl, Br, I und $NO_2$, substituiert ist, darstellt; $R^{48}$ ein H-Atom ist; und

(i) jedes $R^{46}$ jeweils unabhängig voneinander ein H-Atom, oder eine Seitenkette der Aminosäure Alanin,

Arginin, Asparagin, Asparaginsäure, Cystein, Glutamin, Glutaminsäure, Histidin, Isoleucin, Leucin, Lysin, Methionin, Ornithin, Phenylalanin, Prolin, Histidin, Serin, Threonin, Tryptophan, Tyrosin, oder Valin darstellt; und s 0, 1, 2, 3, 4, 5, 6, 7 oder 8 ist; oder

(ii) jedes $R^{46}$ jeweils unabhängig voneinander ein H-Atom, oder eine Gruppe der Formel (IXb):

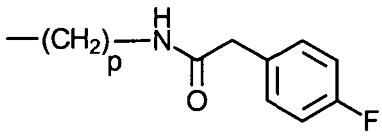

(IXb);

eine Gruppe der Formel (IXc):

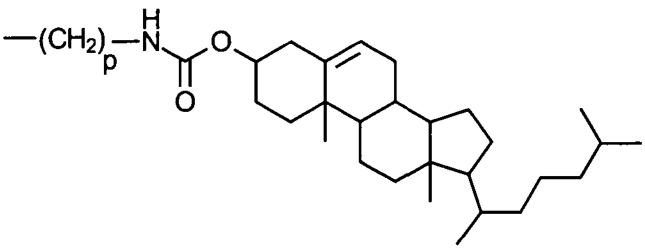

(IXc);

eine Gruppe der Formel (IXd):

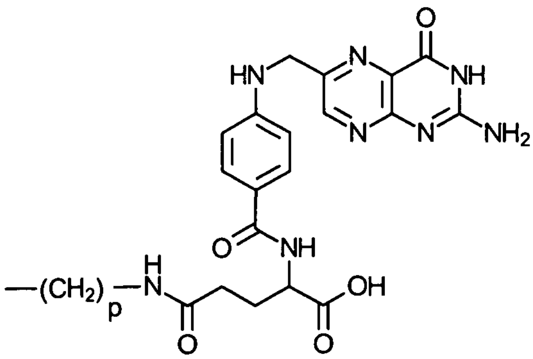

(IXd);

oder eine Gruppe der Formel (IXe):

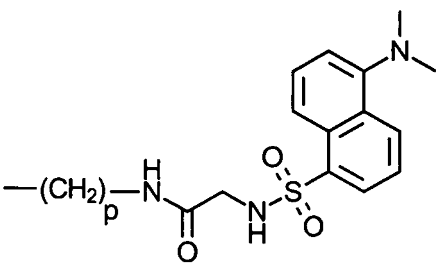

(IXe)

darstellt; p in den Formeln (IXb), (IXc), (IXd), und (IXe) die Zahl 3 oder 4 darstellt; und s = 1, 2, 3 oder 4 ist.

8. Verbindung nach Anspruch 7, wobei jedes $R^{11}$ jeweils eine Gruppe der Formel $-CH_2-CH_2-CH_2-CH_2-NH-CH_2-CH_2-P{=}O(OEt)_2$, oder eine Gruppe der Formel $-CH_2-CH_2-CH_2-NH-CH_2-CH_2-P{=}O(OEt)_2$ darstellt.

9. Verbindung nach Anspruch 7 oder 8, wobei jedes $R^{31}$ ein H-Atom, eine Seitenkette der Aminosäure Lysin, Ornithin, Arginin, Histidin, Tryptophan, Tyrosin, Threonin oder Serin, eine Gruppe der Formel $-CH_2-CH_2-CH_2-CH_2-NH-CH_2-CH_2-P{=}O(OEt)_2$, oder eine Gruppe der Formel $-CH_2-CH_2-CH_2-NH-CH_2-CH_2-P{=}O(OEt)_2$ darstellt.

10. Verbindung nach einem der Ansprüche 7 bis 9 zur Verwendung als Medikament.

11. Pharmazeutische Zusammensetzung, enthaltend mindestens eine Verbindung gemäß einem der Ansprüche 7 bis 9, und fakultativ mindestens einen Trägerstoff und/oder mindestens ein Adjuvanz.

## Claims

1. A compound of general formula (I): wherein

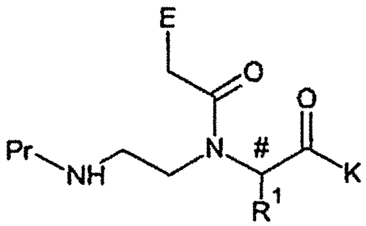

(I)

K represents a carboxylic acid active ester group or $-O-R_M$; wherein $R_M$ represents an H atom, a methyl, ethyl, allyl, benzyl, phenyl, tert.-butyl, or a trimethylsilyl group;
Pr represents an H atom or an amino protective group;
# denotes an asymmetric C atom;
E represents an adeninyl, cytosinyl, pseudoisocytosinyl, guaninyl, thyminyl, uracilyl or phenyl group substituted as necessary with a nucleobase protective group;
$R^1$ is a group, represented by the general formula (II):

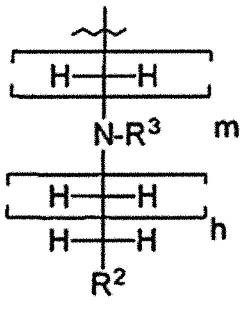

(II)

wherein

$R^2$ is a phosphonic acid ester group or a phosphonic acid group;
$R^3$ is an H atom, or an amino protective group;
m is 1, 2, 3 or 4; and
h is 0, 1, 2 or 3;
provided that the sum of m and h in the general formula (II) is: $2 \leq x \leq 5$.

2. The compound according to claim 1, wherein E represents a thyminyl, uracilyl, phenyl, N2-acetyl-guaninyl, N2-isobutyryl-guaninyl, N2-benzyloxycarbonyl-guaninyl, N2-(4-methoxyphenyl)-diphenylmethyl-guaninyl, N2-benzhydryloxycarbonyl-guaninyl, N2-di-benzhydryloxycarbonyl-guaninyl, N2-tert-butyloxycarbonyl-guaninyl, N2-di-tert-butyloxycarbonyl-guaninyl, N6-benzyloxycarbonyl-adeninyl, N6-(4-methoxyphenyl)-diphenylmethyl-adeninyl, N6-anisoyl-adeninyl, N6-benzhydryloxycarbonyl-adeninyl, N6-di-benzhydryloxycarbonyl-adeninyl, N6-tert-butyloxycarbonyl-adeninyl, N6-di-tert-butyloxycarbonyl-adeninyl, 06-benzylguaninyl, N2-acetyl-O6-diphenylcar-bamoyl-guaninyl, N2-isobutyryl-06-diphenyl-carbamoyl-guaninyl, N2-benzyloxycarbonyl-O6-diphenylcarbamoyl-guaninyl, N2-(4-methoxyphenyl)-diphenylmethyl-O6-diphenyl-carbamoyl-guaninyl, N2-benzhydryloxycarbonyl-O6-diphenylcarbamoyl-guaninyl, N4-benzyloxycarbonyl-cytosinyl, N4-(4-methoxyphenyl)-diphenyl-methyl-cytosinyl, N4-4-tert.-butyl-benzoyl-cytosinyl, N4-benz-hydryloxycarbonyl-cytosinyl, N4-di-benzhydryloxycarbonyl-cytosinyl, N4-tert-butyloxycarbonyl-cytosinyl, N4-di-tert-butyloxycarbonyl-cytosinyl, N2-benzyloxycarbonyl-pseudo-isocytosinyl, N2-(4-methoxyphenyl)-diphenylmethyl-pseudoisocytosinyl, N2-4-tert-butylbenzoyl-pseudo-isocytosinyl, N2-benzhydryloxycarbonyl-pseudo-isocytosinyl, N2-di-benzhydryloxycarbonyl-pseudo-isocytosinyl, N2-tert-butyloxycarbonyl-pseudoisocytosinyl or an N2-di-tert-butyloxycarbonyl-pseudoisocytosinyl group.

3. The compound according to claim 2, wherein E represents a thyminyl, uracilyl, phenyl, N2-benzyloxycarbonyl-guaninyl, N2-benzhydryloxycarbonyl-guaninyl, N2-tert-butyloxycarbonyl-guaninyl, N2-benzyloxycarbonyl-O6-diphenylcarbamoyl-guaninyl, N2-benzhydryloxycarbonyl-O6-diphenylcarbamoyl-guaninyl, N6-benzyloxycarbonyl-adeninyl, N6-benzhydryloxycarbonyl-adeninyl, N6-tert-butyloxycarbonyl-adeninyl, N6-di-tert-butyloxycarbonyl-adeninyl, N4-benzyloxycarbonyl-cytosinyl, N4-benz-hydryloxycarbonyl-cytosinyl, N4-di-tert-butyloxycarbonyl-cytosinyl, N2-benzyloxycarbonyl-pseudo-isocytosinyl, N2-benz-hydryloxycarbonyl-pseudo-isocytosinyl or an N2-tert-butyloxycarbonyl-pseudo-isocytosinyl group.

4. The compound according to any one of claims 1 to 3, wherein $R^2$ represents a phosphonic acid ester group of the formula $-P(=O)(OV)_2$ or $-P(=O)(OV)(OH)$; and each V independently represents a methyl, ethyl, cyclohexyl, or benzyl group.

5. The compound according to any one of claims 1 to 4, wherein $R^3$ is an H atom.

6. The Compound according to any one of claims 1 to 4, wherein $R^3$ represents an oxocarbamate, thiocarbamate, or an Mmt protective group.

7. A compound represented by the general formula (VI):

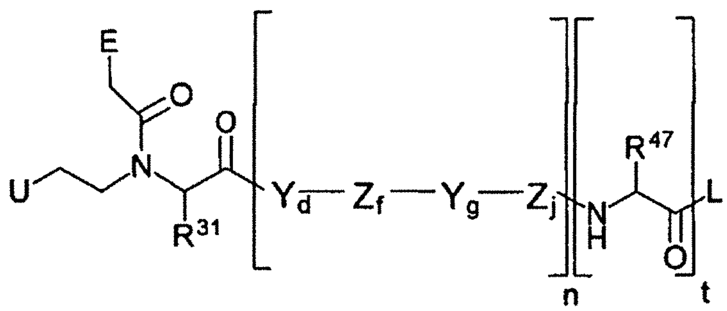

(VI)

wherein
each Y in each case independently represents a group of general formula (IV):

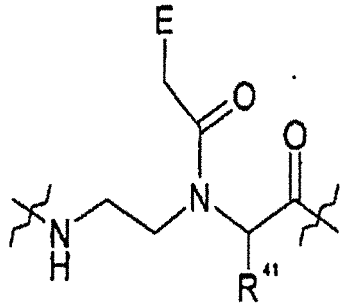

(IV);

each Z in each case independently represents a group of general formula (V):

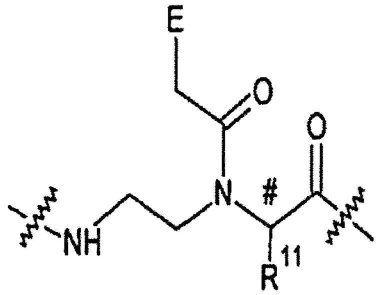

(V)

wherein

each E in each case independently represents an adeninyl, cytosinyl, pseudoisocytosinyl, guaninyl, thyminyl, uracilyl, or phenyl group;
# denotes an asymmetric C atom;
each $R^{41}$ in each case represents an H atom;
each $R^{11}$ represents a group $-(CH_2)_m-NH-(CH_2)_h-CH_2-R^{12}$; wherein $R^{12}$ in each case independently represents a phosphonic acid ester group of the formula $-P(=O)(OV)_2$ or $-P(=O)(OV)(OH)$; and each V in each case independently represents a methyl, ethyl, cyclohexyl, or benzyl group;
m in each case independently is 1, 2, 3 or 4 and h in each case independently is 0, 1, 2, or 3; provided that the sum of m and h is: $2 \leq x \leq 5$;
d in each case independently is 0, 1, 2, 3 or 4;
f in each case independently is 0, 1, 2, 3 or 4;
g in each case independently is 0, 1, 2, 3 or 4;
j in each case independently is 0, 1, 2, 3 or 4;
n = 0, 1, 2, 3, 4, 5, 6, 7 or 8;
provided that the sum of all repeat units Yd, Zf, Yg, and Zj in the general formula (VI) is: $7 \leq x \leq 30$; and at least one of the variables f or j represents an integer of from 1 to 5;
provided that the ratio (sum of repeat units Zf and Zj) : (sum of all repeat units Yd, Zf, Yg, and Zj) in the general formula (VI) is: $0.1 \leq x \leq 0.5$;
$R^{31}$ represents an H atom; a side chain of the amino acid alanine, arginine, asparagine, aspartic acid, cysteine, glutamine, glutamic acid, histidine, isoleucine, leucine, lysine, methionine, ornithine, phenylalanine, proline, histidine, serine, threonine, tryptophan, tyrosine, or valine; or a group $-(CH_2)_m-NH-(CH_2)_h-CH_2-R^{12}$; wherein $R^{12}$ is a phosphonic acid ester group or a phosphonic acid group; m represents an integer of from 1 to 5; and h represents an integer of from 0 to 4; provided that the sum of m and h is: $2 \leq x \leq 5$;
$R^{47}$ in each case independently is an H atom, or a side chain of the amino acid lysine, ornithine, or arginine;
t = 0, 1, 2, 3, 4, 5, 6, 7 or 8;
L represents OH, OEt, $NH_2$ or $-NHNH_2$;

U represents a group of general formula (VII):

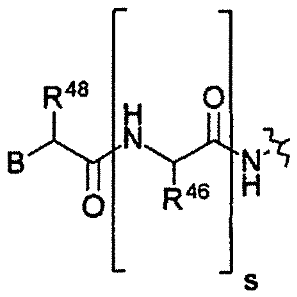

(VII)

wherein B represents an H atom, a phenyl group, or a substituted phenyl group, substituted with 1 to 3 substituents, selected from the group, consisting of OH, F, Cl, Br, I and $NO_2$;
$R^{48}$ is an H atom; and

(i) each $R^{46}$ in each case independently of each other represents an H atom, or a side chain of the amino acid alanine, arginine, asparagine, aspartic acid, cysteine, glutamine, glutamic acid, histidine, isoleucine, leucine, lysine, methionine, ornithine, phenylalanine, proline, histidine, serine, threonine, tryptophan, tyrosine, or valine; and s is 0, 1, 2, 3, 4, 5, 6, 7 or 8; or
(ii) each $R^{46}$ in each case independently of each other represents an H atom, or a group of the formula (IXb):

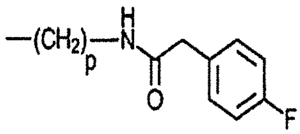

(IXb);

a group of the formula (IXc):

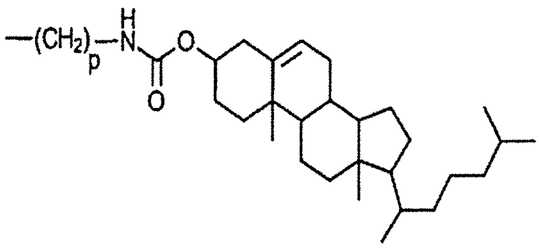

(IXc);

a group of the formula (IXd):

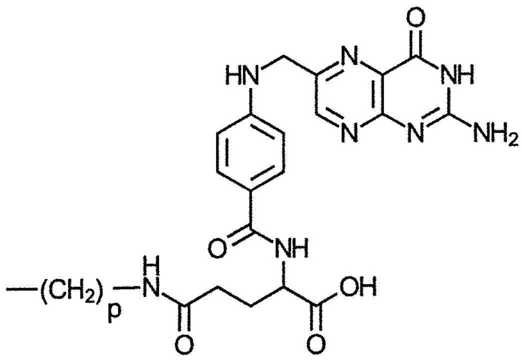

(IXd);

or a group of the formula (IXe):

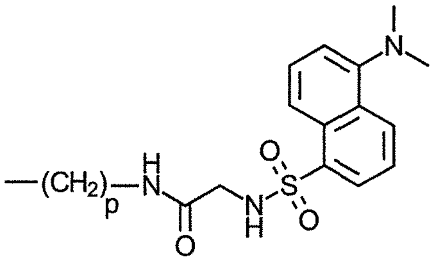

(IXe);

p in formulae (IXb), (IXc), (IXd), and (IXe) represents the number 3 or 4; and s = 1, 2, 3 or 4.

**8.** The compound according to claim 7, wherein each $R^{11}$ in each case represents a group of the formula:

$-CH_2-CH_2-CH_2-CH_2-NH-CH_2-CH_2-P=O(OEt)_2$,

or
a group of the formula: $-CH_2-CH_2-CH_2-NH-CH_2-CH_2-P=O(OEt)_2$.

**9.** The compound according to claim 7 or 8, wherein each $R^{31}$ represents an H atom, a side chain of the amino acid lysine, ornithine, arginine, histidine, tryptophan, tyrosine, threonine or serine, a group of the formula:

$-CH_2-CH_2-CH_2-CH_2-NH-CH_2-CH_2-P=O(OEt)_2$,

or
a group of the formula: $-CH_2-CH_2-CH_2-NH-CH_2-CH_2-P=O(OEt)_2$.

**10.** The compound according to any one of claims 7 to 9 for use as a medicament.

**11.** Pharmaceutical composition containing at least one compound according to any one of claims 7 to 9, and optionally at least one carrier and/or at least one adjuvant.

## Revendications

**1.** Composé de formule générale (I) :

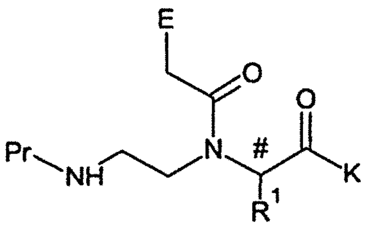

(I)

dans laquelle

K représente un groupe ester actif d'acide carboxylique ou -O-$R_M$ ; où $R_M$ représente un atome de H, un groupe méthyle, éthyle, allyle, benzyle, phényle, tert.-butyle, ou triméthylsilyle ;
Pr représente un atome de H ou un groupe protecteur amino ;
# désigne un atome de C asymétrique ;
E représente un groupe adéninyle, cytosinyle, pseudoisocytosinyle, guaninyle, thyminyle, uracilyle ou phényle éventuellement substitué par un groupe protecteur de nucléobases ;
$R^1$ est un groupe représenté par la formule générale (II) :

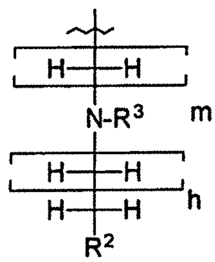

(II)

dans laquelle

$R^2$ est un groupe ester d'acide phosphonique ou acide phosphonique ;
$R^3$ est un atome de H ou un groupe protecteur amino ;
m est 1, 2, 3 ou 4 ; et
h est 0, 1, 2 ou 3 ;
à condition que, pour la somme de m et h, on ait dans la formule générale (II) : $2 \leq x \leq 5$.

2. Composé selon la revendication 1, dans lequel E représente un groupe thyminyle, uracilyle, phényle, N2-acétyl-guaninyle, N2-isobutyryl-guaninyle, N2-benzyloxycarbonyl-guaninyle, N2-(4-méthoxyphényl)-diphénylméthyl-guaninyle, N2-benzhydryloxycarbonyl-guaninyle, N2-dibenzhydryloxycarbonyl-guaninyle, N2-tert-butyloxycarbonyl-guaninyle, N2-di-tert-butyloxycarbonyl-guaninyle, N6-benzyloxycarbonyl-adéninyle, N6-(4-méthoxyphényl)-diphénylméthyl-adéninyle, N6-anisoyl-adéninyle, N6-benzhydryloxycarbonyl-adéninyle, N6-di-benzhydryloxycarbonyl-adéninyle, N6-tert-butyloxycarbonyl-adéninyle, N6-di-tert-butyloxycarbonyl-adéninyle, O6-benzylguaninyle, N2-acétyl-O6-diphénylcarbamoyl-guaninyle, N2-isobutyryl-O6-diphénylcarbamoyl-guaninyle, N2-benzyloxycarbonyl-O6-diphénylcarbamoyl-guaninyle, N2-(4-méthoxyphényl)-diphénylméthyl-O6-diphénylcarbamoyl-guaninyle, N2-benzhydryloxycarbonyl-O6-diphénylcarbamoyl-guaninyle, N4-benzyloxycarbonyl-cytosinyle, N4-(4-méthoxyphényl)-diphénylméthyl-cytosinyle, N4-4-tert.-butylbenzoyl-cytosinyle, N4-benzhydryloxycarbonyl-cytosinyle, N4-di-benzhydryloxycarbonyl-cytosinyle, N4-tert-butyloxycarbonyl-cytosinyle, N4-di-tert-butyloxycarbonyl-cytosinyle, N2-benzyloxycarbonyl-pseudo-isocytosinyle, N2-(4-méthoxyphényl)-diphénylméthyl-pseudoisocytosinyle, N2-4-tert.-butylbenzoyl-pseudoisocytosinyle, N2-benzhydryloxycarbonyl-pseudo-isocytosinyle, N2-di-benzhydryloxycarbonyl-pseudo-isocytosinyle, N2-tert-butyloxycarbonyl-pseudo-isocytosinyle, ou N2-di-tert-butyloxycarbonyl-pseudo-isocytosinyle.

3. Composé selon la revendication 2, dans lequel E représente un groupe thyminyle, uracilyle, phényle, N2-benzyloxycarbonyl-guaninyle, N2-benzhydryloxycarbonyl-guaninyle, N2-tert-butyloxycarbonyl-guaninyle, N2-benzyloxycarbonyl-O6-diphénylcarbamoyl-guaninyle, N6-benzyloxycarbonyl-adéninyle, N6-benzhydryloxycarbonyl-adéninyle, N6-tert-butyloxycarbonyl-adéninyle, N6-di-tert-butyloxycarbonyl-adéninyle, N4-benzyloxycarbonyl-cytosinyle,

N4-benzhydryloxycarbonyl-cytosinyle, N4-di-tert-butyloxycarbonyl-cytosinyle, N2-benzyloxycarbonyl-pseudoisocytosinyle, N2-benzhydryloxycarbonyl-pseudo-isocytosinyle, ou N2-tert-butyloxycarbonyl-pseudo-isocytosinyle.

**4.** Composé selon l'une des revendications 1 à 3, dans lequel $R^2$ représente un groupe ester d'acide phosphonique de formule $-P(=O)(OV)_2$ ou $-P(=O)(OV)(OH)$ ; et chaque V représente indépendamment un groupe méthyle, éthyle, cyclohexyle ou benzyle.

**5.** Composé selon l'une des revendications 1 à 4, dans lequel $R^3$ est un atome H.

**6.** Composé selon l'une des revendications 1 à 4, dans lequel $R^3$ représente un groupe oxocarbamate, thiocarbamate ou un groupe protecteur Mmt.

**7.** Composé représenté par la formule générale (VI) :

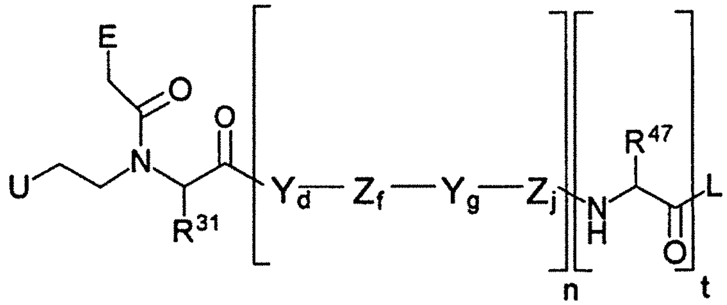

(VI)

dans laquelle
chaque Y représente respectivement indépendamment un groupe de formule générale (IV) :

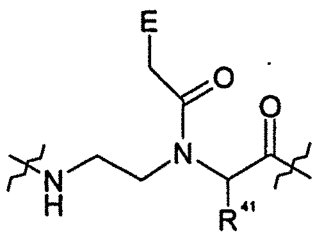

(IV) ;

chaque Z représente respectivement indépendamment un groupe de formule générale (V) :

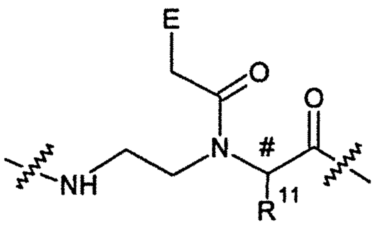

(V) ;

dans laquelle

chaque E représente respectivement indépendamment un groupe adéninyle, cytosinyle, pseudoisocytosinyle, guaninyle, thyminyle, uracilyle, ou phényle ;
# désigne un atome de C asymétrique ;
chaque $R^{41}$ représente respectivement un atome de H ;
chaque $R^{11}$ représente un groupe $-(CH_2)_m-NH-(CH_2)_h-CH_2-R^{12}$ ;

où $R^{12}$ représente respectivement indépendamment un groupe ester d'acide phosponique de formule $-P(=O)(OV)_2$ ou $-P(=O)(OV)(OH)$ ; et chaque V représente respectivement indépendamment un groupe méthyle, éthyle, cyclohexyle ou benzyle ;
m est respectivement indépendamment 1, 2, 3 ou 4 et h est respectivement indépendamment 0, 1, 2 ou 3 ; à condition que, pour la somme de m et h, on ait : $2 \leq x \leq 5$ ;
d est respectivement indépendamment 0, 1, 2, 3 ou 4 ;
f est respectivement indépendamment 0, 1, 2, 3 ou 4 ;
g est respectivement indépendamment 0, 1, 2, 3 ou 4 ;
j est respectivement indépendamment 0, 1, 2, 3 ou 4 ;
n = 0, 1, 2, 3, 4, 5, 6, 7 ou 8 ;
à condition que, pour la somme de tous les motifs récurrents Yd, Zf, Yg, et Zj, on ait dans la formule générale (VI) : $7 \leq x \leq 30$ ; et au moins l'une des variables f ou j représente un nombre entier de 1 à 5 ;
à condition que, pour le rapport (somme des motifs récurrents Zf et Zj) : (somme de tous les motifs récurrents Yd, Zf, Yg, et Zj), on ait dans la formule générale (VI) : $0,1 \leq x \leq 0,5$ ;
$R^{31}$ représente un atome de H ; une chaîne latérale de l'acide aminé alanine, arginine, asparagine, acide aspartique, cystéine, glutamine, acide glutamique, histidine, isoleucine, leucine, lysine, méthionine, ornithine, phénylalanine, proline, histidine, sérine, thréonine, tryptophane, tyrosine ou valine ; ou un groupe $-(CH2)_m-NH-(CH_2)_h-CH_2-R^{12}$ ; où $R^{12}$ représente un groupe ester d'acide phosponique ou acide phosphonique ;
m représente un nombre entier de 1 à 5 ; et h représente un nombre entier de 0 à 4 ; à condition que, pour la somme de m et h, on ait : $2 \leq x \leq 5$ ;
$R^{47}$ représente respectivement indépendamment un atome de H ; ou une chaîne latérale de l'acide aminé lysine, ornithine ou arginine ;
t = 0, 1, 2, 3, 4, 5, 6, 7 ou 8 ;
L représente OH, OEt, $NH_2$ ou $-NHNH_2$ ;
U représente un groupe de formule générale (VII) :

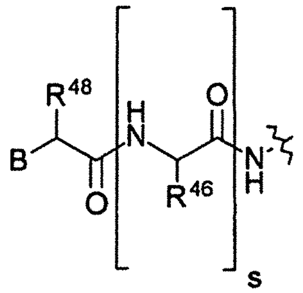

(VII)

dans laquelle B représente un atome de H, un groupe phényle, ou un groupe phényle substitué, qui est substitué par 1 à 3 substituants, choisis dans le groupe constitué de OH, F, Cl, Br, I et $NO_2$ ; $R^{48}$ est un atome de H ; et

(i) chaque $R^{46}$ représente respectivement indépendamment l'un de l'autre un atome de H ou une chaîne latérale de l'acide aminé alanine, arginine, asparagine, acide aspartique, cystéine, glutamine, acide glutamique, histidine, isoleucine, leucine, lysine, méthionine, ornithine, phénylalanine, proline, histidine, sérine, thréonine, tryptophane, tyrosine ou valine ; et s est 0, 1, 2, 3, 4, 5, 6, 7 ou 8 ; ou
(ii) chaque $R^{46}$ représente respectivement indépendamment l'un de l'autre un atome de H, ou un groupe de formule (IXb) :

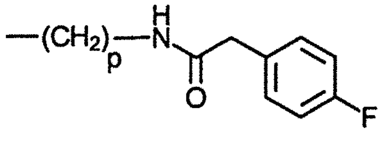

(IXb) ;

un groupe de formule (IXc) :

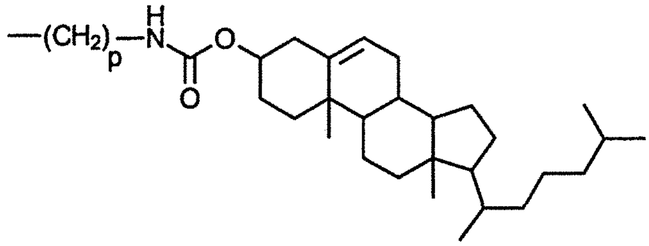

(IXc) ;

un groupe de formule (IXd) :

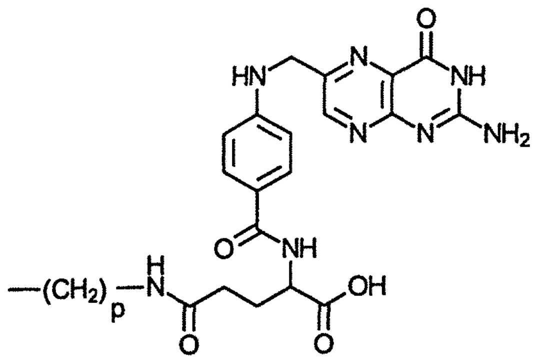

(IXd) ;

ou un groupe de formule (IXe) :

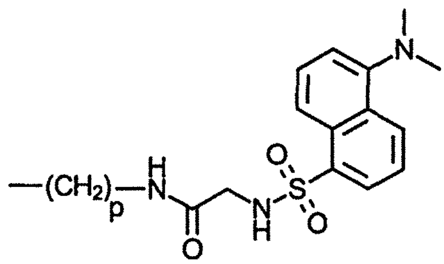

(IXe)

p représente, dans les formules (IXb), (IXc), (IXd) et (IXe) le nombre 3 ou 4 ; et s = 1, 2, 3 ou 4.

**8.** Composé selon la revendication 7, dans lequel chaque $R^{11}$ représente respectivement un groupe de formule $-CH_2-CH_2-CH_2-CH_2-NH-CH_2-CH_2-P{=}O(OEt)_2$, ou un groupe de formule $-CH_2-CH_2-CH_2-NH-CH_2-CH_2-P{=}O(OEt)_2$.

**9.** Composé selon la revendication 7 ou 8, dans lequel chaque $R^{31}$ représente un atome de H, une chaîne latérale de l'acide aminé lysine, ornithine, arginine, histidine, tryptophane, tyrosine, thréonine, ou sérine, un groupe de formule $-CH_2-CH_2-CH_2-CH_2-NH-CH_2-CH_2-P{=}O(OEt)_2$, ou un groupe de formule $-CH_2-CH_2-CH_2-NH-CH_2-CH_2-P{=}O(OEt)_2$.

**10.** Composé selon l'une des revendications 7 à 9 destiné à être utilisé comme médicament.

**11.** Composition pharmaceutique, contenant au moins un composé selon l'une des revendications 7 à 9, et facultativement au moins un excipient et/ou au moins un adjuvant.

Abb. 1

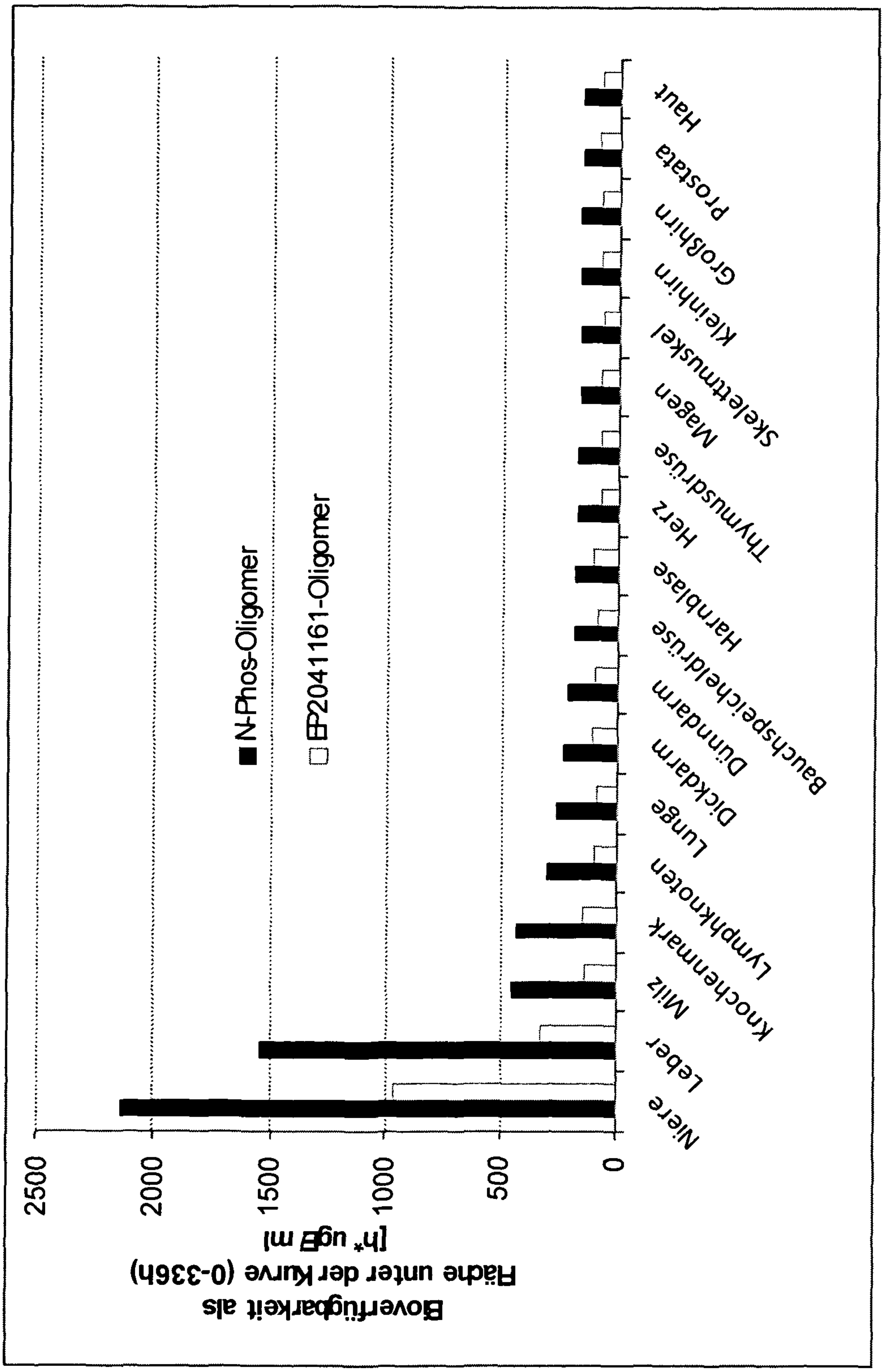
Bioverfügbarkeit als
Fläche unter der Kurve (0-336h)
[h* ug/ ml]
2500
2000
1500
1000
500
0
N-Phos-Oligomer
EP2041161-Oligomer
Niere
Leber
Milz
Knochenmark
Lymphknoten
Lunge
Dickdarm
Dünndarm
Bauchspeicheldrüse
Harnblase
Herz
Thymusdrüse
Magen
Skelettmuskel
Kleinhirn
Großhirn
Prostata
Haut

Abb. 2

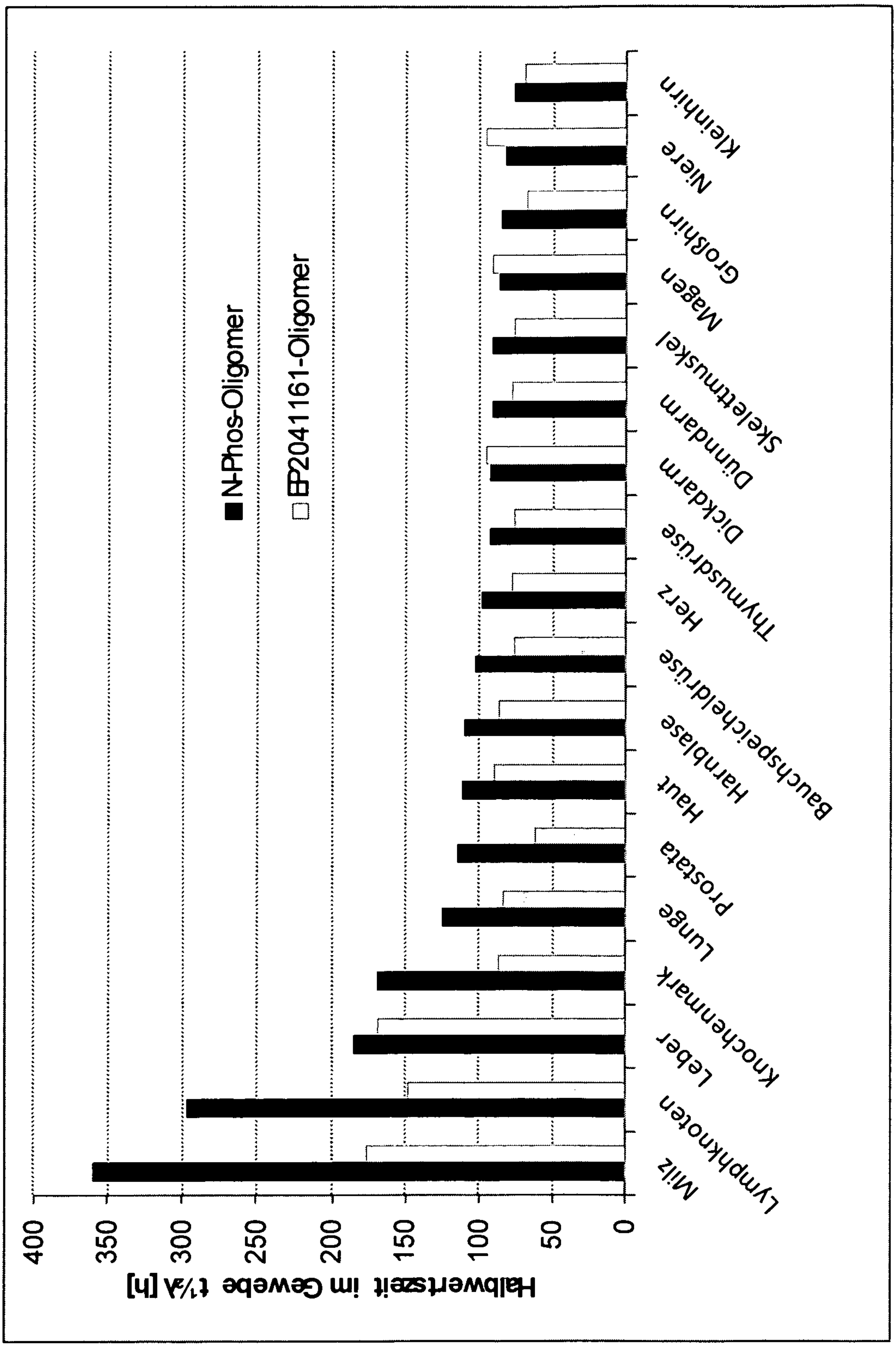
N-Phos-Oligomer
EP2041161-Oligomer
Halbwertszeit im Gewebe t½ [h]
0
50
100
150
200
250
300
350
400
Kleinhirn
Niere
Großhirn
Magen
Skelettmuskel
Dünndarm
Dickdarm
Thymusdrüse
Herz
Bauchspeicheldrüse
Harnblase
Haut
Prostata
Lunge
Knochenmark
Leber
Lymphknoten
Milz

Abb. 3

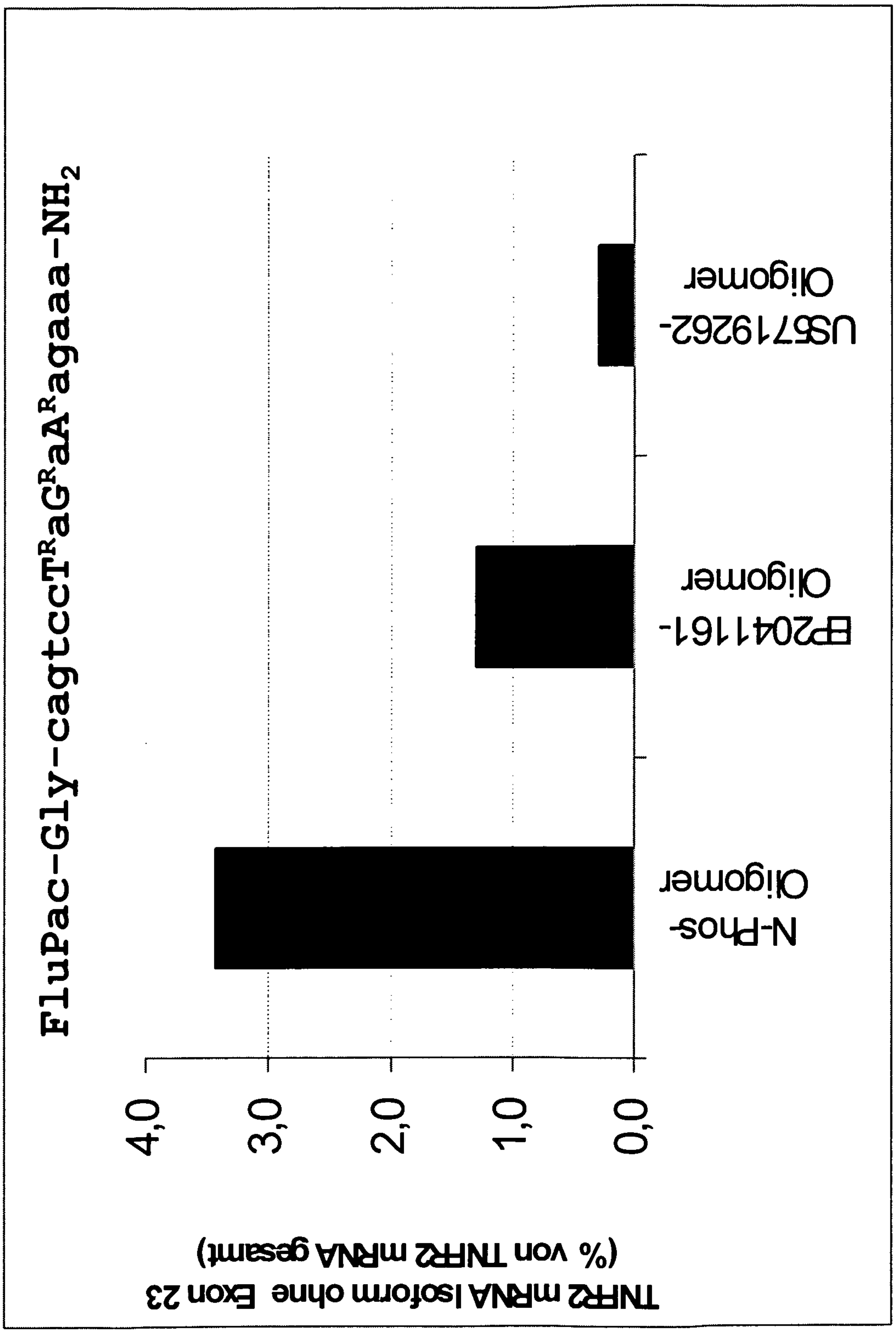
FluPac-Gly-cagtccT^RaG^RaA^Ragaaa-NH2
TNFR2 mRNA Isoform ohne Exon 23
(% von TNFR2 mRNA gesamt)
4,0
3,0
2,0
1,0
0,0
N-Phos-
Oligomer
EP2041161-
Oligomer
US5719262-
Oligomer

Abb. 4

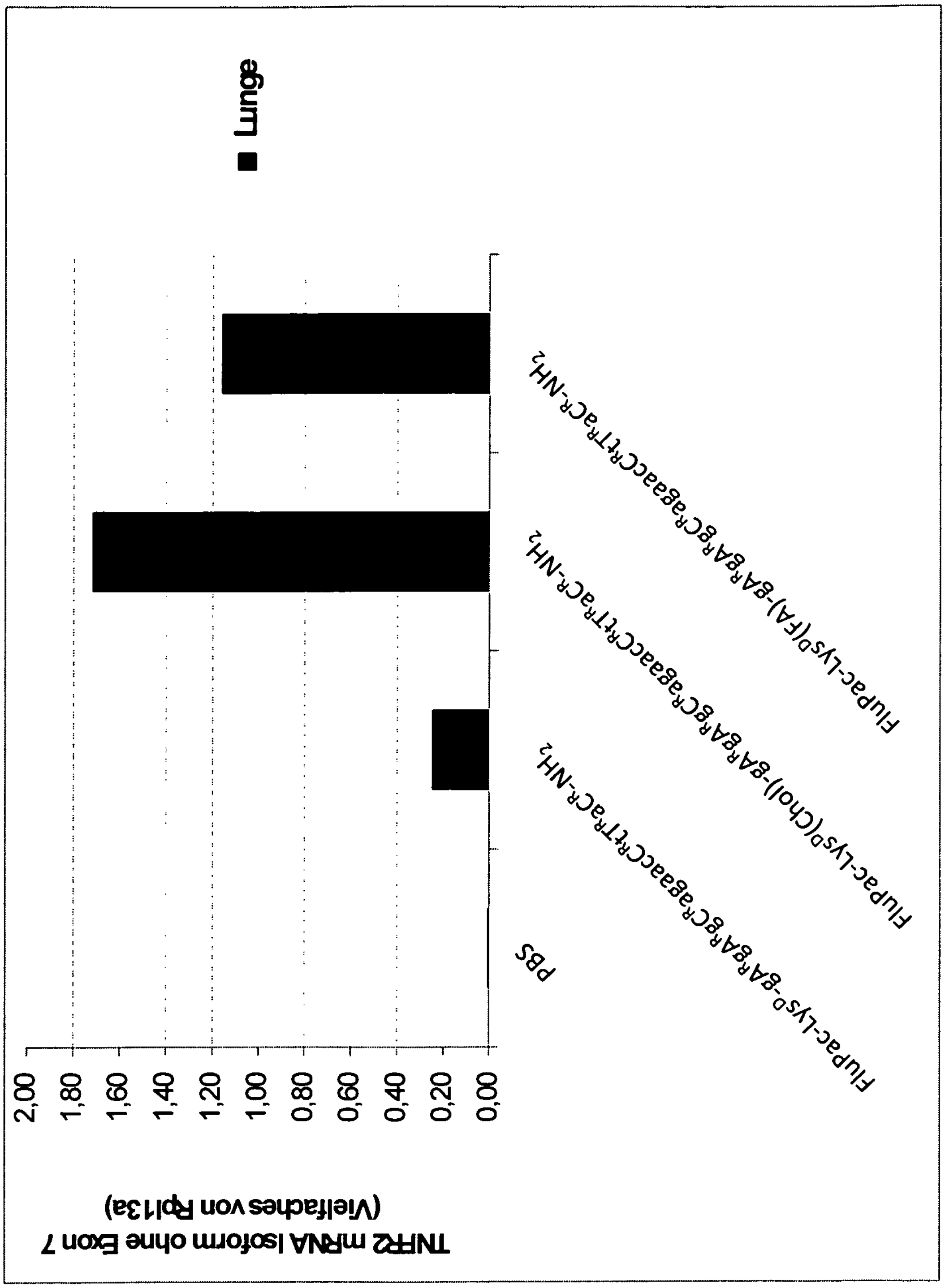
TNFR2 mRNA Isoform ohne Exon 7
(Vielfaches von Rpl13a)
2,00
1,80
1,60
1,40
1,20
1,00
0,80
0,60
0,40
0,20
0,00
Lunge
PBS
FluPac-Lys$^D$-gA$^R$gA$^R$gC$^R$agaaC$^R$tT$^R$aC$^R$-NH$_2$
FluPac-Lys$^D$(Chol)-gA$^R$gA$^R$gC$^R$agaaC$^R$tT$^R$aC$^R$-NH$_2$
FluPac-Lys$^D$(FA)-gA$^R$gA$^R$gC$^R$agaaC$^R$tT$^R$aC$^R$-NH$_2$

Abb. 5

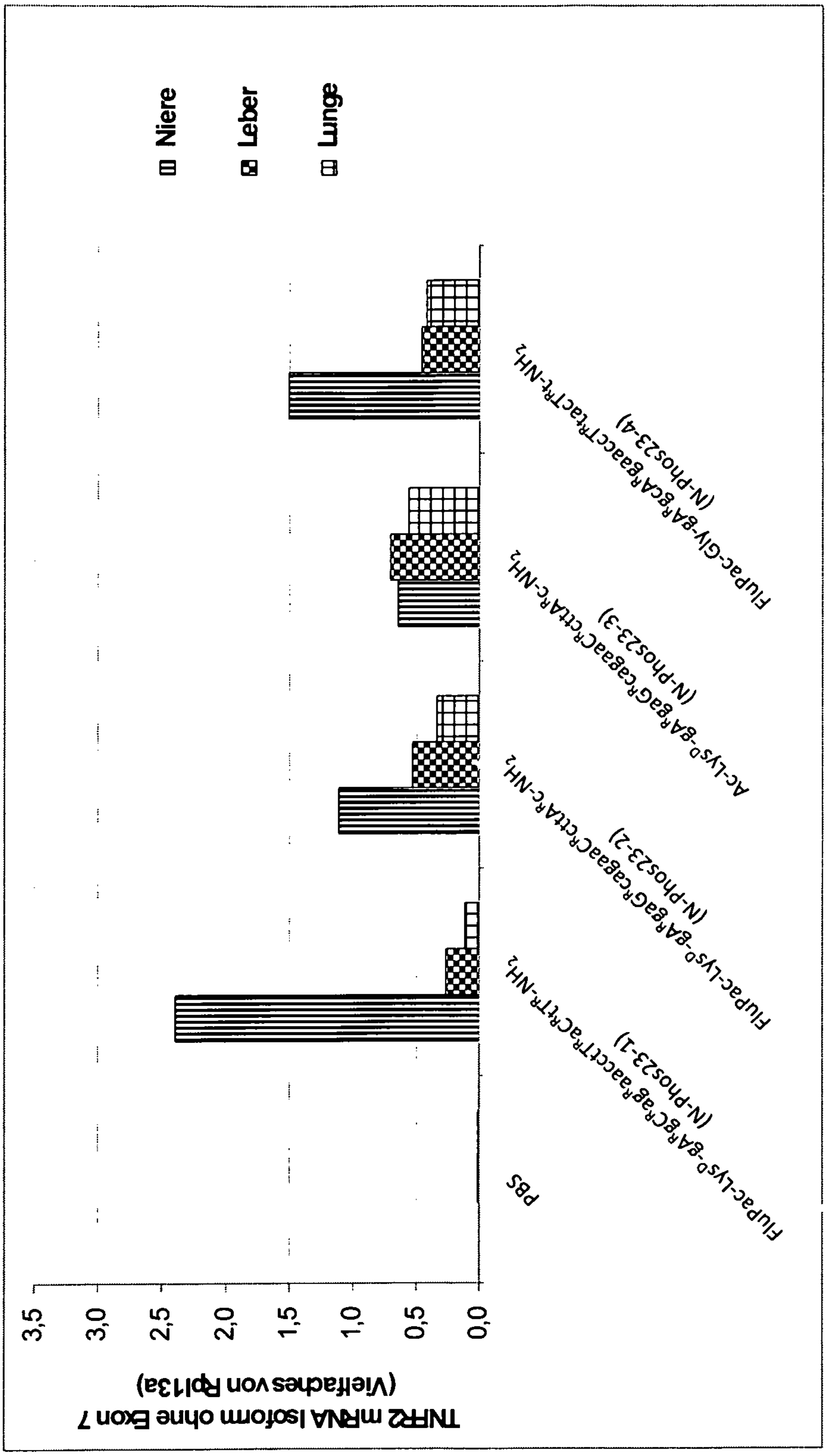
Niere
Leber
Lunge
TNFR2 mRNA Isoform ohne Exon 7
(Vielfaches von Rpl13a)
3,5
3,0
2,5
2,0
1,5
1,0
0,5
0,0
PBS
(N-Phos23-1)
(N-Phos23-2)
(N-Phos23-3)
(N-Phos23-4)

Abb. 6

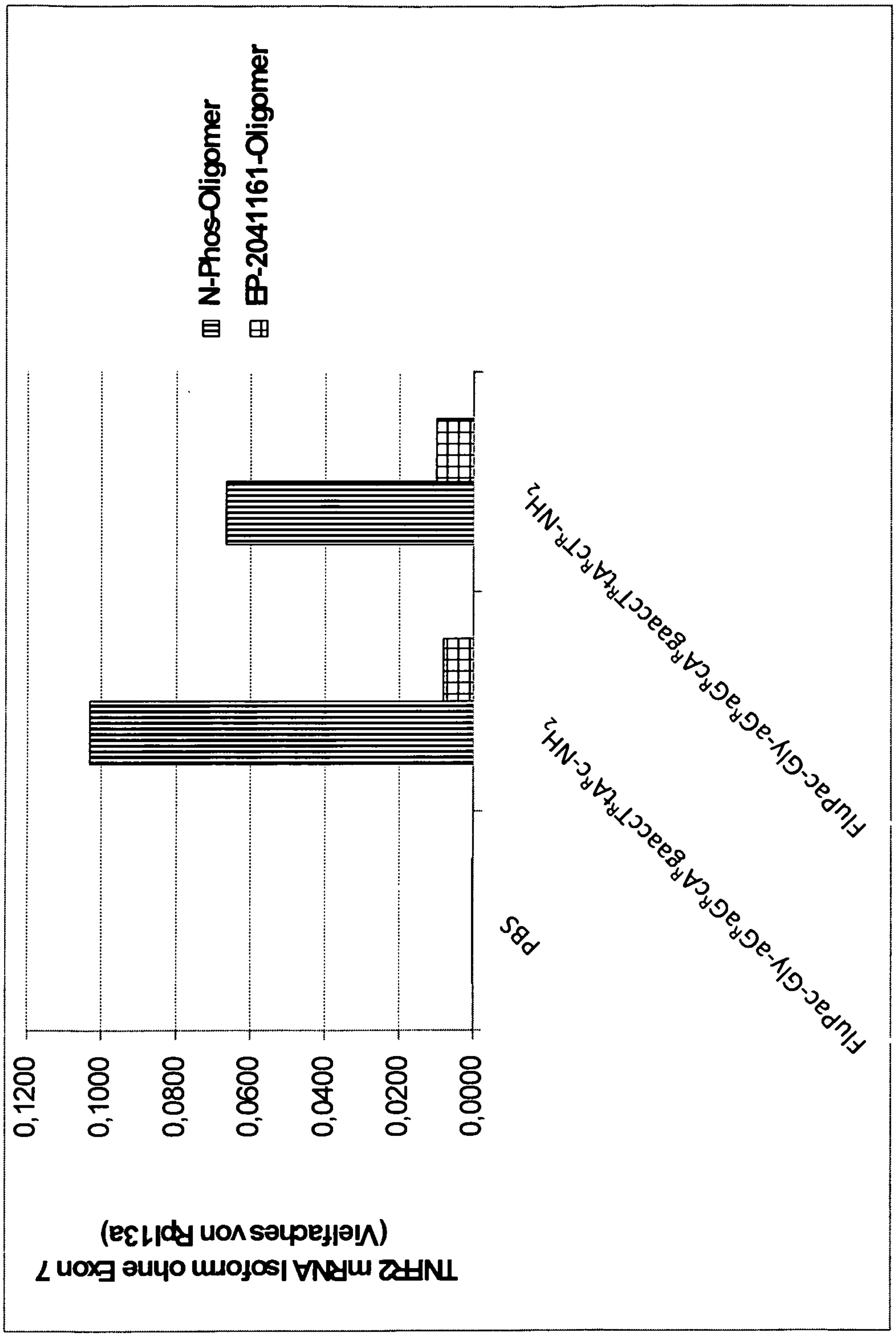
TNFR2 mRNA Isoform ohne Exon 7
(Vielfaches von Rpl13a)
0,0000
0,0200
0,0400
0,0600
0,0800
0,1000
0,1200
PBS
FluPac-Gly-aG$^R$aG$^R$cA$^R$gaacCT$^R$tA$^R$c-NH$_2$
FluPac-Gly-aG$^R$aG$^R$cA$^R$gaacCT$^R$tA$^R$CT$^R$-NH$_2$
N-Phos-Oligomer
EP-2041161-Oligomer

Abb. 7

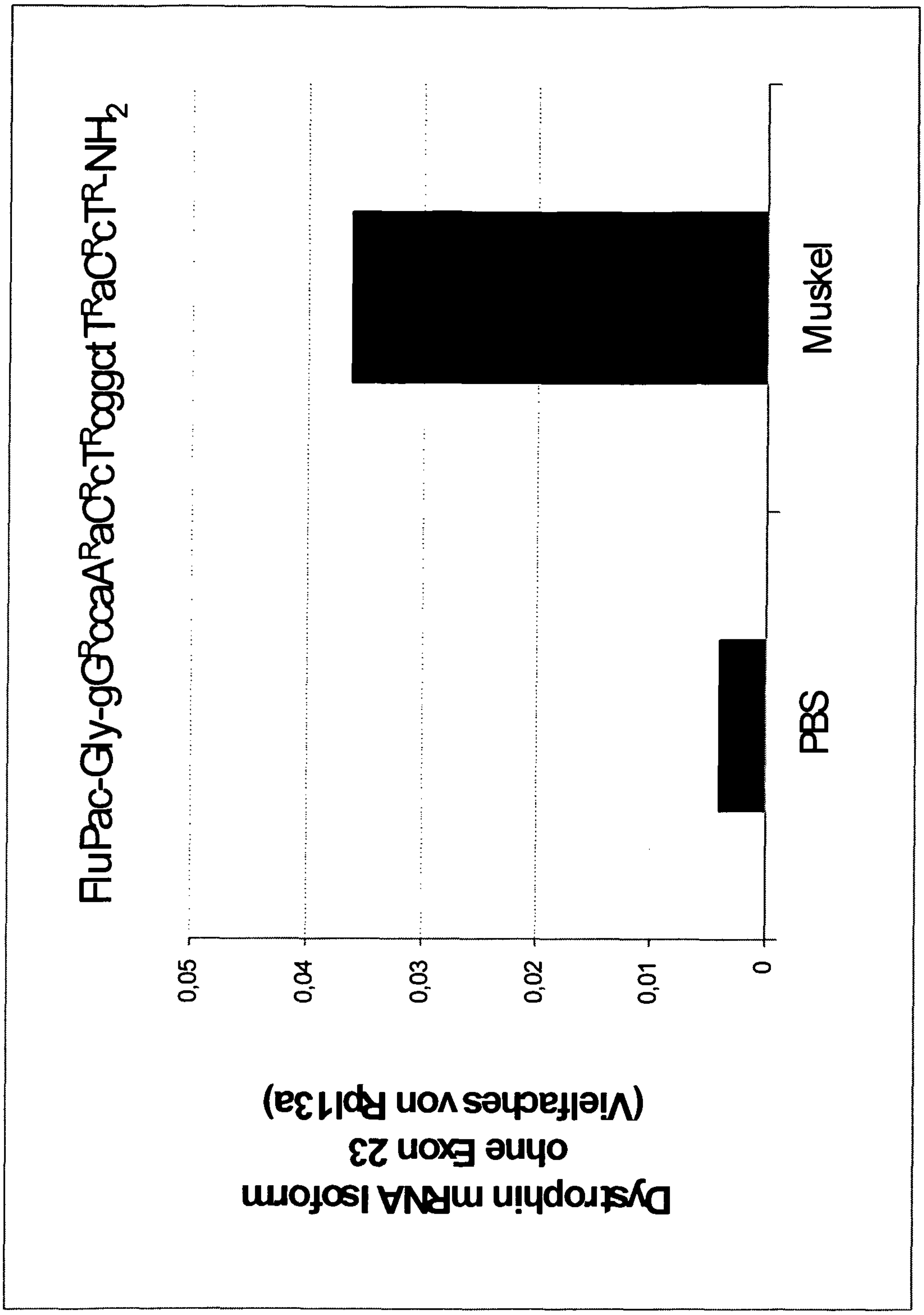
FluPac-Gly-$gG^{R}ccaA^{R}aC^{R}cT^{R}ggct\ T^{R}aC^{R}cT^{R}$-$NH_2$
0,05
0,04
0,03
0,02
0,01
0
Dystrophin mRNA Isoform
ohne Exon 23
(Vielfaches von Rpl13a)
PBS
Muskel

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- US 5719262 A
- EP 1157031 A
- EP 2041161 A


### In der Beschreibung aufgeführte Nicht-Patentliteratur


- **BETH M. et al.** *Antisense & Nucleic Acid Development,* 2002, vol. 12, 65-70
- **PÜSCHL A. et al.** *Tetrahedron Letters,* 1998, vol. 39, 4707-4710
- **NIELSEN P.** *Quarterly Reviews of Biophysics,* 2005, vol. 38 (4), 345-350
- **KOPPELHUS U. et al.** *Antisense & Nucleic Acid Drug Development,* 2002, vol. 12, 51-63
- **WIE R. CORRADINI et al.** *Current Topics in Medicinal Chemistry,* 2011, vol. 11 (12), 1535-1554
- **ZHOU P. et al.** *J. AM. CHEM. SOC.,* 2003, vol. 125, 6878-6879
- **THOMAS S. M. et al.** *ACS Chem. Biol.,* 15. Februar 2013, vol. 8 (2), 345-352
- **POSCH W. et al.** *Mol Med.,* 2012, vol. 18, 111-22
- **O. MARDER ; F.ALBERICIO.** *Chimica Oggi,* 2002, 37
- Peptide Bond Formation. Peptide Chemistry. Wiley-VCH Verlag, 2002, 197
- **R. CORRADINI et al.** *Current Topics in Medicinal Chemistry,* 2011, vol. 11 (12), 1535-1554
- **L. CHRISTENSEN ; R. FITZPATRICK ; B. GILDEA ; K.H. PETERSEN ; H.F. HANSEN ; T. KOCH ; M. EGHOLM ; O. BUCHARDT ; P.E. NIELSEN ; J. COULL.** *J.Pept.Sci.,* 1995, vol. 3, 175-183
- **T. KOCH ; H.F. HANSEN ; P. ANDERSEN ; T. LARSEN ; H.G. BATZ ; K. OTTESON ; H. ÖRUM.** *J.Pept.Res.,* 1997, vol. 49, 80-88
- **F. BERGMANN ; W. BANNWARTH ; S. TAM.** *Tetrahedron Lett.,* 1995, vol. 36, 6823-6826